(19) Europäisches Patentamt
European Patent Office
Office européen des brevets

(11) **EP 2 698 367 A1**

(12) **EUROPEAN PATENT APPLICATION**

(43) Date of publication:
**19.02.2014 Bulletin 2014/08**

(21) Application number: **12180482.7**

(22) Date of filing: **14.08.2012**

(51) Int Cl.:
***C07D 235/08*** *(2006.01)* ***C07D 235/12*** *(2006.01)*
***C07D 235/14*** *(2006.01)* ***C07D 235/18*** *(2006.01)*
***C07D 235/20*** *(2006.01)* ***C07D 401/04*** *(2006.01)*
***C07D 401/06*** *(2006.01)* ***C07D 401/14*** *(2006.01)*
***C07D 403/04*** *(2006.01)* ***C07D 403/12*** *(2006.01)*
***C07D 405/04*** *(2006.01)* ***C07D 405/14*** *(2006.01)*
***C07D 409/04*** *(2006.01)* ***C07D 487/04*** *(2006.01)*
***C07D 493/10*** *(2006.01)* ***A61K 31/404*** *(2006.01)*
***A61P 35/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Max-Planck-Gesellschaft zur Förderung der Wissenschaften e.V.
80539 München (DE)**

(72) Inventors:
- **Waldmann, Herbert
44265 Dortmund (DE)**
- **Triola, Gemma
44139 Dortmund (DE)**
- **Wittinghofer, Alfred
58313 Herdecke (DE)**
- **Shehab, Ismail
44225 Dortmund (DE)**
- **Bastiaens, Philippe
42119 Wuppertal (DE)**
- **Vartak, Nachiket
44139 Dortmund (DE)**
- **Papke, Björn
44137 Dortmund (DE)**
- **Zimmermann, Gunther
44137 Dortmund (DE)**

(74) Representative: **Arth, Hans-Lothar
ABK Patent Attorneys
Jasminweg 9
14052 Berlin (DE)**

(54) **Benzimidazoles for the treatment of cancer**

(57) The present invention relates to novel substituted benzimidazoles and stereoisomeric forms, prodrugs, solvates, hydrates and/or pharmaceutically acceptable salts of these compounds as well as pharmaceutical compositions containing at least one of these substituted benzimidazoles together with pharmaceutically acceptable carrier, excipient and/or diluents. Said novel substituted benzimidazoles binding to the prenyl binding pocket of PDEδ have been identified as useful for the prophylaxis and treatment of cancer by the inhibition of the binding of PDEδ to K-Ras and of oncogenic Ras signalling in cells by altering its localization leading to cell death or inhibition of proliferation.

Figure 4

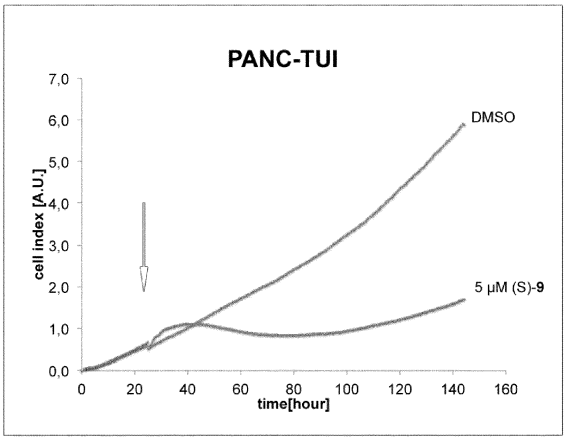


EP 2 698 367 A1

## Description

The present invention relates to novel substituted benzimidazoles and stereoisomeric forms, prodrugs, solvates, hydrates and/or pharmaceutically acceptable salts of these compounds as well as pharmaceutical compositions containing at least one of these substituted benzimidazoles together with pharmaceutically acceptable carrier, excipient and/or diluents. Said novel substituted benzimidazoles binding to the prenyl binding pocket of PDEδ have been identified as useful for the prophylaxis and treatment of cancer by the inhibition of the binding of PDEδ to K-Ras and of Ras signalling in cells by altering its localization leading to the inhibition of cell proliferation and to cell death.

## Background of the invention

In the treatment of cancer there is an ongoing need for the development of novel substances which are effective to induce cell cycle/proliferation arrest or cell death of cancer cells. The fundamental characteristics of these cells is that their control of the cell cycle and proliferation is disturbed and they evade oncogene induced stress response and cell senescence.

Ras proteins are key regulators of diverse cellular processes including proliferation and differentiation and are mutated in ca. 20 - 30 % of human cancers, with the K-Ras isoform most frequently mutated. However, despite substantial efforts to interfere with signaling by oncogenic Ras proteins in particular by means of Ras-farnesyltransferase inhibitors, has not led to clinically useful drugs yet. Signaling by Ras proteins critically depends on their correct subcellular localization which in turn is regulated by lipid modifications at their C-terminus. Thus, K-Ras, a major proto-oncogenic isoform of the Ras proteins, is S-farnesylated at its C-terminal CaaX box. PDEδ binds K-Ras via the farnesylated C-terminus and has an essential role in sustaining its spatial organization and proper localization in cells by facilitating its intracellular diffusion to enhance the kinetics of trapping at the right membrane compartment, i.e. the plasma membrane for K-Ras. This regulation of Ras localization and signaling by PDEδ suggests that interfering with the PDEδ-Ras interaction by means of small molecules might provide a novel opportunity to suppress signaling from oncogenic and normal Ras. Suppressing oncogenic Ras signaling results in halting Ras-dependent tumor proliferation.

It is the objective of the present invention to provide compounds and/or pharmaceutically acceptable salts thereof which can be used as pharmaceutically active agents, especially for the treatment of cancer, tumors and proliferative diseases as well as compositions comprising at least one of those compounds and/or pharmaceutically acceptable salts thereof as pharmaceutically active ingredients.

The objective of the present invention is solved by the teaching of the independent claims. Further advantageous features, aspects and details of the invention are evident from the dependent claims, the description, the figures, and the examples of the present application.

## Brief description of the invention

In the present invention it was surprisingly found that the binding of benzimidazoles of the general formula (I) to the prenyl binding pocket of PDEδ induce strong inhibition of the binding of PDEδ to K-Ras and oncogenic Ras signalling in cells by altering its localization thereby causing inhibition of tumor cell proliferation and tumor cell death. These inventive compounds thereby are useful for the treatment of cancer, tumors and other proliferative diseases.

## Description of the invention

Accordingly the present invention relates to the benzimidazoles of the general formula (I):

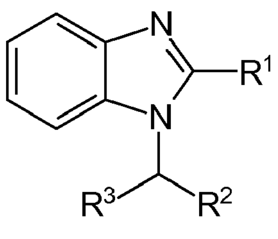

**(I)**

wherein

**$R^1$** represents $—R^4$, $—CH_2R^4$, $—CH_2—OR^4$, $—CH(OH)—R^4$, $—CH=CH—CO—R^4$, or $—C_2H_4—NH—CO—R^4$;

**$R^2$** represents $—C(CH_3)=CH_2$, $—CH_2—CH(CH_3)_2$, $-C=CH$, $—CO—NH—R^{11}$,

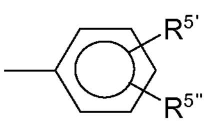, 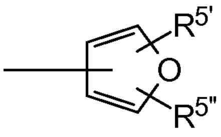, 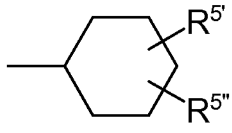, 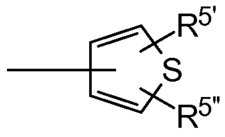,

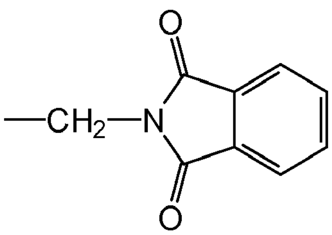, 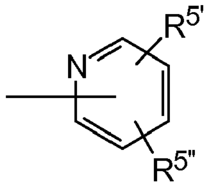, 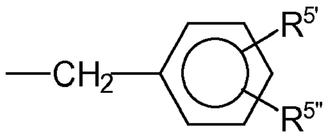,

$—CO—N(R^{11})—COOC(CH_3)_3$, $—CH_2OH$, $—CH_2NH_2$, $—CH_2—CH{=}CH_2$, $—CH{=}CH_2$, or $—COOCH_3$;

**$R^3$** represents -H, $—CH_3$,

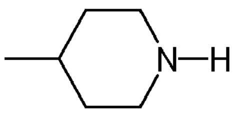, 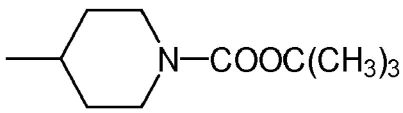,

$—C_2H_5$, $—CH_2OH$, $—COOCH_3$, —cyclo-$C_6H_{11}$, or —Ph, or **$R^3$** together with **$R^5$** is the residue —NH—;

**$R^4$** represents $—CH_3$, $—C_2H_5$, $—C_3H_7$, $—CH(CH_3)_2$, $—C_4H_9$, $—CH_2—CH(CH_3)_2$, $—CH(CH_3)—C_2H_5$, $—C(CH_3)_3$, $—C_5H_{11}$, $—CH(CH_3)—C_3H_7$, $—CH_2—CH(CH_3)—C_2H_5$, $—CH(CH_3)—CH(CH_3)_2$, $—C(CH_3)_2—C_2H_5$, $—CH_2—C(CH_3)_3$, $—CH(C_2H_5)_2$, $—C_2H_4—CH(CH_3)_2$, $—C_6H_{13}$, $—C_3H_6—CH(CH_3)_2$, $—C_2H_4—CH(CH_3)—C_2H_5$, $—CH(CH_3)—C_4H_9$, $—CH_2—CH(CH_3)—C_3H_7$, $—CH(CH_3)—CH_2—CH(CH_3)_2$, $—CH(CH_3)—CH(CH_3)—C_2H_5$, $—CH_2—CH(CH_3)—CH(CH_3)_2$, $—CH_2—C(CH_3)_2—C_2H_5$, $—C(CH_3)_2—C_3H_7$, $—C(CH_3)_2—CH(CH_3)_2$, $—C_2H_4—C(CH_3)_3$, $—CH_2—CH(C_2H_5)_2$, $—CH(CH_3)—C(CH_3)_3$, $—C(C_2H_5)_3$, $—CH_2—C(C_2H_5)_3$, $—C_3H_6—CH(CH_3)R^5$,

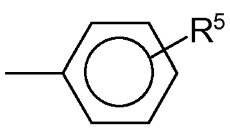, 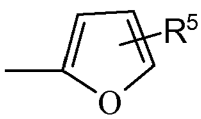, 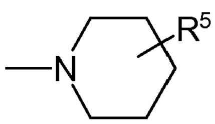, 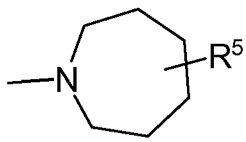

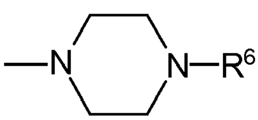, 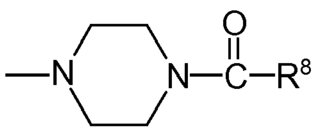, 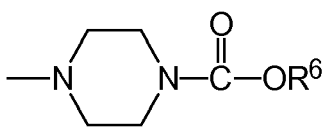,

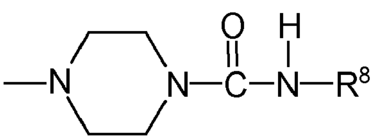

**$R^5$, $R^{5'}$, $R^{5''}$** represent independently of each other -H, $—OCH_3$, $—OC_2H_5$, $—CO—NH—R^6$, $—OR^6$, $—N(CH_3)_2$, $—N(C_2H_5)_2$, $—CO—R^7$, $—CH_2—CH{=}CH_2$, -OH, $—NH_2$, -F, —Cl, -Br, —I, $—CH_3$, $—C_2H_5$, -COOH, or $—COO—R^6$,

**$R^6$** represents $-CH_2-R^7$, $-C_2H_4-R^7$, $-C_3H_6-R^7$,

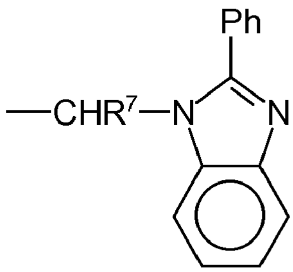

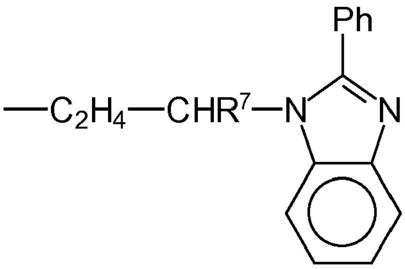,

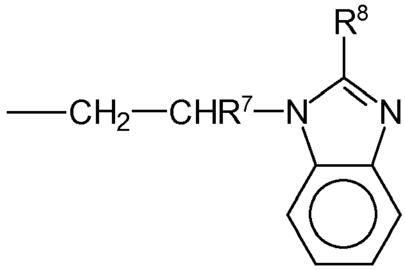

or

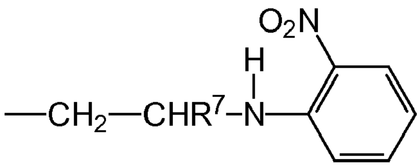;

**$R^7$** represents -H, $-CH_3$, $-CH=CH_2$, $-CH_2COOH$, $-CH_2-CH=CH_2$, $-NHR^8$, $-N(CH_3)R^8$, $-N(C_2H_5)R^8$, $-CH_2CONHC_3H_6(OC_2H_4)_3CH_2NHR^8$,

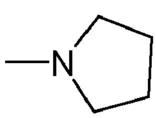, 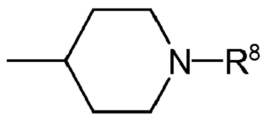, 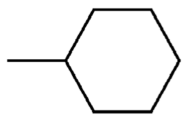,

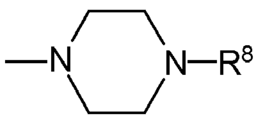, 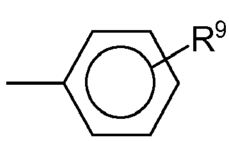, 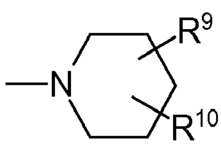;

**$R^8$** represents -H, $-CO_2C(CH_3)_3$,

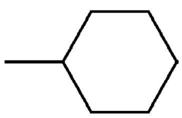, 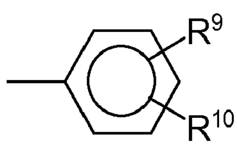,

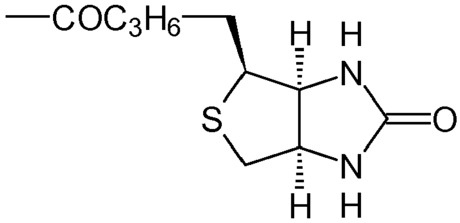

;

$R^9$ and $R^{10}$ represent independently of each other -H, -F, -Br, —Cl, —I, -CN, -OH, —$OCH_3$, —$OC_2H_5$, —$CH_3$, or —$C_2H_5$;

$R^{11}$ represents —$CH_2$—$R^{12}$, or —$C_2H_4$—$R^{12}$;

$R^{12}$ represents -Ph, or -OH;

and enantiomers, stereoisomeric forms, mixtures of enantiomers, diastereomers, mixtures of diastereomers, hydrates, solvates, acid salt forms, tautomers, and racemates of the above mentioned compounds and pharmaceutically acceptable salts thereof.

Preferred are compounds of general formula (I), wherein

$R^1$ represents —$R^4$, —$CH_2R^4$, —$CH_2$—$OR^4$, —CH(OH)—$R^4$, —H=CH—CO—$R^4$, or —$C_2H_4$—NH—CO—$R^4$;

$R^2$ represents —C($CH_3$)=$CH_2$, —$CH_2$—CH($CH_3$)$_2$, -C=CH, —CO—NH—$R^{11}$,

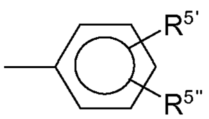
,
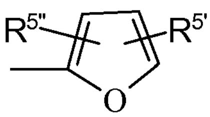
,
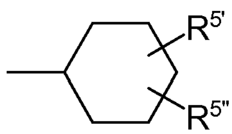
,
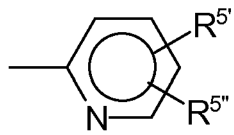
,

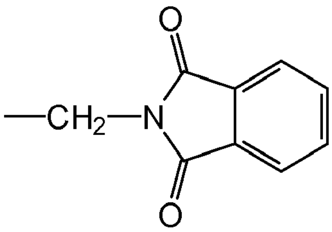
,
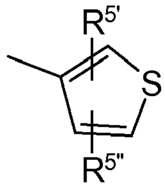
,
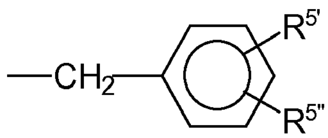
,

— CO—N($R^{11}$)—COOC($CH_3$)$_3$, —$CH_2OH$, —$CH_2NH_2$, —$CH_2$—CH=$CH_2$, or —$COOCH_3$;

$R^3$ represents -H, —$CH_3$,

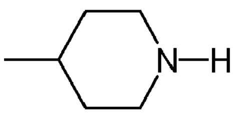
,
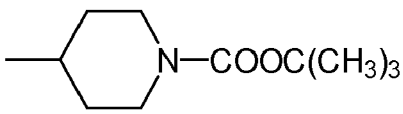
,

— $CH_2OH$, —$COOCH_3$, —cyclo-$C_6H_{11}$, or —Ph, or $R^3$ together with $R^5$ is the residue —NH—;

$R^4$ represents —$CH_3$, —$C_2H_5$, —$C_3H_7$, —CH($CH_3$)$_2$, —$C_4H_9$, —$CH_2$—CH($CH_3$)$_2$, —CH($CH_3$)—$C_2H_5$, —C($CH_3$)$_3$, —$C_5H_{11}$, —CH($CH_3$)—$C_3H_7$, —$CH_2$—CH($CH_3$)—$C_2H_5$, —CH($CH_3$)—CH($CH_3$)$_2$, —C($CH_3$)$_2$—$C_2H_5$, —$CH_2$—C($CH_3$)$_3$, —CH($C_2H_5$)$_2$, —$C_2H_4$—CH($CH_3$)$_2$, —$C_6H_{13}$, —$C_3H_6$—CH($CH_3$)$_2$, —$C_2H_4$—CH($CH_3$)—$C_2H_5$, —CH($CH_3$)—$C_4H_9$, —$CH_2$—CH($CH_3$)—$C_3H_7$, —CH($CH_3$)—$CH_2$—CH($CH_3$)$_2$, —CH($CH_3$)—CH($CH_3$)—$C_2H_5$, —$CH_2$—CH

$(CH_3)—CH(CH_3)_2$, $—CH_2—C(CH_3)_2—C_2H_5$, $—C(CH_3)_2—C_3H_7$, $—C(CH_3)_2—CH(CH_3)_2$, $—C_2H_4—C(CH_3)_3$, $—CH_2—CH(C_2H_5)_2$, $—CH(CH_3)—C(CH_3)_3$, $—C(C_2H_5)_3$, $—CH_2—C(C_2H_5)_3$, $—C_3H_6—CH(CH_3)R^5$,

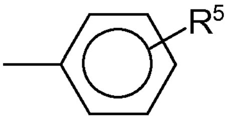 , 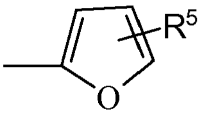 , 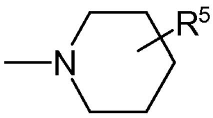 , 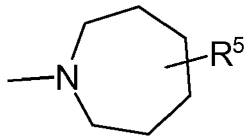 ,

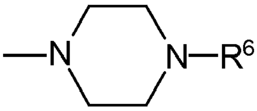 , 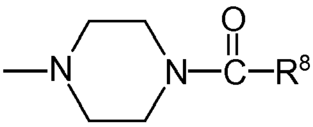 , 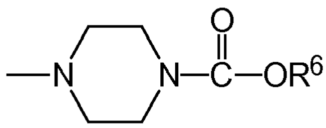 ,

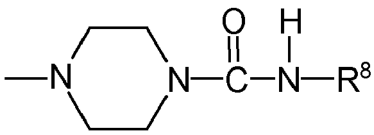

**$R^5$, $R^{5'}$, $R^{5''}$** represent independently of each other -H, $—OCH_3$, $—OC_2H_5$, $—CO—NH—R^6$, $—OR^6$, $—N(CH_3)_2$, $—N(C_2H_5)_2$, $—CO—R^7$, $—CH_2—CH=CH_2$, -OH, $—NH_2$, -F, —Cl, -Br, —I, $—CH_3$, $—C_2H_5$, -COOH, or $—COO—R^6$,

**$R^6$** represents $—CH_2—R^7$, $—C_2H_4—R^7$, $—C_3H_6—R^7$,

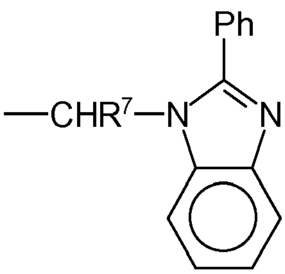 ,

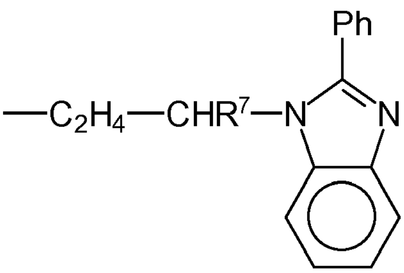 , 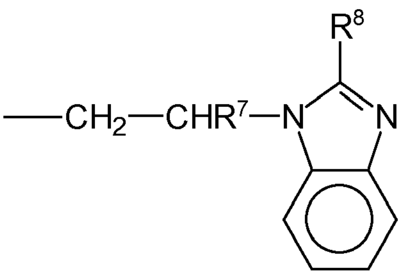

or

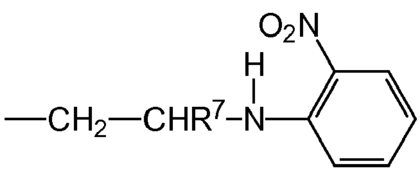 ;

**$R^7$** represents -H, $-CH_2COOH$, $-NHR^8$, $-N(CH_3)R^8$, $-N(C_2H_5)R^8$, $-CH_3$, $-CH=CH_2$, $-CH_2-CH=CH_2$, $-CH_2CONHC_3H_6(OC_2H_4)_3CH_2NHR^8$,

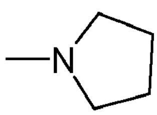 , 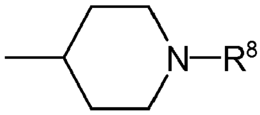 , 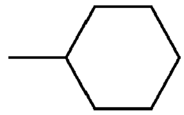 ,

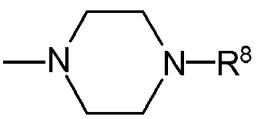 , 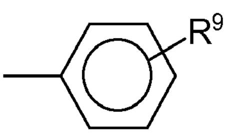 , 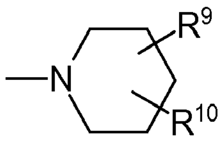 ;

**$R^8$** represents -H, $-CO_2C(CH_3)_3$,

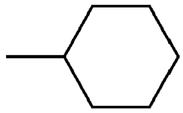 , 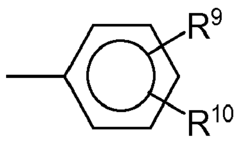

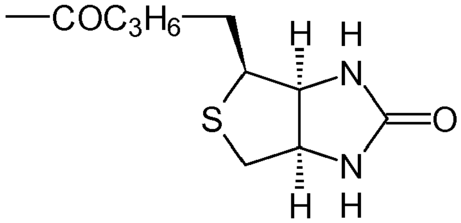 ;

**$R^9$** and **$R^{10}$** represent independently of each other -H, -F, -Br, -Cl, -I, -CN, -OH, $-OCH_3$, $-OC_2H_5$, $-CH_3$, or $-C_2H_5$;

**$R^{11}$** represents $-CH_2-R^{12}$ or $-C_2H_4-R^{12}$;

**$R^{12}$** represents -Ph or -OH;

and enantiomers, stereoisomeric forms, mixtures of enantiomers, diastereomers, mixtures of diastereomers, hydrates, solvates, acid salt forms, tautomers, and racemates of the above mentioned compounds and pharmaceutically acceptable salts thereof.

Moreover preferred are compounds of general formula (I), wherein

**$R^1$** represents $-R^4$, $-CH_2R^4$, $-CH_2-OR^4$, $-CH(OH)-R^4$, $-CH=CH-CO-R^4$, or $-C_2H_4-NH-CO-R^4$;

**$R^2$** is $-C(CH_3)=CH_2$, $-CH_2-CH(CH_3)_2$, $-CH_2-Ph$, $-C\equiv CH$, $-CO-NH-R^{11}$, $-COOCH_3$, $-CO-N(R^{11})-COOC(CH_3)_3$, $-cyclo-C_6H_{11}$, $-CH_2OH$, $-CH_2NH_2$, $-CH_2-CH=CH_2$,

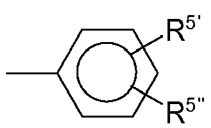 , 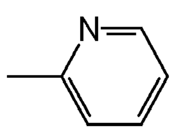 , 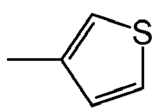 , 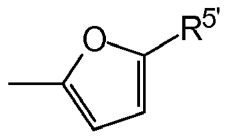 ,

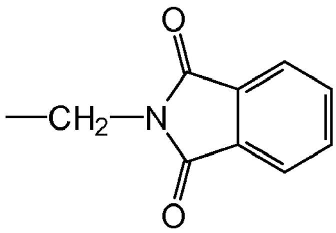

;

**R**$^{3}$ represents -H, —$CH_3$, —$CH_2OH$, —$COOCH_3$, —cyclo-$C_6H_{11}$, —Ph,

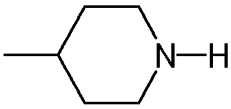

, or

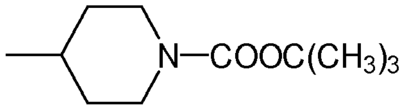

or **R**$^{3}$ together with **R**$^{5}$ form the residue —NH—;

**R**$^{4}$ represents —$CH_3$, —$C_2H_5$, —$C_3H_7$, —$CH(CH_3)_2$, —$C_4H_9$, —$CH_2$—$CH(CH_3)_2$, —$C(CH_3)_3$, —$C_5H_{11}$, —$CH(C_2H_5)_2$, —$CH_2$—$C(CH_3)_3$, —$CH_2$—$CH(C_2H_5)_2$, —$C(C_2H_5)_3$, —$CH_2$—$C(C_2H_5)_3$, —$C_3H_6$—$CH(CH_3)R^5$,

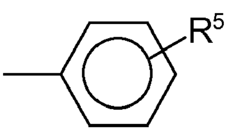

,

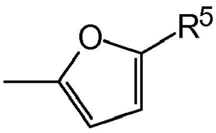

,

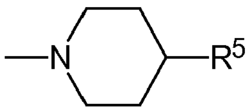

,

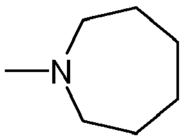

,

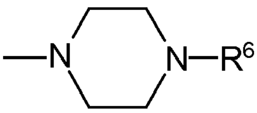

,

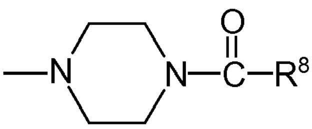

,

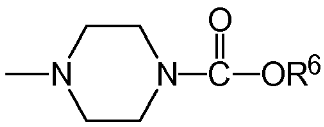

,

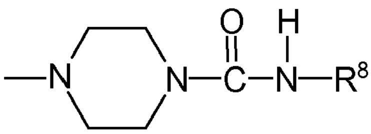

;

**R**$^{5}$**, R**$^{5'}$**, R**$^{5''}$ represent independently of each other -H, —$OCH_3$, —$CH_3$, —CO—NH—$R^6$, —$OR^6$, —$N(CH_3)_2$, —CO—$R^7$, —$CH_2$—$CH{=}CH_2$, —OH, —F, —Cl, —Br, —I, —COOH, or —COO—$R^6$,

**R**$^{6}$ represents —$CH_2$—$R^7$, —$C_2H_4$—$R^7$, —$C_3H_6$—$R^7$,

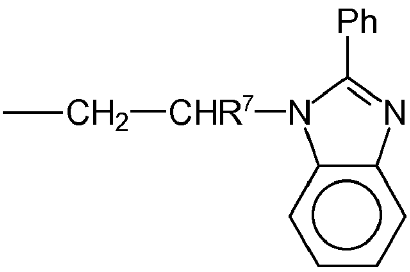

or

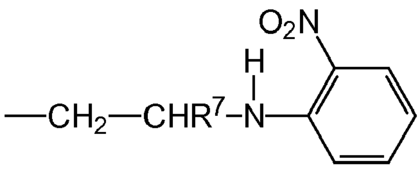;

**$R^7$** represents -H, $-CH_2COOH$, $-NHR^8$, $-N(CH_3)R^8$, $-N(C_2H_5)R^8$, $-CH=CH_2$, $-CH_2-CH=CH_2$, $-CH_3$,

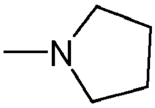, 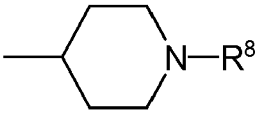, 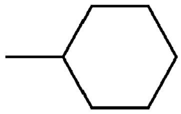,

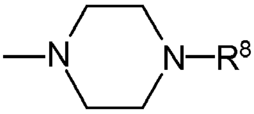, 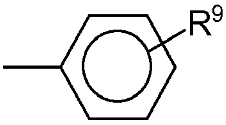, 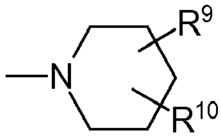;

**$R^8$** represents -H, $-CO_2C(CH_3)_3$,

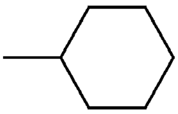, or 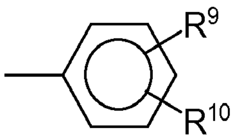;

**$R^9$** and **$R^{10}$** represent independently of each other -H, $-Cl$, -OH, $-OCH_3$, $-OC_2H_5$, or $-CH_3$;

**$R^{11}$** represents $-CH_2-R^{12}$ or $-C_2H_4-R^{12}$;

**$R^{12}$** represents -Ph or -OH.

Also preferred is if **$R^5$** represents -H, $-OCH_3$, $-CH_3$, $-CO-NH-R^6$, $-OR^6$, $-N(CH_3)_2$, $-CO-R^7$, -OH, -F, $-Cl$, -Br, $-I$, -COOH, or $-COO-R^6$.

Also preferred is if **$R^{5'}$** and **$R^{5''}$** represent independently of each other -H, $-CH_3$, -OH, -F, $-Cl$, -Br, or -I.

Also preferred is if **$R^6$** represents

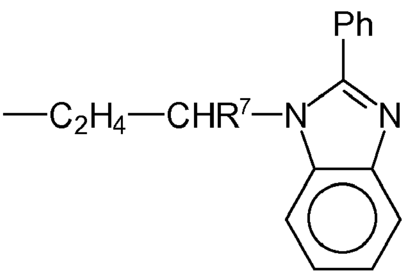 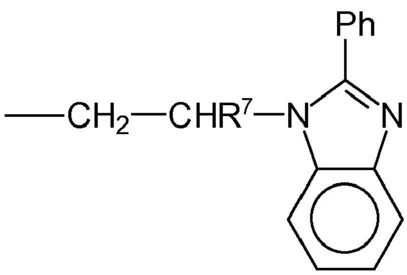

or

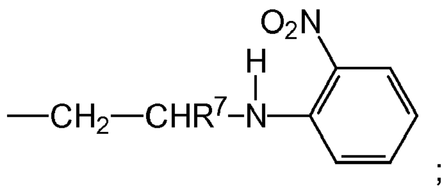

Also preferred are compounds wherein **$R^2$** represents p-hydroxyphenyl.

Still preferred are compounds wherein **$R^2$** represents

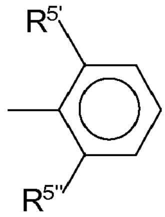

wherein $R^5$, $R^{5'}$, $R^{5''}$ represent independently of each other -F, —Cl, or -Br;

The chemical IUPAC names of the most preferred compounds read as follows (Table 1):

Table 1

| Compound | Name |
|---|---|
| S02 | 2-(4-(Allyloxy)phenyl)-1-benzyl-1H-benzo[d]imidazole |
| S03 | 2-(4-(Allyloxy)phenyl)-1-(pyridin-2-ylmethyl)-1H-benzo[d]imidazole |
| S04 | 2-(4-(Allyloxy)phenyl)-1-(2-methylbenzyl)-1H-benzo[d]imidazole |
| S05 | 2-(4-(Allyloxy)phenyl)-1-(3-methylbenzyl)-1H-benzo[d]imidazole |
| S06 | 2-(4-(Allyloxy)phenyl)-1-(4-fluorobenzyl)-1H-benzo[d]imidazole |
| S07 | 4-(1-Benzyl-1H-benzo[d]imidazol-2-yl)phenol |
| S08 | 4-(1-(Pyridin-2-ylmethyl)-1H-benzo[d]imidazol-2-yl)phenol |
| S09 | 4-(1-(4-Fluorobenzyl)-1H-benzo[d]imidazol-2-yl)phenol |
| S10 | 4-(1-(2-Methylbenzyl)-1H-benzo[d]imidazol-2-yl)phenol |
| S11 | 4-(1-(3-Methylbenzyl)-1H-benzo[d]imidazol-2-yl)phenol |
| S12 | N-Benzyl-2-cyclohexyl-2-(2-phenyl-1H-benzo[d]imidazol-1-yl)acetamide |
| S13 | N-Benzyl-2-(2-phenyl-1H-benzo[d]imidazol-1-yl)-2-(piperidin-4-yl)acetamide |
| S16 | 2-Cyclohexyl-2-(2-phenyl-1H-benzo[d]imidazol-1-yl)ethanol |
| S18 | Methyl 2-(2-phenyl-1H-benzo[d]imidazol-1-yl)acetate |
| S19 | Methyl 2-(2-phenyl-1H-benzo[d]imidazol-1-yl)pent-4-enoate |
| S20 | 2-(2-Phenyl-1H-benzo[d]imidazol-1-yl)pent-4-en-1-ol |
| S21 | 2-(2-Phenyl-1H-benzo[d]imidazol-1-yl)ethanol |
| S26 | 2-Phenyl-1-(1-(piperidin-4-yl)-2-(4-(1-(pyridin-2-ylmethyl)-1H-benzo[d]imidazol-2-yl)phenoxy)ethyl)-1H-benzo[d]imidazole |
| S27 | 1-(3-Methylbenzyl)-2-(4-(2-(2-phenyl-1H-benzo[d]imidazol-1-yl)-2-(piperidin-4-yl)ethoxy)phenyl)-1H-benzo[d]imidazole |
| S28 | 1-(4-Fluorobenzyl)-2-(4-(2-(2-phenyl-1H-benzo[d]imidazol-1-yl)-2-(piperidin-4-yl)ethoxy)phenyl)-1H-benzo[d]imidazole |

(continued)

| Compound | Name |
|---|---|
| S29 | 1-(2-Methylbenzyl)-2-(4-(2-(2-phenyl-1H-benzo[d]imidazol-1-yl)-2-(piperidin-4-yl)ethoxy)phenyl)-1H-benzo[d]imidazole |
| S31 | Ethyl 1-(1-benzyl-1H-benzo[d]imidazol-2-yl)piperidine-4-carboxylate |
| S32 | Ethyl 1-(1-(2-chloro-6-fluorobenzyl)-1H-benzo[d]imidazol-2-yl)piperidine-4-carboxylate |
| S33 | Ethyl 1-(1-(thiophen-3-ylmethyl)-1H-benzo[d]imidazol-2-yl)piperidine-4-carboxylate |
| S34 | 1-(1-(Thiophen-3-ylmethyl)-1H-benzo[d]imidazol-2-yl)piperidine-4-carboxylic acid |
| S35 | 1-(1-Benzyl-1H-benzo[d]imidazol-2-yl)piperidine-4-carboxylic acid |
| S36 | 1-(1-(2-Chloro-6-fluorobenzyl)-1H-benzo[d]imidazol-2-yl)piperidine-4-carboxylic acid |
| S37 | 2-Cyclohexyl-2-(2-phenyl-1H-benzo[d]imidazol-1-yl)ethyl 1-(1-benzyl-1H-benzo[d]imidazol-2-yl) piperidine-4-carboxylate |
| S38 | 2-Cyclohexyl-2-(2-phenyl-1H-benzo[d]imidazol-1-yl)ethyl 1-(1-(2-chloro-6-fluorobenzyl)-1H-benzo[d] imidazol-2-yl)piperidine-4-carboxylate |
| S39 | 2-Cyclohexyl-2-(2-phenyl-1H-benzo[d]imidazol-1-yl)ethyl 1-(1-(thiophen-3-ylmethyl)-1H-benzo[d] imidazol-2-yl)piperidine-4-carboxylate |
| S43 | 2-(2-Phenyl-1H-benzo[d]imidazol-1-yl)-2-(piperidin-4-yl)ethyl 1-(1-(2-chloro-6-fluorobenzyl)-1H-benzo [d]imidazol-2-yl)piperidine-4-carboxylate |
| S48 | 2-(2-Phenyl-1H-benzo[d]imidazol-1-yl)-2-(piperidin-4-yl)ethyl 4-(1-benzyl-1H-benzo[d]imidazol-2-yl) piperazine-1-carboxylate |
| S53 | (S)-tert-Butyl 4-(1-(2-(3-cyanophenyl)-1H-benzo[d]imidazol-1-yl)-2-(1-(1-(thiophen-3-ylmethyl)-1H-benzo[d]imidazol-2-yl)piperidine-4-carbonyloxy)ethyl)piperidine-1-carboxylate |
| 01 | 1-Benzyl-2-phenyl-1H-benzo[d]imidazole |
| 02 | 6-Pyiridyl-5,6-dihydrobenzo[4-5]imidazo[1,2-c]quinazoline |
| 03 | 1-Benzyl-2-(4-(2-(2-phenyl-1H-benzo[d]imidazol-1-yl)pent-4-enyloxy)phenyl)-1H-benzo[d]imidazole |
| 04 | 1-Benzyl-2-(4-(2-cyclohexyl-2-(2-phenyl-1H-benzo[d]imidazol-1-yl)ethoxy)phenyl)-1H-benzo[d] imidazole |
| 05 | 4-(4-(1-Benzyl-1H-benzo[d]imidazol-2-yl)phenoxy)-3-(2-phenyl-1H-benzo[d]imidazol-1-yl)butanoic acid |
| 06 | 1-Benzyl-2-(4-(2-(2-phenyl-1H-benzo[d]imidazol-1-yl)ethoxy)phenyl)-1H-benzo[d]imidazole |
| 07 | N-(18-(4-(1-Benzyl-1H-benzo[d]imidazol-2-yl)phenoxy)-15-oxo-17-(2-phenyl-1H-benzo[d]imidazol-1-yl)-4,7,10-trioxa-14-azaoctadecyl)-5-((3aS,4S,6aR)-2-oxohexahydro-1H-thieno[3,4-d]imidazol-4-yl) pentanamide |
| 08 | tert-Butyl-4-(2-(4-(1-benzyl-1H-benzo[d]imidazol-2-yl)phenoxy)-1-(2-phenyl-1H-benzo[d]imidazol-1-yl)ethyl)piperidine-1-carboxylate |
| *(S)*-08 | (S)-tert-Butyl 4-(2-(4-(1-benzyl-1H-benzo[d]imidazol-2-yl)phenoxy)-1-(2-phenyl-1H-benzo[d]imidazol-1-yl)ethyl)piperidine-1-carboxylate |
| 09 | 1-Benzyl-2-(4-(2-(2-phenyl-1H-benzo[d]imidazol-1-yl)-2-(piperidin-4-yl)ethoxy)phenyl)-1H-benzo[d] imidazole |
| *(S)*-09 | (*S*)-1-Benzyl-2-(4-(2-(2-phenyl-1H-benzo[d]imidazol-1-yl)-2-(piperidin-4-yl)ethoxy)phenyl)-1H-benzo [d]imidazole |
| (R)-09 | (*R*)-1-Benzyl-2-(4-(2-(2-phenyl-1H-benzo[d]imidazol-1-yl)-2-(piperidin-4-yl)ethoxy)phenyl)-1H-benzo [d]imidazole |
| 10 | N-(2-(2-Phenyl-1H-benzo[d]imidazol-1-yl)-2-(piperidin-4-yl)ethyl)-1-(1-(thiophen-3-ylmethyl)-1H-benzo[d]imidazol-2-yl)piperidine-4-carboxamide |

(continued)

| Compound | Name |
|---|---|
| 11 | 2-(2-Phenyl-1H-benzo[d]imidazol-1-yl)-2-(piperidin-4-yl)ethyl 1-(1-benzyl-1H-benzo[d]imidazol-2-yl) piperidine-4-carboxylate |
| (*S*)-12 | (*S*)-2-(2-Phenyl-1H-benzo[d]imidazol-1-yl)-2-(piperidin-4-yl)ethyl 1-(1-(thiophen-3-ylmethyl)-1H-benzo[d]imidazol-2-yl)piperidine-4-carboxylate |
| (*R*)-12 | (*R*)-2-(2-Phenyl-1H-benzo[d]imidazol-1-yl)-2-(piperidin-4-yl)ethyl 1-(1-(thiophen-3-ylmethyl)-1H-benzo[d]imidazol-2-yl)piperidine-4-carboxylate |

**Chemical synthesis of benzimidazoles**

The inventive benzimidazoles (I) according to the present invention can be prepared by methods known to one skilled in the art. The synthesis is preferably carried out according to the general synthetic approaches, shown in scheme 1. As depicted in scheme 1 (a) in a first step 1,2-diaminobenzene (II) was condensed with any suitable aldehyde $R^1$-CHO that is commercially available or can be synthesized according to or in analogy to literature procedures, to give benzimidazoles (III). The inventive benzimidazoles (I) were obtained through the alkylation of these intermediates (III) with any suitable bromide, Br-$CHR^2R^3$ or alcohol, HO-$CHR^2R^3$ that are available either commercially or by synthetic means.

As alternative synthetic approach at C-2 position chlorinated benzimidazoles (IV) provide the basis to react with many classes of C-, S-, N- or O-nucleophiles,H-$R^1$ that are available either commercially or by synthetic means (scheme 1 (b)).

As depicted in scheme 1 (c) the Ugi-reaction (4-components reaction) of a protected 1,2-diaminobezene (V) with carboxylic acid $R^1CO_2H$, aldehyde $R^3CHO$ and isonitrile $R^{11}NC$ and the following acidification of intermediates (VI) provide a convenient one pot synthesis of the inventive benzimidazoles (I).

Therefore, the synthetic approaches according to scheme 1 enable the synthesis of any of the benzimidazoles disclosed in the present invention.

In the following schemes occurring abbreviations mean DBAD (dibenzyl azodicarboxylate), DCM (dichloromethane), DIPEA (N-ethyl-N,N-diisopropylamine), DMF (dimethylformamide), HOBT (hydroxybenzotriazole), MeOH (methanol), MeCN (acetonitrile), Boc (tert-butyloxycarbonyl), Ph (phenyl), $PBu_3$ (tributylphosphine), PS-$PPh_3$ (Polystyrene-supported triphenylphosphine), PyBoP (benzotriazol-1-yl-oxytripyrrolidinophosphonium hexafluorophosphate), Pyr (pyridine), THF (tetrahydrofurane), TMAD (tetramethylazodicarboxamide), Tol (toluene).

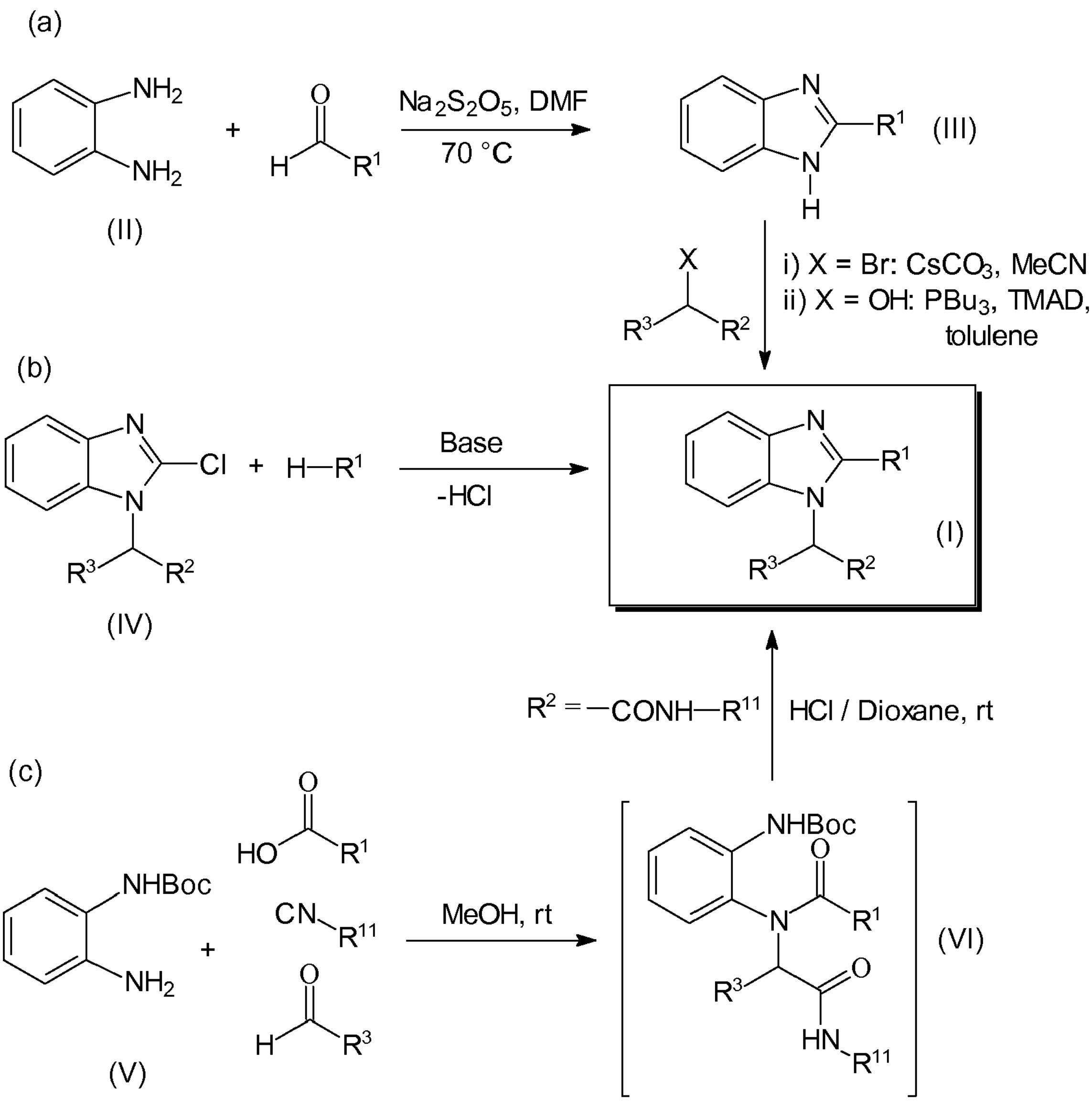


scheme 1.

**Synthesis of linked benzimidazoles**

As depicted in scheme 2 fragments A, B and C can be linked with fragments D and E by the appropriate coupling reactions. In such embodiment the residue $R^2$ represents phenyl or thiophen-3-yl.

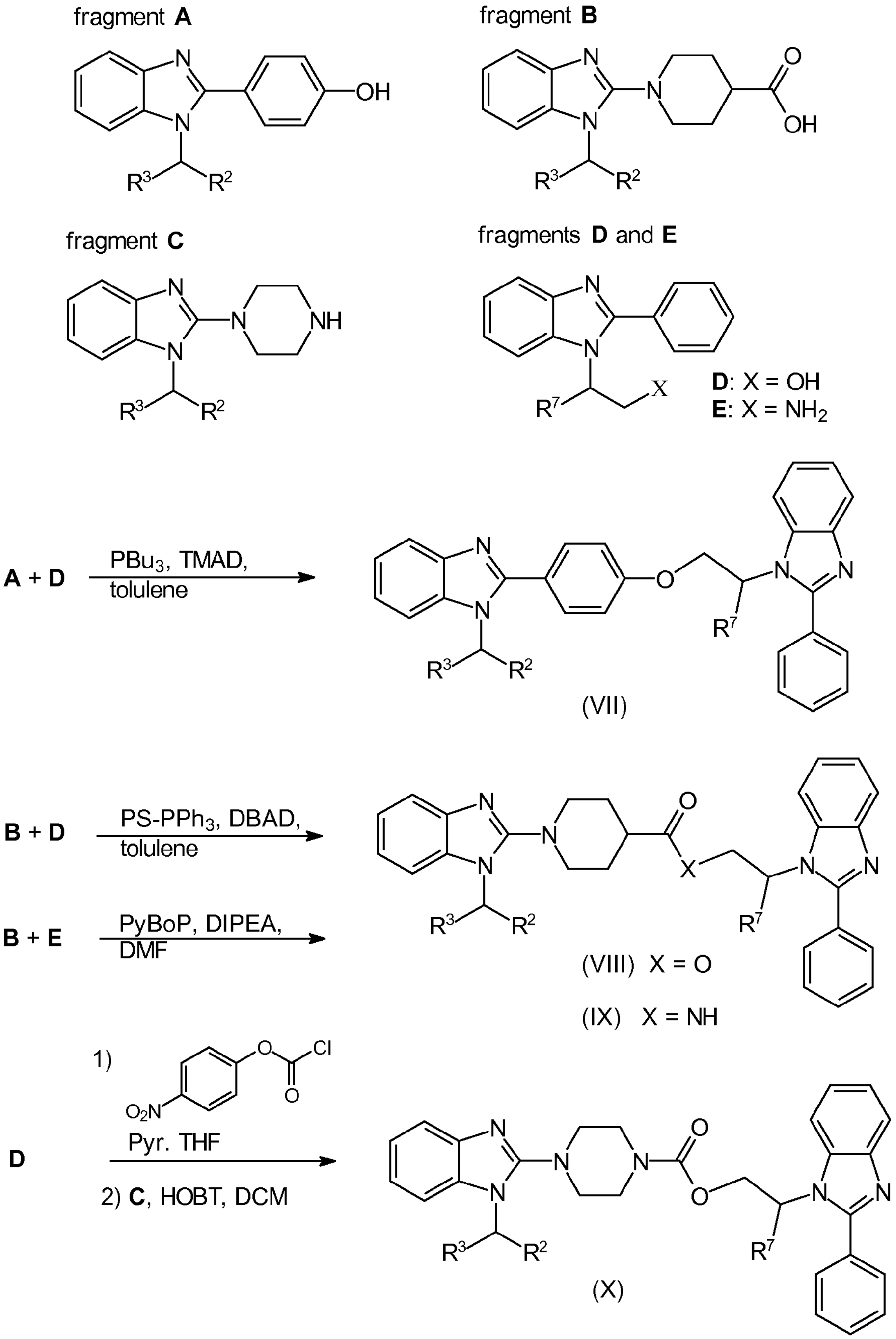
fragment A
fragment B
fragment C
fragments D and E
D: X = OH
E: X = NH2
A + D
PBu3, TMAD, toluene
(VII)
B + D
PS-PPh3, DBAD, toluene
B + E
PyBoP, DIPEA, DMF
(VIII) X = O
(IX) X = NH
D
1)
Pyr. THF
2) C, HOBT, DCM
(X)

scheme 2.

**Enantioselective synthesis of linked benzimidazoles**

The enantioselective synthesis is important for these inventive compounds, where the single enantiomers display enantiomer specific biological activities, for strong inhibition of the PDEδ/Ras interaction at low nano molar concentrations and inhibition of proliferation in K-Ras dependent cell lines at micro molar concentrations.

By these enantioselective syntheses which are described in more detail below, an enantiomeric excess (ee) of at least 80%, more preferably of at least 85%, still more preferably of at least 90% and most preferably of at least 95% can be obtained.

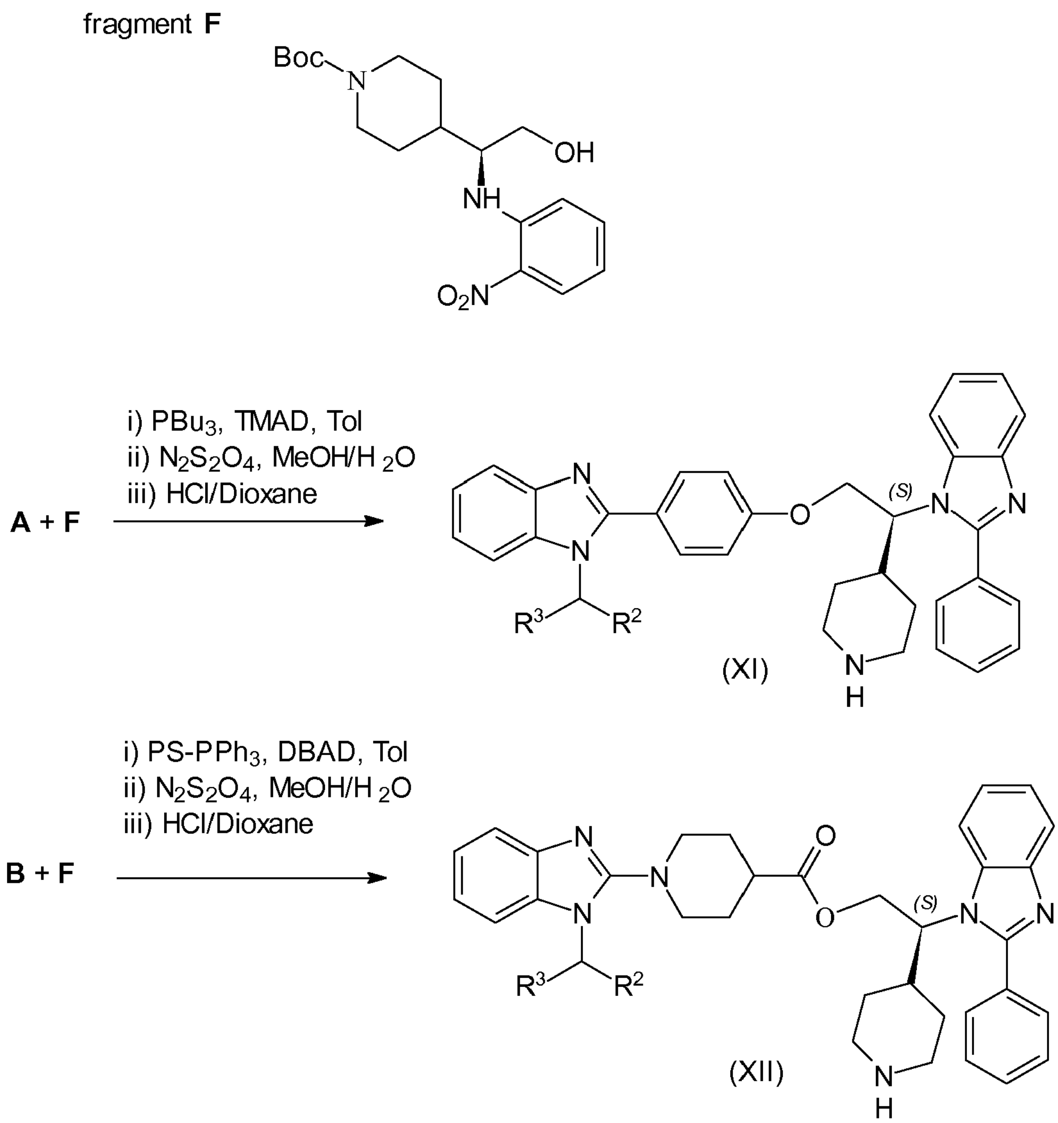


**scheme 3.**

As depicted in scheme 3 a enantiopure synthesized fragment F can be coupled with fragments A and B under the coupling conditions. The reduction of a nitro group, following intramolecular condensation and deprotection give the (S)-configurated enantiopure compounds XI and XII. In such embodiment the residue $R^2$ represents phenyl or thiophen-3-yl.

The present invention also comprises pharmaceutically acceptable salts of the compounds according to the general formula (I), all stereoisomeric forms of the compounds according to the general formula (I) as well as solvates, especially hydrates or prodrugs thereof.

The compounds of the present invention may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid,

succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

In the case the inventive compounds bear acidic groups, salts could also be formed with inorganic or organic bases. Examples for suitable inorganic or organic bases are, for example, NaOH, KOH, $NH_4OH$, tetraalkylammonium hydroxide, lysine or arginine and the like. Salts may be prepared in a conventional manner using methods well known in the art, for example by treatment of a solution of the compound of the general formula (I) with a solution of an acid, selected out of the group mentioned above.

Some of the compounds of the present invention may be crystallised or recrystallised from solvents such as aqueous and organic solvents. In such cases solvates may be formed. This invention includes within its scope stoichiometric solvates including hydrates as well as compounds containing variable amounts of water that may be produced by processes such as lyophilisation.

The compounds of the general formula (I) exist in the form of optical isomers, i.e. enantiomers and mixtures of said isomers in all ratios, e.g. racemic mixtures. The invention includes all such forms, in particular the pure isomeric forms or enantiomeric forms. The different isomeric forms may be separated or resolved one from the other by conventional methods, or any given isomer may be obtained by conventional synthetic methods or by stereospecific or asymmetric syntheses. Where a compound according to the general formula (I) contains an alkene moiety, the alkene can be presented as a cis or trans isomer or a mixture thereof. When an isomeric form of a compound of the invention is provided substantially free of other isomers, it will preferably contain less than 5% w/w, more preferably less than 2% w/w and especially less than 1 % w/w of the other isomer(s).

Another aspect of the present invention relates to the use of the inventive substituted benzimidazoles as drugs, i.e. as pharmaceutically active agents applicable in medicine.

Surprisingly it was found that the above-mentioned benzimidazoles as well as the pharmaceutical compositions comprising said benzimidazoles are useful for treatment or prophylaxis of cancer, tumors and proliferative diseases.

Thus, the benzimidazole compounds of the present invention can be used for prophylaxis and treatment of cancer, tumors and proliferative diseases or for the preparation of a pharmaceutical formulation for prophylaxis and treatment of cancer, tumors and proliferative diseases.

Examples for cancer types which can be treated and/or prevented by the inventive compounds are selected from the group comprising or consisting of adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, non-small cell lung cancer (NSCLC), breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioma, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma and tongue cancer.

Cancer cells often depend on the survival signaling emanating from oncogene products, particularly from oncogenic KRas, for their survival. The induction of programmed cell death by the loss of such survival signaling as disclosed for the compounds of the present invention is especially useful in the treatment of cancer by inducing the death of oncogenic Ras depedent malignant cells. Since all kinds of cancer cells are destroyable through the induction of programmed cell death, all different kinds of cancer and abnormal proliferating cells can be treated with the compounds

of the present invention.

Therefore, another aspect of the present invention is directed to pharmaceutical compositions comprising at least one compound of the present invention as active ingredient, together with at least one pharmaceutically acceptable carrier, excipient and/or diluents. The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present invention containing at least one compound according to the present invention, and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, extrudates, deposits, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95 weight % of the benzimidazole derived compound and/or the respective pharmaceutically active salt as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimise the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration. Aerosol preparations suitable for inhalation may include solutions and solids in powder form, which may be present in combination with a pharmaceutically acceptable carrier such as an inert, compressed gas, e.g. nitrogen. For preparing suppositories, a low melting fat or wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations which are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methyl cellulose, polyvinyl alcohols, or denatured gelatins or starch for holding or containing compositions comprising the active ingredient (s). Capsules with hard shells are typically made of blended of relatively high gel strength gelatins from bones or pork skin. The capsule itself may contain small amounts of dyes, opaquing agents, plasticisers and/or preservatives. Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix. Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn

rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to about 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat corn rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 weight %.

Said pharmaceutical compositions further comprise at least one active benzimidazole of the general formula (I).

**Description of the figures:**

**Figure 1:** Inhibitory effect of compound **1** at a 20 μM concentration on interaction of Rheb (Ras homolog enriched in brain) with PDEδ and localization in live MDCK cells. To address whether the novel compounds that target the farnesyl binding pocket in PDEδ inhibit the interaction between PDEδ and Ras family proteins in live cells, fluorescence lifetime imaging microscopy (FLIM)-based quantitative fluorescence resonance energy transfer (FRET) measurements were carried out. These experiments allow the quantitative determination of the fraction (α) of interacting mCitrine (a yellow fluorescent protein) tagged Ras proteins with mCherry (a red fluorescent protein) tagged PDEδ in living cells by the change in the fluorescence lifetime ($\tau_{av}$) of the mCitrine fused to Ras. mCitrine-Rheb was selected as the farnesylated Ras family protein due to its more clearly measurable interaction with mCherry-PDEδ in live cells. The fluorescence patterns of both mCitrine-Rheb (first row) and mCherry-PDEδ (second row) were homogeneous in untreated cells (Pre) showing a clear solubilization of mCitrine-Rheb by mCherry-PDEδ. Under these conditions a substantial drop in the mCitrine fluorescence lifetime (third row) corresponds to a substantial fraction (fourth row) of mCitrine-Rheb that was in complex with mCherry-PDEδ. This interaction as measured by FLIM is lost upon incubation with compound **1** leading to an increased average fluorescence lifetime of mCitrine and reduced computed fraction (α) of interacting PDEδ-Rheb over time.

**Figure 2:** Inhibitory effect of compound (*S*)-9 at a 5 μM concentration on interaction of Rheb with PDEδ and localization in live MDCK cells.

**Figure 3:** Quantification of the inhibition of Rheb -PDEδ interaction as measured by FLIM (see figure 1 and 2) in live MDCK cells by compounds **1** (blue line) and (*S*)-**9** (red line). Upon addition of 20 μM of compound **1** the interaction between mCitrine-Rheb and mCherry-PDEδ was reduced in the course of 30 minutes. The interaction between mCitrine-Rheb and mCherry-PDEδ was more rapidly (within one minute) and more strongly reduced after addition of 5 μM of compound *(S)*-**9**.

**Figure 4:** Real time cell analysis (RTCA) of PANC-TUI cell proliferation. PANC-TUI is a K-Ras dependent pancreatic tumor cell. Vehicle control, DMSO curve shows as the control experiment the normal proliferation of PANC-TUI cells without inhibitor. After addition of 5μM (*S*)-**9** K-Ras dependent PANC-TUI cells stopped proliferating and underwent cell death within a few hours. [A.U.] is an arbitrary electrical impedance unit. An Arrow shows the time of *(S)*-**9** administration to the cells.

**Figure 5:** RTCA of CAPAN-1 cells. CAPAN-1 cell is an another K-Ras dependent pancreatic tumor cell. Proliferation of CAPAN-1 cells was inhibited strongly by the PDEδ-inhibitor (*S*)-**9**.

**Figure 6:** Attenuation of proliferative/survival ERK signaling in PANC-TUI cells by the PDEδ-inhibitor (*S*)-**9**. Ras proteins are involved in the activation of Erk (Extracellular signal-regulated kinase) that transmits proliferative and survival signals in the cell. Thus, attenuation of Erk activity as measured by its phosphorylation is evidence for inhibition of Ras mediated proliferative/survival cell signaling. EGF (Epidermal growth factor) is a growth factor that stimulates cell growth, proliferation and survival. Left bars show the activation of Erk under control conditions (without inhibitor). Right bars show the attenuated activation of Erk with the treatment of inhibitor (*S*)-**9** at a 5 μM concentration. Each bar represents the mean value.

**Figure 7:** Time series of mCherry-K-Ras redistribution upon application of 5μM of (*S*)-**9** in human pancreatic ductal adenocarcinoma cells; Panc1 cells (upper row) and PancTul cells (lower row). Time in minutes after administration of (*S*)-**9** is indicated above the images. This experiment shows that inhibition of PDEδ-KRas interactions by (*S*)-**9** leads to a loss of K-Ras spatial organization as maintained by the solubilizing activity of PDEδ. The randomized distribution of Ras over all membranes in the cell leads to a reduced coupling of KRas to its effectors at the plasma membrane and thereby reduces both proliferative and survival signals in cells.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description.

Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

## EXAMPLES

Abbreviations used in the present description have the following meanings: ACN (acetonitrile), Boc (tert-butyloxycarbonyl), DCM (dichloromethane), $CDCl_3$ (deuterated chloroform), DBAD (Dibenzyl azodicarboxylate), DIPEA (N-ethyl-N,N-diisopropylamine), DMAP (4-(Dimethylamino)pyridine), DMF (dimethylformamide), DMSO-$d_6$ (deuterated dimethylsulfoxide), EtOH (ethanol), FITC (Fluorescein isothiocyanate), HEPES (2-(4-(2-Hydroxyethyl)-1-piperazinyl)-ethansulfonic aicd), HOBT (hydroxybenzotriazole), MeOH (methanol), MeCN (acetonitrile), NMP (N-Methyl-2-pyrrolidone), Ph (phenyl), PBS (phosphate buffered saline), $PBu_3$ (tributylphosphine), PEG (poly ethylene glycol), $Pd(PPh_3)_4$ (tetrakis(triphenylphosphine)palladium(0)), PS-$PPh_3$ (Polystyrene-supported triphenylphosphine), PyBoP (benzotriazol-1-yl-oxytripyrrolidinophosphonium hexafluorophosphate), Pyr (pyridine), TFA (trifluoroacetic acid), THF (tetrahydrofurane), TMAD (tetramethylazodicarboxamide).

## PREPARATIVE EXAMPLES

### General considerations.

NMR spectroscopic data were recorded on a 400 MHz or 600 MHz instrument at room temperature. NMR spectra were calibrated to the solvent signals of deuterated DMSO-$d_6$ or $CDCl_3$. The following abbreviations are used to indicate the signal multiplicity: s (singlet), d (doublet), t (triplet), q (quartet), br (broad), m (multiplet). Analytical HPLC-MS data were recorded on a HPLC system with a C18 reverse column coupled to an ESI spectrometer, flow rate: 1.0 mL/min; time: 15 min; solvent A: 0.1% HCOOH in water, Solvent B: 0.1% HCOOH in Acetonitrile; 1 min 10% B, in 10 min to 100% B. High resolution mass spectra (HR-MS) were measured using electrospray ionization (ESI). Chiral HPLC analysis was performed on a Chiralpak IC Column 250 mm x 4.6 mm, flow rate: 0.5 mL/min; ethanol in *iso*-hexane. Preparative HPLC separations were carried out using a reversed-phase C18 column (RP C18, flow 20.0 mL/min, solvent A: 0.1% TFA in water, solvent B: 0.1% TFA in Acetonitrile, from 10 % B to 100 % B.

### General Procedure A for the N-alkylation of benzimidazoles

To a suspension of benzimidazole in acetonitrile (1 mL/ 0.1 mM benzimidazole) was added caesium carbonate (1.5 eq) and the corresponding alkyl bromide (1.05 eq). The reaction mixture was stirred at room temperature for 1-3 hours and after this time it was concentrated *in vacuo.* The residue was suspended in a mixture of $CH_2Cl_2$ and sat. $NaHCO_3$ (vol % 50:50, 10 mL/0.1 mmol benzimidazole). The aqueous layer was extracted with $CH_2Cl_2$ (2 x, 10 mL/0.1 mmol benzimidazole). The combined organic layers were dried over $Na_2SO_4$, filtered and the solvent was removed under reduced pressure. The crude product was purified by flash chromatography as described below.

### General Procedure B for the deprotection of allyl-protected phenols

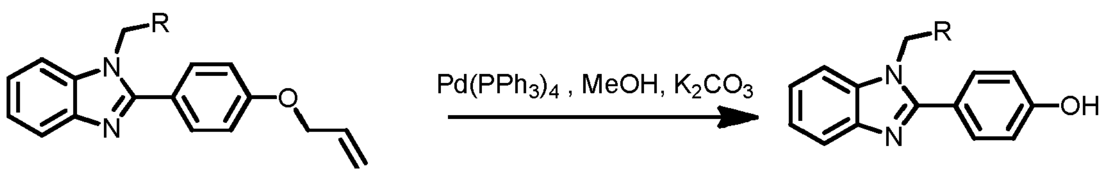


The allyl-protected phenol (1eq) was dissolved in degassed methanol (5 mL/mmol protected phenol). To this mixture was added $K_2CO_3$ (3 eq) and $Pd(PPh_3)_4$ (0.2 eq). The reaction mixture was stirred at room temperature for 1-3 hours and after this time it was concentrated *in vacuo.* The residue was resuspended in $CH_2Cl_2$ (10 mL/mmol) and a sat. $NH_4Cl$-solution was subsequently added. The pH of the aqueous layer was adjusted to 8 and the aqueous layer was extracted with $CH_2Cl_2$ (3 x 20 mL/mmol phenol). The combined organic layers were dried over $Na_2SO_4$, filtered and concentrated *in vacuo.* The crude product was purified as described below.

### General Procedure C for the synthesis of phenol ethers via Mitsunobu reaction

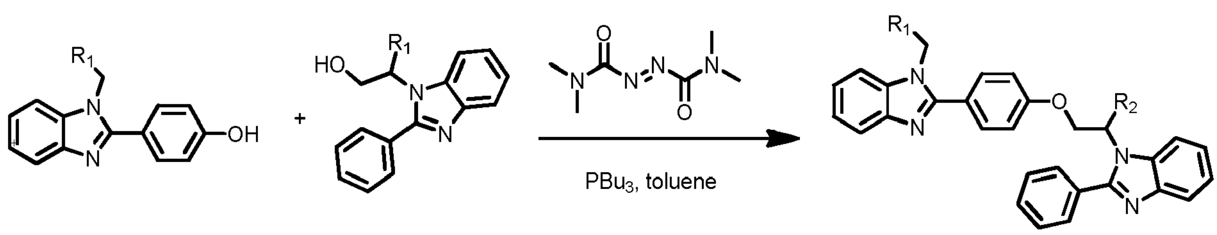


To a mixture of phenol, alcohol (1.0 eq) and tri-*n*-butyl phosphine (2 eq) in toluene (3 mL/mmol phenol) was added at 0°C TMAD (2 eq) in one portion. The resulting suspension was heated for 16 h at 60°C. If the reaction was not complete tri-*n*-butyl phosphine (1 eq) and TMAD (1 eq) were added at room temperature and the mixture was heated for 16 h at 60°C. The reaction mixture was diluted with $CH_2Cl_2$ (20 mL/mmol phenol) and washed with sat. $NaHCO_3$ (10

mL /mmol phenol). The aqueous phase was reextracted with $CH_2Cl_2$ (3 x 20 mL/mmol phenol). The combined organic phases were dried ($Na_2SO_4$), filtered and concentrated *in vacuo.* The crude product was purified by column chromatography as described below.

**General Procedure D for the Boc-deprotection of piperidine-containing benzimidazoles**

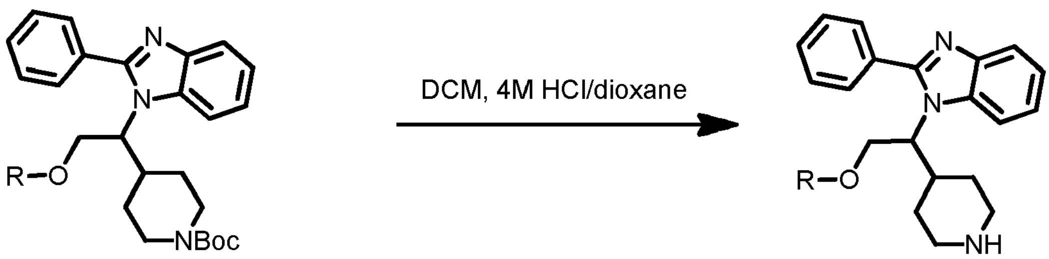


To a solution of Boc-protected piperidine in DCM (1 mL / 20 μM protected piperidine) was added a 4 M HCl/dioxane solution to a final concentration of 2 M HCl. The reaction mixture was stirred at room temperature for 3 h. The solvent was removed *in vacuo* to afford the product in quantitative yield as an HCl-salt. Further purification, if required, is described below.

**General Procedure E for esterification of two benzimidazole units**

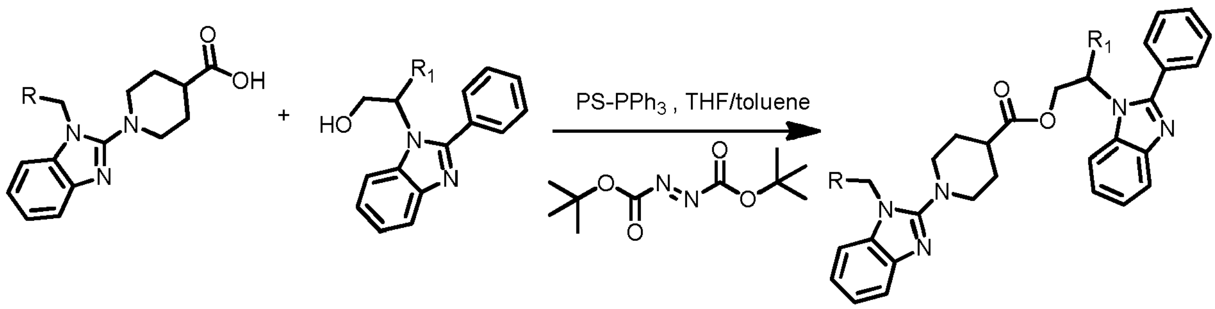


To a mixture of carboxylic acid, alcohol (1.0 eq) and polymer bound triphenylphosphine (3 meq) in THF/toluene (1:1, 3 mL/ mmol benzimidazole) was added at 0°C di-*tert*-butyl azodicarboylate (3eq). The reaction mixture is stirred at room temperature for 16 h, diluted with $CH_2Cl_2$ (15 mL/mmol benzimidazole) and filtered. The filtrate was concentrated *in vacuo* and purified by automated flash chromatography as described below.

**Example 01: Preparation of 2-(4-(Allyloxy)phenyl)-1H-benzo[d]imidazole (S1)**

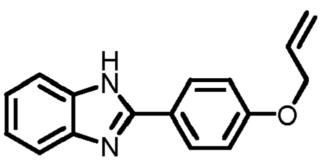

To a mixture of 4-allyloxybenzaldehyde (4.00 g, 23.9 mmol) and phenylendiamine (2.72 g, 25.1 mmol) in DMF (50 mL) at room temperature was added $Na_2S_2O_5$ (4.50 g, 23.9 mmol). The reaction mixture was heated at 70 °C for 3 h. To the reaction mixture was added ice water (150 mL) and the resulting suspension was stirred for 2 h at 0°C. The slightly brown precipitate was filtered, washed with cold water (100 mL), ethyl acetate (3x 20 mL) and dried *in vacuo* to

afford the product. (4.85 g, 19.8 mmol, 81 %).
$^1H$ NMR (400 MHz, DMSO-$d_6$) δ 8.11 (d, *J* = 8.7 Hz, 2H), 7.56 (dd, *J* = 5.9, 3.1 Hz, 2H), 7.23 — 7.06 (m, 4H), 6.11-5.99 (m, 1H), 5.41 (d, *J* = 16.8 Hz, 1H), 5.27 (d, *J* = 10.6 Hz, 1H), 4.64 (d, *J* = 4.7 Hz, 2H); $^{13}C$ NMR (101 MHz, DMSO-$d_6$) δ 160.3, 151.9, 139.7, 134.1, 128.8, 123.1, 122.6, 118.3, 115.8, 115.4, 69.02; LC-MS (ESI): calcd for $C_{16}H_{14}N_2O$: 251.11789 $[M+H]^+$, found 251.13 $[M+H]^+$, $R_t$ = 5.84 min; HR-MS found 251.11800 $[M+H]^+$.

**Example 02: Preparation of 2-(4-(Allyloxy)phenyl)-1-benzyl-1H-benzo[d]imidazole (S2)**

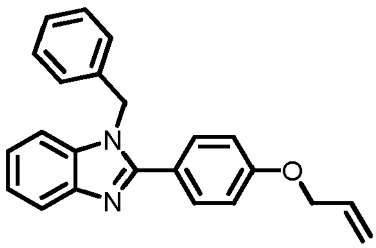

Compound **S2** was synthesized according to Procedure (A) for the synthesis of N-alkylated benzimidazoles starting from **S1** (262 mg, 1.04 mmol). The crude product was purified by automated flash chromatography using a gradient of cyclohexane : ethyl acetate 10:1 to 1:2 to yield the benzimidazole (325 mg, 0.95 mmol, 91%). $^1H$ NMR (400 MHz, DMSO-$d_6$) δ 7.70 - 7.60 (m, 3H), 7.43 - 7.37 (m, 1H), 7.30 - 7.14 (m, 5H), 7.07 (d, *J* = 8.8 Hz, 2H), 6.99 (d, *J* = 7.4 Hz, 2H), 6.11 - 5.93 (m, 1 H), 5.55 (s, 2H), 5.40 (dd, *J* = 17.3, 1.7 Hz, 1H), 5.26 (dd, *J* = 10.5, 1.7 Hz, 1H), 4.62 (d, *J* = 5.2 Hz, 2H). $^{13}C$ NMR (101 MHz, DMSO-$d_6$) δ 160.00, 153.89, 143.40, 137.68, 136.61, 134.08, 131.13, 129.47, 128.12, 126.72, 123.14, 123.07, 122.75, 119.72, 118.40, 115.60, 111.59, 68.99, 48.14. LC-MS (ESI): calcd for $C_{23}H_{20}N_2O$: 341.16484 $[M+H]^+$, found: 341.25 $[M+H]^+$, $R_t$ = 7.43 min; HR-MS found 341.16508 $[M+H]^+$.

**Example 03: Preparation of 2-(4-(Allyloxy)phenyl)-1-(pyridin-2-ylmethyl)-1H-benzo[d]imidazole (S3)**

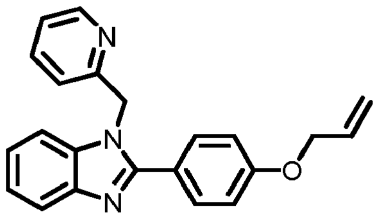

The product was synthesized according to Procedure A for the N-alkylation of benzimidazoles starting from **S1** (384 mg, 1.53 mmol). The crude product was purified by automated flash chromatography (gradient $CH_2Cl_2$ : MeOH 50:1 to 20:1) to yield the product (83%, 1.27 mmol, 432 mg).
$^1H$ NMR (400 MHz, DMSO-$d_6$) δ 8.45 - 8.39 (m, 1H), 8.29 - 8.24 (m, 1H), 7.71 - 7.62 (m, 3H), 7.53 - 7.46 (m, 1H), 7.31 - 7.16 (m, 4H), 7.08 (t, *J* = 5.8 Hz, 2H), 6.10 - 5.98 (m, 1H), 5.61 (s, 2H), 5.41 (dd, *J* = 17.3, 1.7 Hz, 1H), 5.27 (dd, *J* = 10.5, 1.7 Hz, 1H), 4.63 (d, *J* = 5.2 Hz, 2H). $^{13}C$ NMR (101 MHz, DMSO-$d_6$) δ 160.04, 153.86, 149.45, 148.47, 143.40, 136.40, 134.66, 134.08, 133.30, 131.21, 124.47, 123.22, 123.02, 122.90, 119.81, 118.43, 115.66, 111.54, 69.00, 45.91. LC-MS (ESI): 342.18 $[M+H]^+$, $R_t$ = 5.95 min.

**Example 04: Preparation of 2-(4-(Allyloxy)phenyl)-1-(2-methylbenzyl)-1H-benzo[d]imidazole (S4)**

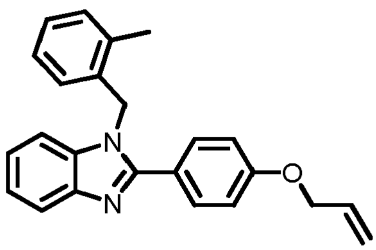

The product was synthesized according to Procedure A for the N-alkylation of benzimidazoles starting from **S1** (291 mg, 1.27 mmol). The crude product was purified by automated flash chromatography (gradient cylohexane : ethyl acetate 10:1 to 1:2) to yield the product (387 mg, 1.09 mmol, 86%).
$^1$H NMR (400 MHz, DMSO-$d_6$) δ 7.71 (dd, *J* = 7.9, 0.6 Hz, 1H), 7.61 - 7.54 (m, 2H), 7.30 (dd, *J* = 7.9, 0.7 Hz, 1H), 7.27 - 7.08 (m, 5H), 7.07 - 6.97 (m, 3H), 6.37 (d, *J* = 7.7 Hz, 1H), 6.03 (ddt, *J* = 17.3, 10.5, 5.3 Hz, 1H), 5.49 (s, 2H), 5.46 - 5.31 (m, 1H), 5.25 (dd, *J* = 10.5, 1.7 Hz, 1H), 4.63 - 4.54 (m, 2H), 2.29 (s, 3H). $^{13}$C NMR (101 MHz, DMSO-$d_6$) δ 160.01, 153.97, 143.37, 136.75, 135.62, 135.55, 134.06, 131.05, 130.84, 127.88, 126.93, 125.11, 123.07, 123.04, 122.79, 119.73, 118.41, 115.59, 111.53, 68.97, 46.56, 19.34; LC-MS (ESI): calcd for $C_{24}H_{22}N_2O$: 355.18049 [M+H]$^+$, found: 355.19 [M+H]$^+$, $R_t$ = 7.75 min; HR-MS found 355.18092 [M+H]$^+$.

**Example 05: Preparation of 2-(4-(Allyloxy)phenyl)-1-(3-methylbenzyl)-1H-benzo[d]imidazole (S5)**

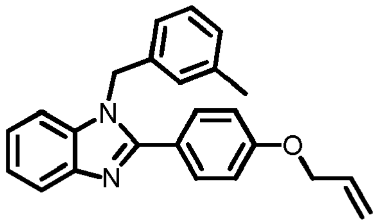

Compound **S5** was synthesized according to Procedure A for the N-alkylation of benzimidazoles starting from **S1** (316 mg, 1.26 mmol). The crude product was purified by automated flash chromatography (gradient cyclohexane : ethyl acetate 10:1 to 1:2) to yield the product (394 mg, 1.11 mmol, 88%).
$^1$H NMR (400 MHz, DMSO-$d_6$) δ 7.72 - 7.58 (m, 3H), 7.38 (dt, *J* = 6.7, 3.7 Hz, 1H), 7.25 — 7.11 (m, 3H), 7.09-7.03 (m, 3H), 6.85 (s, 1H), 6.74 (d, *J* = 7.6 Hz, 1H), 6.12 - 5.94 (m, 1H), 5.50 (s, 2H), 5.40 (dd, *J* = 17.3, 1.7 Hz, 1H), 5.26 (dd, *J* = 10.5, 1.7 Hz, 1H), 4.63-4.60 (m, 2H), 2.19 (s, 3H). $^{13}$C NMR (101 MHz, DMSO-$d_6$) δ 160.00, 153.89, 143.38, 138.65, 137.63, 136.62, 134.09, 131.13, 129.40, 128.82, 127.31, 123.75, 123.15, 123.06, 122.74, 119.70, 118.40, 115.61, 111.58, 68.99, 48.13, 21.68. LC-MS (ESI): calcd for $C_{24}H_{22}N_2O$: 355.18049 [M+H]$^+$, found: 355.23 [M+H]$^+$, $R_t$ = 7.67 min; HR-MS found 355.18107 [M+H]$^+$.

**Example 06: Preparation of 2-(4-(Allyloxy)phenyl)-1-(4-fluorobenzyl)-1H-benzo[d]imidazole (S6)**

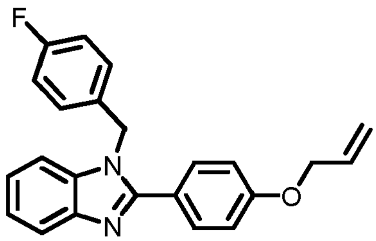

Compound **S6** was synthesized according to Procedure A for the N-alkylation of benzimidazoles starting from **S1** (243 mg, 0.97 mmol). The crude product was purified by automated flash chromatography (gradient cyclohexane : ethylacetate 10:1 to 1:2) to yield the product (301 mg, 0.84 mmol, 86%).

$^{1}H$ NMR (400 MHz, DMSO-$d_6$) δ 7.72 - 7.58 (m, 3H), 7.46 - 7.37 (m, 1H), 7.24 - 7.16 (m, 2H), 7.14 - 6.93 (m, 6H), 6.05 (ddt, *J* = 17.3, 10.5, 5.2 Hz, 1H), 5.54 (s, 2H), 5.44 — 5.36 (m, 1H), 5.29 — 5.23 (m, 1H), 4.67 — 4.57 (m, 2H). $^{13}C$ NMR (101 MHz, DMSO-$d_6$) δ 163.26, 160.84, 160.01, 153.82, 143.40, 136.49, 134.08, 133.86, 131.15, 128.89, 128.81, 123.13, 123.08, 122.80, 119.75, 118.41, 116.40, 116.19, 115.62, 111.56, 68.99, 47.48. LC-MS (ESI): calcd for $C_{23}H_{19}FN_2O$ : 359.15542 [M+H]$^+$, found: 359.19 [M+H]$^+$, $R_t$ = 7.41 min; HR-MS found 359.15576 [M+H]$^+$.

**Example 07: Preparation of 4-(1-Benzyl-1H-benzo[d]imidazol-2-yl)phenol (S7)**

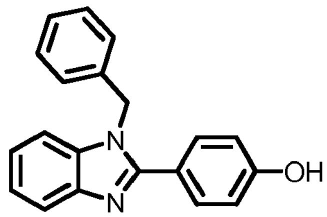

Compound **S7** was synthesized according to Procedure B for the allyl-deprotection of benzimidazoles. The compound was purified by automated flash chromatography using a gradient of cyclohexane: ethyl acetate 20:1 to 1:1 to yield compound **S7** (143 mg, 0.47 mmol, 83%).

$^{1}H$ NMR (400 MHz, DMSO-$d_6$) δ 9.93 (s, 1H), 7.72 - 7.59 (m, 1H), 7.54 (d, *J* = 8.6 Hz, 2H), 7.41 - 7.33 (m, 1H), 7.32 - 7.12 (m, 5H), 6.99 (d, *J* = 7.4 Hz, 2H), 6.87 (d, *J* = 8.7 Hz, 2H), 5.53 (s, 2H); $^{13}C$ NMR (101 MHz, DMSO-$d_6$) δ = 160.3, 151.9, 139.7, 134.1, 128.8, 123.1, 122.6, 118.3, 115.7, 115.4, 69.0 LC-MS (ESI): calcd for $C_{20}H_{16}N_2O$ : 301.13354 [M+H]$^+$, found: 301.18 [M+H]$^+$, $R_t$ = 5.89 min; HR-MS found 301.13367 [M+H]$^+$.

**Example 08: Preparation of 4-(1-(Pyridin-2-ylmethyl)-1H-benzo[d]imidazol-2-yl)phenol (S8)**

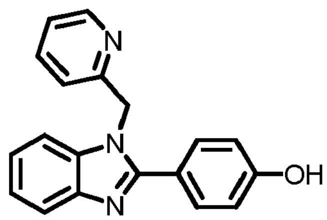

Compound **S8** was synthesized according to Procedure B for the allyl-deprotection of benzimidazoles. The crude product was purified by automated flash chromatography using a gradient of $CH_2Cl_2$: MeOH 100:1 to 10:1 to yield compound the product (123 mg, 0.41 mmol, 80%).

$^{1}H$ NMR (400 MHz, DMSO-$d_6$) δ 9.95 (s, 1H), 8.42 (dd, *J* = 4.3, 1.8 Hz, 1H), 8.27 (s, 1H), 7.71 - 7.60 (m, 1H), 7.56 - 7.49 (m, 2H), 7.50 - 7.44 (m, 1H), 7.31 - 7.24 (m, 2H), 7.24 - 7.15 (m, 2H), 6.95 - 6.83 (m, 2H), 5.60 (s, 2H). $^{13}C$ NMR (101 MHz, DMSO-$d_6$) δ 159.59, 154.30, 149.41, 148.49, 143.42, 136.37, 134.68, 133.36, 131.27, 124.45, 123.04, 122.80, 121.25, 119.69, 116.30, 111.46, 45.89. LC-MS (ESI): 302.13 [M+H]$^+$, $R_t$ = 4.13 min.

**Example 09: Preparation of 4-(1-(4-Fluorobenzyl)-1H-benzo[d]imidazol-2-yl)phenol (S9)**

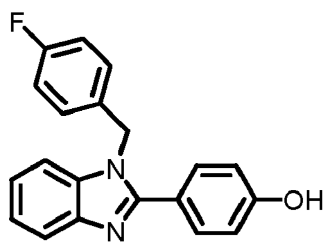

Compound **S9** was synthesized according to Procedure B for the allyl-deprotection of benzimidazoles. The crude product was purified by automated flash chromatography using a gradient of of cyclohexane: ethyl acetate 20:1 to 1:1 to yield compound **S9** (156 mg, 0.49 mmol, 84%).

$^1$H NMR (400 MHz, DMSO-*d*$_6$) δ 9.94 (s, 1H), 7.69 - 7.63 (m, 1H), 7.56 - 7.48 (m, 2H), 7.44 — 7.37 (m, 1H), 7.24 - 7.14 (m, 2H), 7.10 (t, *J* = 8.8 Hz, 2H), 7.06 - 6.98 (m, 2H), 6.87 (d, *J* = 8.4 Hz, 2H), 5.52 (s, 2H). $^{13}$C NMR (101 MHz, DMSO-*d*$_6$) δ 163.24, 160.82, 159.56, 154.27, 143.42, 136.46, 133.93, 133.90, 131.22, 128.91, 128.83, 122.95, 122.69, 121.31, 119.62, 116.37, 116.25, 116.16, 111.48, 47.45. LC-MS (ESI): calcd for $C_{20}H_{15}FN_2O$: 319.12412 [M+H]$^+$, found: 319.11 [M+H]$^+$, $R_t$ = 6.21 min; HR-MS found 319.12438 [M+H]$^+$.

**Example 10: Preparation of 4-(1-(2-Methylbenzyl)-1H-benzo[d]imidazol-2-yl)phenol (S10)**

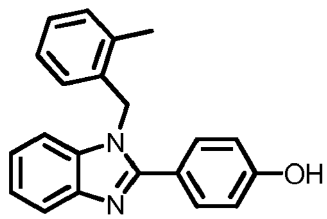

Compound **S10** was synthesized according to Procedure B for the allyl-deprotection of benzimidazoles. The crude product was purified by automated flash chromatography using a gradient of of cyclohexane: ethyl acetate 20:1 to 1:1 to yield compound **S10** (160 mg, 0.51 mmol, 81 %).

$^1$H NMR (400 MHz, DMSO-*d*$_6$) δ 9.92 (s, 1H), 7.68 (dd, *J* = 7.9, 0.5 Hz, 1H), 7.65 — 7.57 (m, 1H), 7.57 - 7.50 (m, 1H), 7.47 (t, *J* = 5.6 Hz, 2H), 7.32 - 7.10 (m, 5H), 7.01 (t, *J* = 7.5 Hz, 1H), 6.86 - 6.78 (m, 2H), 6.37 (d, *J* = 7.6 Hz, 1H), 5.46 (s, 2H), 2.28 (s, 3H).$^{13}$C NMR (101 MHz, DMSO-*d*$_6$) δ 159.56, 154.42, 143.39, 136.72, 135.67, 135.54, 132.68, 132.20, 132.11, 131.02, 130.94, 129.48, 129.36, 127.84, 126.89, 125.15, 122.88, 122.68, 121.30, 119.61, 116.21, 111.45, 46.53, 19.33. LC-MS (ESI): calcd for $C_{21}H_{18}N_2O$ : 315.14919 [M+H]$^+$, found: 315.09 [M+H]$^+$, $R_t$ = 6.29 min; HR-MS found 315.14919 [M+H]$^+$.

**Example 11: Preparation of 4-(1-(3-Methylbenzyl)-1H-benzo[d]imidazol-2-yl)phenol (S11)**

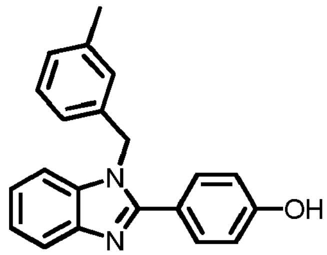

Compound **S11** was synthesized according to Procedure **B** for the allyl-deprotection of benzimidazoles. The crude product was purified by automated flash chromatography using a gradient of of cyclohexane: ethyl acetate 20:1 to 1:1 to yield compound **S11** (167 mg, 0.53 mmol, 83%).

$^1$H NMR (400 MHz, DMSO-*d*$_6$) δ 9.98 (s, 1H), 7.70 - 7.64 (m, 1H), 7.57 - 7.48 (m, 2H), 7.39 - 7.33 (m, 1H), 7.23 - 7.10 (m, 3H), 7.03 (d, *J* = 7.6 Hz, 1H), 6.92 - 6.83 (m, 3H), 6.75 (d, *J* = 7.5 Hz, 1H), 5.48 (s, 2H), 2.18 (s, 3H). $^{13}$C NMR (101 MHz, DMSO-*d*$_6$) δ 159.59, 154.36, 143.41, 138.63, 137.68, 136.60, 131.22, 129.36, 128.79, 127.35, 123.78, 122.89, 122.64, 121.40, 119.58, 116.26, 111.51, 48.13, 21.67. LC-MS (ESI): calcd for $C_{21}H_{18}N_2O$ : 315.14919 [M+H]$^+$, found: 315.12 [M+H]$^+$, $R_t$ = 6.33 min; HR-MS found 315.14939 [M+H]$^+$.

**Example 12: Preparation of N-Benzyl-2-cyclohexyl-2-(2-phenyl-1H-benzo[d]imidazol-1-yl)acetamide (S12)**

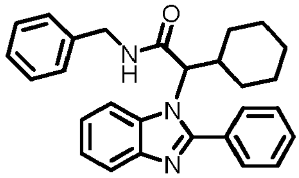

A mixture of the aniline (1.80 g, 9.0 mmol) and cyclohexyl carbaldehyde (1.10 g, 9.9 mmol) was stirred at room temperature in MeOH (4 mL) for 10 minutes. To the reaction mixture was added benzylisocyanide (1.10 mL, 9 mmol) and a solution of benzoic acid (1.10 g 9.0 mmol) in MeOH (4 mL). The reaction mixture was stirred at room temperature for 36 h. After this time HCl in dioxane (4 M, 2 mL) was added to the reaction mixture. The reaction mixture was stirred for 36 h at room temperature and then the solvent was removed under reduced pressure. The residue was redissolved in $CH_2Cl_2$ (20 mL) and washed with a sat. $NaHCO_3$ solution (30 mL). The aqueous layer was extracted with $CH_2Cl_2$ (3x 20 mL). The combined organic layers were dried ($Na_2SO_4$), filtered and concentrated to dryness under reduced pressure. The residue was purified by flash chromatography using a gradient of cyclohexane :ethyl acetate 50:1 to 1:2 to afford the product (3.42 g, 8.06 mmol, 89 %,).
$^1$H NMR (400 MHz, $CDCl_3$) δ 7.97 - 7.86 (m, 1H), 7.81 - 7.69 (m, 1H), 7.56 - 7.35 (m, 5H), 7.36 - 7.19 (m, 5H), 7.10 (dd, *J* = 6.8, 2.4 Hz, 2H), 6.13 (s, 1H), 4.50 - 4.29 (m, 3H), 2.76 - 2.56 (m, 1H), 1.96 (d, *J* = 12.3 Hz, 1H), 1.71 - 1.41 (m, 3H), 1.36 — 1.18 (m, 1H), 1.12 — 0.86 (m, 3H), 0.86 — 0.69 (m, 1H), 0.66 — 0.46 (m, 1H). $^{13}$C NMR (101 MHz, $CDCl_3$) δ 168.50, 154.93, 143.37, 137.77, 134.33, 130.74, 130.24, 129.92, 129.14, 128.96, 127.91, 123.51, 123.12, 120.18, 113.97, 66.39, 43.97, 37.84, 31.36, 29.18, 26.13, 25.81. LC-MS (ESI): calcd for $C_{28}H_{29}N_3O$: 424.23834 $[M+H]^+$, found 424.21 $[M+H]^+$; HR-MS found 424.23733 $[M+H]^+$.

**Example 13: Preparation of N-Benzyl-2-(2-phenyl-1H-benzo[d]imidazol-1-yl)-2-(piperidin-4-yl)acetamide (S13)**

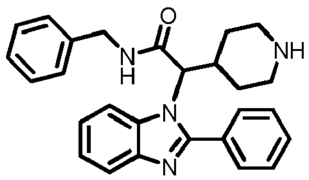

A mixture of the aniline (1.20 g, 5.7 mmol) and the *tert*-butyl 4-formylpiperidine-1-carboxylate (1.34 g, 6.3 mmol) was stirred at room temperature in MeOH (3 mL) for 10 minutes. To the reaction mixture was added benzylisocyanide (0.70 mL, 9.0 mmol) and a solution of benzoic acid (0.70 g 9.0 mmol) in MeOH (4 mL). The reaction mixture was stirred at room temperature for 36 h. To the reaction mixture was added a HCl/dioxane (4 M, 2 mL). The reaction mixture was stirred for 36 h at room temperature and then concentrated *in vacuo.* The residue was redissolved in $CH_2Cl_2$ (20 mL) and washed with sat. $NaHCO_3$ solution (30 mL). The aqueous layer was reextracted with $CH_2Cl_2$ (3x 20 mL). The combined organic layers were dried ($Na_2SO_4$), filtered and concentrated to dryness under reduced pressure. The residue was purified by automated flash chromatography (gradient cyclohexane : ethylacetate 100:1 to 1:2, containing 0.1% triethylamine) to afford the product (4.11 mmol, 1.75 g, 72%).
$^1$H NMR (400 MHz, DMSO-$d_6$) δ 8.89 (t, *J* = 5.6 Hz, 1H), 8.10 - 7.89 (m, 1H), 7.68 - 7.47 (m, 6H), 7.34 - 7.17 (m, 7H), 2.84 (d, *J* = 11.5 Hz, 1H), 2.67 - 2.51 (m, 2H), 2.37 (t, *J* = 12.4 Hz, 1H), 2.08 (t, *J* = 11.2 Hz, 1H), 1.60 (d, *J* = 13.0 Hz, 1H), 1.12 - 0.97 (m, 1H), 0.62 - 0.48 (m, 1H), 0.47 - 0.36 (m, 1H). $^{13}$C NMR (101 MHz, DMSO-$d_6$) δ 173.11, 155.19, 154.32, 152.61, 143.70, 138.15, 131.13, 130.49, 130.22, 129.37, 129.03, 127.91, 127.51, 123.29, 122.95, 120.05, 115.57, 84.79, 79.34, 63.36, 48.55, 37.71, 28.72, 27.85. LC-MS (ESI): calcd for $C_{27}H_{28}N_4O$: 425.23376 $[M+H]^+$, found 425.17 $[M+H]^+$, $R_t$ = 5.15 min; HR-MS found 425.23359 $[M+H]^+$.

**Example 14: Preparation of tert-Butyl benzyl(2-cyclohexyl-2-(2-phenyl-1H-benzo[d]imidazol-1-yl)acetyl)carbamate (S14)**

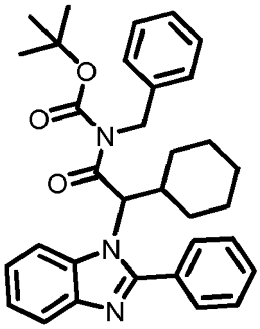

To a solution of amide **S12** (620 mg, 1.46 mmol) in $CH_2Cl_2$ (5 mL) was added Boc-anhydride (639 mg, 2.92 mmol), triethylamine (405 μL, 2.92 mmol) and DMAP (179 mg, 1.46 mmol). The reaction mixture was stirred at room temperature for 5 h. After this time Boc-anhydride (639 mg, 2.92 mmol), triethylamine (405 μL, 2.92 mmol) and DMAP (179 mg, 1.46 mmol) were added to the reaction mixture and stirring was continued for an additional 12 h. The reaction mixture was concentrated to dryness under reduced pressure. Purification by automated flash chromatography using a gradient of cyclohexane : ethyl acetate 20:1 to 1:1 afforded the product (0.66 g, 1.26 mmol, 86%).
$^1$H NMR (400 MHz, $CDCl_3$) δ 8.07 (d, *J* = 7.4 Hz, 1H), 7.82 - 7.73 (m, 1H), 7.56 - 7.48 (m, 2H), 7.47 - 7.39 (m, 3H), 7.34 - 7.24 (m, 2H), 7.24 - 7.15 (m, 3H), 7.05 (d, *J* = 7.2 Hz, 2H), 6.38 (d, *J* = 10.6 Hz, 1H), 4.83 (d, *J* = 15.0 Hz, 1H), 4.68 (d, *J* = 14.9 Hz, 1H), 2.87 (d, *J* = 10.8 Hz, 1H), 1.84 - 1.68 (m, 2H), 1.72 - 1.50 (m, 2H), 1.38 - 0.74 (m, 15H). $^{13}$C NMR (101 MHz, $CDCl_3$) δ 173.81, 155.26, 152.49, 143.59, 137.78, 134.92, 131.22, 130.10, 129.81, 128.75, 128.56, 127.55, 127.42, 123.03, 122.68, 120.04, 115.08, 84.19, 64.13, 48.53, 39.52, 29.87, 29.66, 27.95, 26.38, 26.25, 26.16. LC-MS (ESI): calcd for $C_{33}H_{37}N_3O_3$: 524.29077 [M+H]$^+$, found 524.18 [M+H]$^+$, $R_t$ = 11.55 min; HR-MS found 524.29035 [M+H]$^+$.

**Example 15: Preparation of tert-Butyl 4-(2-(benzyl(tert-butoxycarbonyl)amino)-2-oxo-1-(2-phenyl-1H-benzo[d]imidazol-1-yl)ethyl)piperidine-1-carboxylate (S15)**

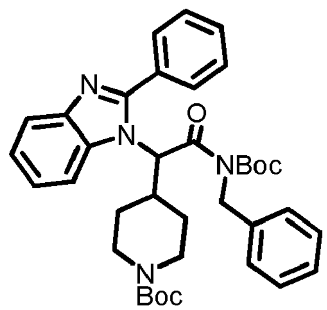

To a solution of amide **S14** (300 mg, 0.71 mmol) in $CH_2Cl_2$ (5 mL) was added Boc-anhydride (463 mg, 2.13 mmol), triethylamine (293 μL, 2.13 mmol) and DMAP (86 mg, 0.71 mmol). The reaction mixture was stirred at room temperature for 5 h. Boc-anhydride (463 mg, 2.13 mmol), triethylamine (293 μL, 2.13 mmol) and DMAP (86 mg, 0.71 mmol) were added to the reaction mixture and stirring was continued for an additional 12 h. The reaction mixture was concentrated to dryness under reduced pressure. Purification by flash chromatography using a gradient of cyclohexane: ethyl acetate 20:1 to 1:1 afforded the product (0.36 g, 0.58 mmol, 81%).
$^1$H NMR (400 MHz, DMSO-*d$_6$*) δ 8.04 (d, *J* = 7.5 Hz, 1H), 7.65 (d, *J* = 8.0 Hz, 1H), 7.55 - 7.37 (m, 5H), 7.31 - 7.10 (m, 5H), 7.04 (d, *J* = 7.2 Hz, 2H), 6.23 (d, *J* = 10.4 Hz, 1H), 4.77 (d, *J* = 15.5 Hz, 1H), 4.64 (d, *J* = 15.1 Hz, 1H), 3.95 (d, *J* = 11.9 Hz, 1H), 3.82 (d, *J* = 11.2 Hz, 1H), 2.97 (d, *J* = 10.2 Hz, 1H), 2.86 - 2.59 (m, 1H), 2.56 - 2.40 (m, 2H), 1.59 (d, *J* = 11.7 Hz, 1H), 1.47 - 1.24 (m, 10H), 1.25 - 1.05 (m, 9H), 1.05 - 0.88 (m, 2H). $^{13}$C NMR (101 MHz, DMSO-*d$_6$*) δ 173.11, 155.19, 154.32, 152.61, 143.70, 138.15, 131.13, 130.49, 130.22, 129.37, 129.03, 127.91, 127.51, 123.29, 122.95, 120.05, 115.57, 84.79, 79.34, 63.36, 48.55, 37.71, 28.72, 27.85. LC-MS (ESI): calcd for $C_{37}H_{44}N_4O_5$: 625.33845 [M+H]$^+$, found 625.19 [M+H]$^+$, $R_t$ = 10.76 min; HR-MS found 625.33840.

**Example 16: Preparation of 2-Cyclohexyl-2-(2-phenyl-1H-benzo[d]imidazol-1-yl)ethanol (S16)**

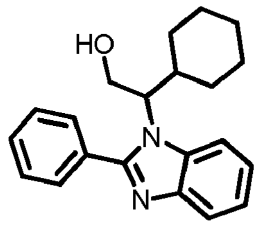

A solution of Boc-protected amide **S14** (650 mg, 1.24 mmol) in EtOH:THF (1:5, 5 mL) was added to a suspension of $NaBH_4$ (188 mg, 5 mmol) in THF (4 mL) at room temperature over 1 h. The reaction mixture was stirred at room temperature for 12 h. After this time $NaBH_4$ (94 mg, 2.5 mmol) was added to the reaction mixture and stirring was continued for 3 h. To the reaction mixture was added a $NH_4Cl$-solution (15 mL), followed by $CH_2Cl_2$ (20 mL). The pH of the aqueous layer was adjusted to 9 by addition of sat. $NaHCO_3$-solution. The aqueous layers were extracted with $CH_2Cl_2$ (2x 20 mL). The combined organic layers were dried ($Na_2SO_4$), filtered and concentrated under reduced pressure and the residue was purified by flash chromatography (gradient cyclohexane: ethyl acetate 10:1 to 1:1) to afford the product (330 mg, 1.03 mmol, 83%).

$^1H$ NMR (400 MHz, $CDCl_3$) δ 7.73 - 7.61 (m, 3H), 7.52 (d, *J* = 8.1 Hz, 1H), 7.48 - 7.35 (m, 3H), 7.25 - 7.10 (m, 2H), 4.36 - 4.21 (m, 2H), 4.06 - 4.02 (m, 1H), 3.13 (s, 1H), 2.18 - 2.05 (m, 1H), 1.80 (d, *J* = 11.9 Hz, 1H), 1.67 (d, *J* = 13.6 Hz, 1H), 1.53 (d, *J* = 10.0 Hz, 1H), 1.41 (d, *J* = 12.5 Hz, 1H), 1.31 - 1.10 (m, 1H), 1.04 - 0.77 (m, 4H), 0.57 - 0.37 (m, 1H). $^{13}C$ NMR (101 MHz, $CDCl_3$) δ 156.21, 143.34, 133.52, 130.86, 130.56, 129.69, 128.63, 122.62, 122.51, 120.24, 112.34, 65.04, 61.68, 37.67, 30.88, 29.62, 26.06, 25.87, 25.74. LC-MS (ESI): calcd for $C_{37}H_{44}N_4O_5$: 321.19614 $[M+H]^+$, found 321.18 $[M+H]^+$, $R_t$ = 6.24 min; HR-MS found 321.19642.

**Example 17: Preparation of tert-Butyl 4-(2-hydroxy-1-(2-phenyl-1H-benzo[d]imidazol-1-yl)ethyl)piperidine-1-carboxylate (S17)**

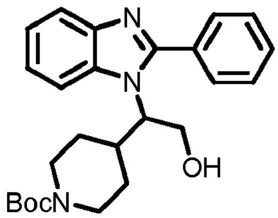

A solution of Boc-protected amide **S15** (1.0 g, 1.6 mmol) in EtOH:THF (1:5, 15 mL) was added to a suspension of $NaBH_4$ (242 mg, 6.4 mmol) in THF (15 mL) at room temperature over 1 h. The reaction mixture was stirred at room temperature for 12 h. $NaBH_4$ (120 mg, 3.2 mmol) was added to the reaction mixture and stirring was continued for 3 h. To the reaction mixture was added $NH_4Cl$-solution (40 mL), followed by $CH_2Cl_2$ (30 mL). The pH of the aqueous layer was adjusted to 8 by addition of sat. $NaHCO_3$-solution. The aqueous layers were reextracted with $CH_2Cl_2$ (3x 30 mL). The combined organic layers were concentrated *in vacuo* and the residue was purified by flash chromatography (gradient cyclohexane: ethyl acetate 10:1 to 1:2) to afford the product (538 mg, 1.27 mmol, 80%).

$^1H$ NMR (400 MHz, DMSO-$d_6$) δ 7.87 - 7.78 (m, 1H), 7.74 (d, *J* = 3.6 Hz, 2H), 7.70 - 7.62 (m, 1H), 7.58 - 7.46 (m, 3H), 7.26 - 7.11 (m, 2H), 5.29 - 5.07 (m, 1H), 4.30 - 4.07 (m, 2H), 4.04 - 3.73 (m, 2H), 3.71 - 3.44 (m, 1H), 2.79 - 2.56 (m, 1H), 2.45 - 2.23 (m, 2H), 1.81 (d, *J* = 12.2 Hz, 1H), 1.46 - 1.20 (m, 10H), 0.93 (d, *J* = 10.4 Hz, 1H), 0.68 - 0.38 (m, 2H). $^{13}C$ NMR (101 MHz, DMSO-$d_6$) δ 155.95, 154.28, 143.86, 134.19, 131.67, 130.82, 130.09, 129.08, 122.74, 122.40, 120.11, 113.62, 79.18, 64.71, 60.33, 35.71, 30.18, 29.06, 28.67.LC-MS (ESI): calcd for $C_{25}H_{31}N_3O_3$: 422.24382 $[M+H]^+$, found 422.20 $[M+H]^+$, $R_t$ = 6.31 min; HR-MS found 422.24271.

**Example 18: Preparation of Methyl 2-(2-phenyl-1H-benzo[d]imidazol-1-yl)acetate (S18)**

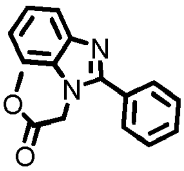

To a suspension of 2-phenyl benzimidazole (0.32 g, 1.6 mmol) in acetonitrile (5 mL) was added caesium carbonate (0.80 g, 2.5 mmol) and methyl bromoacetate (0.16 mL, 1.7 mmol). The reaction mixture was stirred at room temperature for 3 hours and concentrated *in vacuo.* The residue was suspended in a mixture of $CH_2Cl_2$ and sat. $NaHCO_3$ (vol % 50:50, 15 mL). The aqueous layer was reextracted with $CH_2Cl_2$ (2 x 20 mL). The combined organic layers were dried over $Na_2SO_4$, filtered and concentrated in *in vacuo* to afford the product (0.40 g, 1.5 mmol, 91 %).
$^1$H NMR (400 MHz, DMSO-*d6*) δ 7.77 - 7.63 (m, 3H), 7.63 - 7.44 (m, 4H), 7.33 - 7.09 (m, 2H), 5.21 (s, 2H), 3.64 (s, 3H). $^{13}$C NMR (101 MHz, DMSO-*d6*) δ 169.40, 153.91, 143.08, 136.91, 130.57, 130.51, 129.62, 129.53, 123.45, 123.00, 119.87, 111.36, 53.17, 46.57. LC-MS (ESI): calcd for $C_{16}H_{14}N_2O_2$: 267.11280 $[M+H]^+$, found: 267.18 [M+H]+; HR-MS found 267.11311 $[M+H]^+$.

**Example 19: Preparation of Methyl 2-(2-phenyl-1H-benzo[d]imidazol-1-yl)pent-4-enoate (S19)**

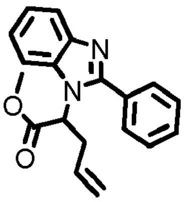

To a solution of **S18** (500 mg, 1.88 mmol) in DMF (5 mL) was added at 0°C NaH (83 mg, 60% mineral oil suspension). The reaction mixture was stirred for 60 minutes, then a solution of allyl bromide (244 μL, 2.90 mmol) in DMF (2 mL) was added. The reaction mixture was allowed to warm to room temperature and stirred for 3 h. A saturated solution of $NH_4Cl$ (10 mL) was added at 0°C to the reaction mixture. The aqueous suspension was extracted with $CH_2Cl_2$ (3 x 20 mL). The combined organic phases were dried over $Na_2SO_4$, filtered and concentrated to dryness. The crude product was purified by automated flash chromatography using a of gradient cyclohexane : ethyl acetate 10:1 to 1:3 to yield the product (340 mg, 1.11 mmol, 59%).
$^1$H NMR (400 MHz, DMSO-$d_6$) δ 7.71 - 7.66 (m, 1H), 7.65 - 7.59 (m, 2H), 7.60 - 7.53 (m, 3H), 7.51 - 7.43 (m, 1H), 7.30 - 7.19 (m, 2H), 5.36 (dd, *J* = 9.5, 6.3 Hz, 1H), 5.32 - 5.16 (m, 1H), 4.76 (d, *J* = 10.2 Hz, 1H), 4.65 (d, *J* = 17.1 Hz, 1H), 3.68 (s, 3H), 2.99 - 2.77 (m, 2H). $^{13}$C NMR (101 MHz, DMSO-$d_6$) δ 170.08, 154.65, 143.50, 134.57, 133.12, 130.73, 130.61, 130.17, 129.53, 123.50, 122.94, 120.30, 119.37, 112.40, 58.12, 53.61, 33.85. LC-MS (ESI): calcd for $C_{19}H_{18}N_2O_2$ : 307.14410 $[M+H]^+$, found: 307.16 $[M+H]^+$; HR-MS found 307.14440 $[M+H]^+$.

**Example 20: Preparation of 2-(2-Phenyl-1H-benzo[d]imidazol-1-yl)pent-4-en-1-ol (S20)**

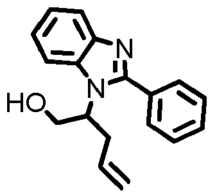

A solution of benzimidazole **S19** (320 mg, 0.94 mmol) in EtOH:THF (1:5, 5 mL) was added to a suspension of $NaBH_4$ (188 mg, 5 mmol) in THF (4 mL) at room temperature over 1 h. The reaction mixture was stirred at room temperature for 12 h. $NaBH_4$ (94 mg, 2.5 mmol) was added to the reaction mixture and stirring was continued for 3 h. To the reaction mixture was added sat. $NH_4Cl$-solution (15 mL), followed by $CH_2Cl_2$ (30 mL). The pH of the aqueous

layer was adjusted to 9 by addition of sat. $NaHCO_3$-solution. The aqueous layer was again extracted with $CH_2Cl_2$ (2x 20 mL). The combined organic layers were dried ($Na_2SO_4$), filtered, concentrated to dryness and the residue was purified by flash chromatography (gradient cyclohexane : ethyl acetate 100:1 to 1:1) to afford the product (217 mg, 0.77 mmol, 83%).
$^1$H NMR (400 MHz, $CDCl_3$) δ 7.69 - 7.62 (m, 1H), 7.63 - 7.56 (m, 2H), 7.47 (d, *J* = 7.9 Hz, 1H), 7.44 - 7.30 (m, 3H), 7.27 - 7.11 (m, 2H), 5.41 - 5.19 (m, 1H), 4.88 - 4.65 (m, 2H), 4.65 - 4.45 (m, 1H), 4.35 - 4.10 (m, 1H), 3.94 - 3.76 (m, 1H), 2.85 - 2.62 (m, 1H), 2.57 - 2.43 (m, 1H). $^{13}$C NMR (101 MHz, $CDCl_3$) δ 155.69, 143.40, 133.48, 133.05, 130.70, 130.37, 129.78, 128.62, 122.64, 122.53, 120.20, 118.56, 112.31, 62.97, 59.55, 34.09. LC-MS (ESI): calcd for $C_{18}H_{18}N_2O$: 279.14919 $[M+H]^+$, found: 279.10 $[M+H]^+$; HR-MS found 279.14941 $[M+H]^+$.

**Example 21: Preparation of 2-(2-Phenyl-1H-benzo[d]imidazol-1-yl)ethanol (S21)**

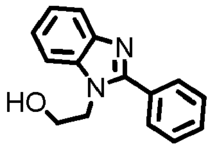

To a solution of benzimidazole **S18** (400 mg, 1.43 mmol) in MeOH (5 mL) was added $NaBH_4$ (376 mg, 10.0 mmol) at room temperature. The reaction mixture was stirred at room temperature for 12 h. To the reaction mixture was added sat. $NH_4Cl$-solution (15 mL), followed by $CH_2Cl_2$ (30 mL). The pH of the aqueous layer was adjusted to 8 by addition of sat. $NaHCO_3$-solution. The aqueous layer was reextracted with $CH_2Cl_2$ (2x 20 mL). The combined organic layers were dried ($Na_2SO_4$), filtered and concentrated *in vacuo* and the residue was purified by flash chromatography (gradient cyclohexane : ethyl acetate 100:1 to 1:1) to afford the product (302 mg, 1.26 mmol, 84%).
$^1$H NMR (400 MHz, DMSO-*d*$_6$) δ 7.97 - 7.84 (m, 2H), 7.76 - 7.60 (m, 2H), 7.60 - 7.45 (m, 3H), 7.34 - 6.99 (m, 2H), 5.08 (s, 1H), 4.33 (t, *J* = 5.5 Hz, 2H), 3.89 - 3.56 (m, 2H). $^{13}$C NMR (101 MHz, DMSO-*d*$_6$) δ 154.25, 143.32, 136.56, 131.27, 130.32, 130.20, 129.21, 122.93, 122.56, 119.71, 111.89, 59.92, 47.49. LC-MS (ESI): calcd for $C_{15}H_{14}N_2O$: 239.11789 $[M+H]^+$, found: 239.19 $[M+H]^+$, Rt = 4.32 min; HR-MS found 239.11806 $[M+H]^+$.

**Example 22: Preparation of tert-Butyl 4-(2-(4-(1-(2-methylbenzyl)-1H-benzo[d]imidazol-2-yl)phenoxy)-1-(2-phenyl-1H-benzo[d]imidazol-1-yl)ethyl)piperidine-1-carboxylate (S22)**

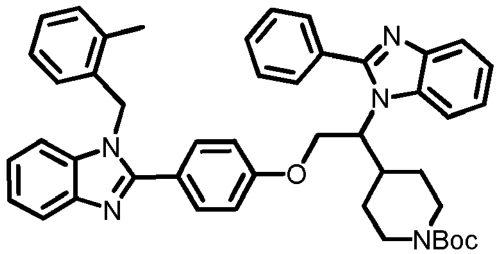

The product was synthesized from phenol **S10** (39 mg, 0.12 mmol) and alcohol **S17** according to Procedure C for the synthesis of phenol ethers via Mitsunobu reaction. The crude product was purified by automated flash chromatography using a gradient of of cyclohexane: ethyl acetate 10:1 to 1:7 to yield compound **S22** (56 mg, 0.08 mmol, 64%).
$^1$H NMR (400 MHz, $CDCl_3$) δ 7.90 - 7.78 (m, 2H), 7.78 - 7.64 (m, 3H), 7.64 - 7.45 (m, 5H), 7.35 - 7.17 (m, 6H), 7.16 - 7.06 (m, 2H), 6.93 - 6.83 (m, 2H), 6.74 (d, *J* = 7.6 Hz, 1H), 5.31 (s, 2H), 4.70 (t, *J* = 8.2 Hz, 1H), 4.63 - 4.51 (m, 1H), 4.51 - 4.36 (m, 1H), 4.18 - 4.04 (m, 1H), 4.02 - 3.74 (m, 1H), 2.70 (t, *J* = 12.0 Hz, 1H), 2.63 - 2.35 (m, 2H), 2.30 (s, 3H), 1.88 (d, *J* = 12.4 Hz, 1H), 1.44 - 1.30 (m, 9H), 1.29 - 1.18 (m, 1H), 1.00 - 0.73 (m, 2H). $^{13}$C NMR (101 MHz, $CDCl_3$) δ 159.50, 155.80, 154.75, 153.98, 143.82, 143.22, 136.42, 134.69, 134.43, 130.83, 130.79, 130.48, 130.31, 130.07, 128.96, 127.84, 126.95, 125.39, 123.44, 123.18, 123.02, 122.94, 122.80, 120.82, 119.98, 114.92, 112.06, 110.55, 79.92, 67.46, 61.68, 46.89, 36.46, 30.15, 28.79, 28.60, 19.32. LC-MS (ESI): calcd for $C_{46}H_{47}N_5O_3$ : 718.37517 $[M+H]^+$, found: 718.32 $[M+H]^+$, $R_t$ = 8.16 min; HR-MS found 718.37640 $[M+H]^+$.

**Example 23: Preparation of tert-Butyl 4-(2-(4-(1-(3-methylbenzyl)-1H-benzo[d]imidazol-2-yl)phenoxy)-1-(2-phenyl-1H-benzo[d]imidazol-1-yl)ethyl)piperidine-1-carboxylate (S23)**

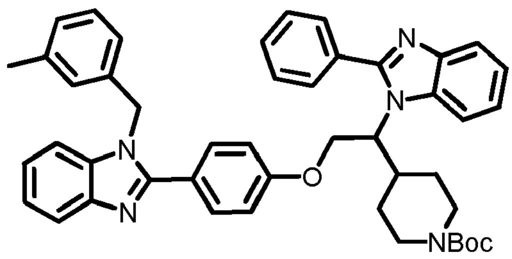

The product was synthesized from phenol **S11** (70 mg, 0.17 mmol) and alcohol **S17** according to Procedure C for the synthesis of phenol ethers via Mitsunobu reaction. The crude product was purified by automated flash chromatography using a gradient of of cyclohexane: ethyl acetate 10:1 to 1:7 to yield compound **S23** (86 mg, 0.12 mmol, 70%). $^1$H NMR (400 MHz, $CDCl_3$) δ 7.81 - 7.73 (m, 2H), 7.63 (dd, *J* = 14.8, 5.8 Hz, 3H), 7.53 (d, *J* = 8.7 Hz, 2H), 7.46 - 7.39 (m, 3H), 7.26 - 7.19 (m, 3H), 7.17 - 7.09 (m, 3H), 7.02 (d, *J* = 7.6 Hz, 1H), 6.87 - 6.73 (m, 4H), 5.28 (s, 2H), 4.63 (d, *J* = 8.7 Hz, 1H), 4.54 - 4.43 (m, 1 H), 4.38 (dt, *J* = 18.9, 9.5 Hz, 1H), 4.04 (d, *J* = 7.3 Hz, 1H), 3.88 - 3.77 (m, 1H), 2.63 (t, *J* = 12.8 Hz, 1H), 2.57 - 2.32 (m, 2H), 2.21 (s, 3H), 1.80 (d, *J* = 12.6 Hz, 1H), 1.22 - 1.07 (m, 2H), 0.90 - 0.67 (m, 3H). $^{13}$C NMR (101 MHz, $CDCl_3$) δ 159.63, 155.75, 154.75, 153.69, 143.70, 139.15, 136.34, 136.13, 133.55, 131.10, 130.72, 130.53, 130.32, 130.11, 129.23, 128.98, 128.84, 126.63, 123.37, 123.11, 123.07, 122.85, 120.77, 119.68, 114.97, 112.09, 110.75, 79.93, 67.47, 61.69, 48.62, 36.45, 30.15, 28.80, 28.60, 21.67. LC-MS (ESI): calcd for $C_{46}H_{47}N_5O_3$ : 718.37517 [M+H]$^+$, found: 718.33 [M+H]$^+$, $R_t$ = 8.17 min; HR-MS found 718.37523 [M+H]$^+$.

**Example 24: Preparation of tert-Butyl 4-(2-(4-(1-(4-fluorobenzyl)-1H-benzo[d]imidazol-2-yl)phenoxy)-1-(2-phenyl-1H-benzo[d]imidazol-1-yl)ethyl)piperidine-1-carboxylate (S24)**

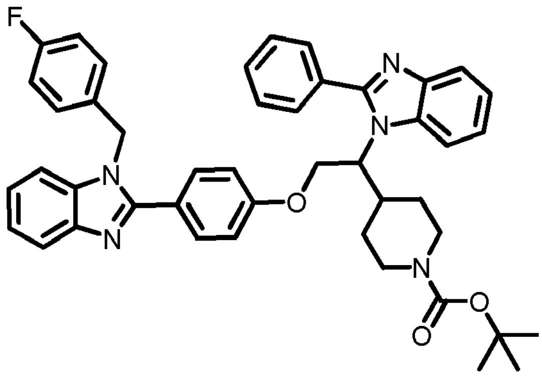

The product was synthesized from phenol **S9** (50 mg, 0.12 mmol) and alcohol **S17** according to Procedure C for the synthesis of phenol ethers via Mitsunobu reaction. The crude product was purified by automated flash chromatography using a gradient of of cyclohexane: ethyl acetate 10:1 to 1:7 to yield the product (58 mg, 0.08 mmol, 66%). $^1$H NMR (400 MHz, $CDCl_3$) δ 7.88 - 7.79 (m, 2H), 7.71 (dd, *J* = 19.7, 5.3 Hz, 3H), 7.61 - 7.44 (m, 5H), 7.34 - 7.15 (m, 5H), 7.01 (p, *J* = 8.8 Hz, 4H), 6.92 (d, *J* = 8.4 Hz, 2H), 5.35 (s, 2H), 4.80 - 4.64 (m, 1H), 4.64 - 4.53 (m, 1H), 4.48 (d, *J* = 9.7 Hz, 1H), 4.21 - 4.02 (m, 1H), 4.01 - 3.87 (m, 1H), 2.71 (t, *J* = 12.4 Hz, 1H), 2.64 - 2.37 (m, 2H), 1.89 (d, *J* = 12.9 Hz, 1H), 1.34 - 1.19 (m, 1H), 1.02 - 0.79 (m, 2H). $^{13}$C NMR (101 MHz, $CDCl_3$) δ 163.67, 161.22, 159.53, 155.80, 154.75, 153.84, 143.82, 143.28, 136.12, 133.57, 132.32, 132.29, 130.96, 130.82, 130.32, 130.08, 128.97, 127.85, 127.77, 123.43, 123.26, 123.01, 122.81, 120.83, 120.09, 116.38, 116.16, 114.95, 112.06, 110.44, 79.94, 67.47, 61.70, 47.92, 36.46, 30.16, 28.80, 28.60. LC-MS (ESI): calcd for $C_{45}H_{44}FN_5O_3$ : 722.35009 [M+H]$^+$, found: 722.31 [M+H]$^+$, $R_t$ = 8.16 min; HR-MS found 722.35052 [M+H]$^+$.

**Example 25: Preparation of tert-Butyl 4-(1-(2-phenyl-1H-benzo[d]imidazol-1-yl)-2-(4-(1-(pyridin-2-ylmethyl)-1H-benzo[d]imidazol-2-yl)phenoxy)ethyl)-piperidine-1-carboxylate (S25)**

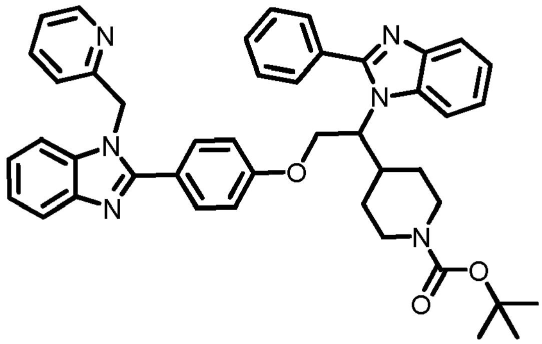

The product was synthesized from phenol **S8** (61 mg, 0.14 mmol) and alcohol **S17** according to Procedure C for the synthesis of phenol ethers via Mitsunobu reaction. The crude product was purified by automated flash chromatography using a gradient of of gradient $CH_2Cl_2$ : MeOH 100:1 to 5:1 to yield the product (60 mg, 0.08 mmol, 61 %).
$^1$H NMR (400 MHz, DMSO-*d6*) δ 8.45 - 8.34 (m, 1H), 8.30 - 8.22 (m, 1H), 7.94 (d, *J* = 7.7 Hz, 1H), 7.78 - 7.39 (m, 10H), 7.30 - 7.14 (m, 6H), 7.06 (d, *J* = 8.6 Hz, 2H), 5.57 (s, 2H), 4.86 (t, *J* = 9.8 Hz, 1H), 4.63 - 4.53 (m, 1H), 4.53 - 4.43 (m, 1H), 3.91 (d, *J* = 12.2 Hz, 1H), 3.79 - 3.59 (m, 1H), 2.79 - 2.51 (m, 2H), 2.45 - 2.36 (m, 1H), 1.92 (d, *J* = 12.0 Hz, 1H), 1.30 (s, 9H), 1.18 - 0.93 (m, 1H), 0.88 - 0.55 (m, 2H). $^{13}$C NMR (101 MHz, DMSO-*d6*) δ 159.69, 155.85, 154.30, 153.72, 149.42, 148.39, 143.83, 143.36, 136.37, 134.58, 133.82, 133.28, 131.38, 131.23, 130.69, 130.30, 129.56, 129.29, 128.87, 125.98, 124.45, 123.38, 123.26, 123.15, 122.92, 122.72, 120.32, 119.81, 115.47, 113.30, 111.54, 79.30, 67.13, 61.64, 45.89, 35.87, 29.08, 28.71.LC-MS (ESI): calcd for $C_{44}H_{44}N_6O_3$: 705.35477 $[M+H]^+$, found: 705.30 $[M+H]^+$, $R_t$ = 6.94 min; HR-MS found 705.35474 $[M+H]^+$.

**Example 26: Preparation of 2-Phenyl-1-(1-(piperidin-4-yl)-2-(4-(1-(pyridin-2-ylmethyl)-1H-benzo[d]imidazol-2-yl) phenoxy)ethyl)-1H-benzo[d]imidazole (S26)**

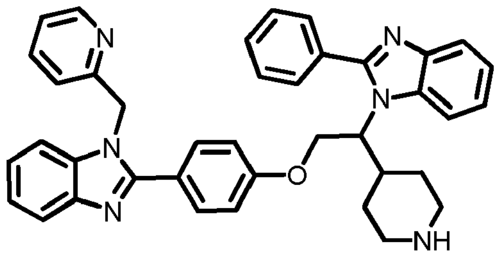

The product was synthesized according to General Procedure (D) starting from **S25** (42 mg, 0.060 mmol) to afford the HCl-salt in quantitative yield. The residue was dissolved in methanol (500 μL) and applied to a preparative C18-RP column eluting with a gradient ($CH_3CN$: $H_2O$ 1:5 to 9:1 containing 0.1% TFA), to afford the desired piperidine (27 mg, 0.044 mmol, 74%) as a TFA-salt.
$^1$H-NMR (600 MHz, DMSO-$d_6$) δ 9.35 - 9.24 (m, 1H), 9.00 - 8.86 (m, 1H), 8.72 - 8.65 (m, 2H), 8.34 - 8.26 (m, 1H), 8.03 - 7.96 (m, 1H), 7.93 - 7.85 (m, 4H), 7.80 - 7.67 (m, 6H), 7.59 - 7.48 (m, 4H), 7.20 (d, *J* = 8.5 Hz, 2H), 5.84 (s, 2H), 4.92 - 4.86 (m, 1H), 4.75 (d, *J* = 8.3 Hz, 1H), 4.69 - 4.53 (m, 1H), 3.26 (d, *J* = 11.6 Hz, 1H), 3.04 (d, *J* = 12.1 Hz, 1H), 2.97 - 2.84 (m, 2H), 2.70 (d, *J* = 9.6 Hz, 1H), 2.09 (d, *J* = 12.5 Hz, 1H), 1.64 - 1.53 (m, 1H), 1.28 - 1.18 (m, 2H). $^{13}$C NMR (151 MHz, DMSO-$d_6$) δ 161.32, 153.01, 151.57, 133.82, 133.34, 132.85, 132.59, 131.32, 131.19, 130.03, 126.82, 126.49, 126.40, 126.12, 116.84, 116.09, 115.73, 115.28, 113.69, 66.71, 62.57, 46.64, 42.93, 40.70, 33.41, 26.16, 25.90. LC-MS (ESI): calcd for $C_{39}H_{36}N_6O$: 605.30234 $[M+H]^+$, found: 605.28$[H]^+$, $R_t$ = 4.89 min; HR-MS found 605.30247 $[M+H]^+$.

**Example 27: Preparation of 1-(3-Methylbenzyl)-2-(4-(2-(2-phenyl-1H-benzo[d]imidazol-1-yl)-2-(piperidin-4-yl) ethoxy)phenyl)-1H-benzo[d]imidazole** (S27)

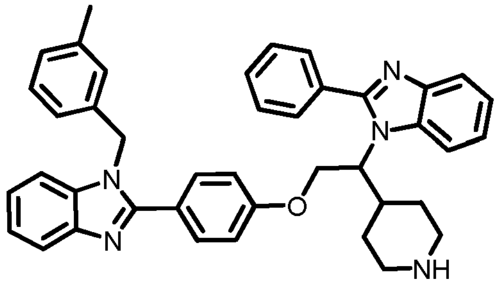

The product was synthesized according to General Procedure (D) from compound **S23** to afford the HCl salt. The residue is dissolved in methanol (500 μL) and applied to a preparative C18-RP column eluting with a gradient (ACN: $H_2O$ 1:5 to 9:1 containing 0.1% acetic acid), to afford the desired piperidine (69%, 15 mg).
$^1H$ NMR (600 MHz, DMSO-$d_6$) δ 7.91 (d, J = 7.3 Hz, 1H), 7.77 - 7.67 (m, 2H), 7.70 - 7.62 (m, 2H), 7.58 (t, *J* = 8.3 Hz, 2H), 7.57 - 7.49 (m, 3H), 7.37 (d, *J* = 7.9 Hz, 1H), 7.25 - 7.10 (m, 5H), 7.09 - 7.00 (m, 3H), 6.82 (s, 1H), 6.70 (d, *J* = 7.7 Hz, 1H), 5.45 (s, 2H), 4.85 (t, *J* = 10.0 Hz, 1H), 4.58 (d, *J* = 8.1 Hz, 1H), 4.46 (t, *J* = 8.5 Hz, 1H), 2.91 (d, *J* = 12.1 Hz, 1H), 2.70 (d, *J* = 12.1 Hz, 1H), 2.25 - 2.16 (m, 1H), 1.94 - 1.83 (m, 1H), 1.16 - 1.00 (m, 1H), 0.83 - 0.56 (m, 2H). $^{13}C$ NMR (151 MHz, DMSO-$d_6$) δ 159.67, 155.86, 153.72, 151.53, 148.65, 143.75, 143.29, 138.64, 137.61, 136.59, 133.86, 131.42, 131.12, 130.68, 130.30, 129.39, 129.30, 128.78, 127.20, 123.66, 123.45, 123.12, 122.77, 122.69, 120.27, 119.68, 115.39, 113.27, 111.58, 67.07, 62.18, 48.06, 45.63, 35.92, 30.73, 29.41, 22.5, 21.66. LC-MS (ESI): calcd for $C_{41}H_{39}N_5O$: 618.32274 $[M+H]^+$, found: 618.30$[M+H]^+$, $R_t$ = 5.79 min; HR-MS found 618.32299 $[M+H]^+$.

**Example 28: Preparation of 1-(4-Fluorobenzyl)-2-(4-(2-(2-phenyl-1H-benzo-[d]imidazol-1-yl)-2-(piperidin-4-yl) ethoxy)phenyl)-1H-benzo[d]imidazole (S28)**

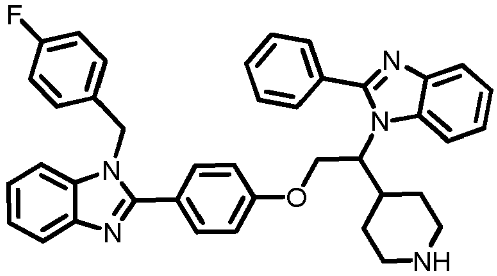

The product was synthesized according to General Procedure (D) to afford the HCl salt. The residue was dissolved in methanol (500 μL) and applied to a preparative C18-RP column eluting with a gradient ($CH_3CN$: $H_2O$ 1:5 to 9:1 containing 0.1% acetic acid), to afford the desired piperidine (65%, 16 mg).
$^1H$ NMR (600 MHz, DMSO-$d_6$) δ 7.92 (d, *J* = 7.4 Hz, 1H), 7.73 (d, *J* = 6.9 Hz, 2H), 7.66 (d, *J* = 8.0 Hz, 2H), 7.61 - 7.49 (m, 5H), 7.44 - 7.37 (m, 1H), 7.25 - 7.16 (m, 4H), 7.12 - 7.02 (m, 4H), 7.02 - 6.96 (m, 2H), 5.49 (s, 2H), 4.85 (t, *J* = 9.9 Hz, 1H), 4.58 (d, *J* = 8.1 Hz, 1H), 4.46 (t, *J* = 8.6 Hz, 1H), 2.92 (d, *J* = 12.1 Hz, 1H), 2.70 (d, *J* = 11.9 Hz, 1H), 2.26 - 2.15 (m, 1H), 1.88 (d, *J* = 11.9 Hz, 1H), 1.12-0.99 (m, 1H), 0.78 - 0.59 (m, 2H). $^{13}C$ NMR (151 MHz, DMSO-$d_6$) δ 173.50, 162.82, 161.20, 159.69, 155.87, 153.67, 143.76, 143.32, 136.45, 133.85, 133.83, 131.43, 131.16, 130.68, 130.30, 129.30, 128.80, 128.74, 123.39, 123.18, 123.12, 122.84, 122.70, 120.28, 119.73, 116.36, 116.22, 115.41, 113.28, 111.57, 67.07, 62.18, 47.41, 45.64, 45.55, 35.89, 30.68, 29.35, 22.85. LC-MS (ESI): calcd for $C_{40}H_{36}FN_5O$: 622.29767 $[M+H]^+$, found: 622.28$[M+H]^+$, $R_t$ = 5.70 min; HR-MS found 622.29806 $[M+H]^+$.

**Example 29: Preparation of 1-(2-Methylbenzyl)-2-(4-(2-(2-phenyl-1H-benzo-[d]imidazol-1-yl)-2-(piperidin-4-yl) ethoxy)phenyl)-1H-benzo[d]imidazole (S29)**

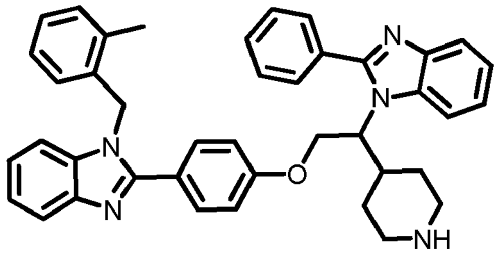

The product was synthesized according to General Procedure (D) starting from **S22** (38 mg, 0.061 mmol) to afford the product as an HCl-salt in quantitative yield. $^{1}H$ NMR (400 MHz, DMSO-*d6*) δ 8.64 (d, *J* = 10.7 Hz, 1 H), 8.22 - 8.07 (m, 1H), 7.99 (d, *J* = 5.1 Hz, 1H), 7.87 - 7.68 (m, 4H), 7.67 - 7.55 (m, 5H), 7.48 (d, *J* = 8.2 Hz, 1H), 7.42 (t, *J* = 7.6 Hz, 1H), 7.38 - 7.29 (m, 3H), 7.24 (d, *J* = 7.1 Hz, 1H), 7.20 - 7.07 (m, 3H), 7.02 (t, *J* = 7.5 Hz, 1H), 6.49 (d, *J* = 7.7 Hz, 1H), 5.54 (s, 2H), 4.86 (t, *J* = 9.8 Hz, 1H), 4.64 (d, *J* = 10.3 Hz, 1H), 4.58 - 4.42 (m, 1H), 3.27 (d, *J* = 11.7 Hz, 1H), 3.06 (d, *J* = 11.5 Hz, 1H), 2.94 - 2.60 (m, 3H), 2.27 (s, 3H), 2.08 (d, *J* = 12.8 Hz, 1H), 1.41 (d, *J* = 12.5 Hz, 1H), 1.19 - 0.90 (m, 2H). $^{13}C$ NMR (101 MHz, DMSO-*d6*) δ 160.65, 158.66, 152.61, 135.68, 135.15, 134.48, 133.14, 131.49, 131.15, 131.00, 130.80, 129.84, 129.52, 128.21, 127.00, 125.23, 124.98, 123.94, 123.71, 119.60, 117.61, 115.81, 113.67, 112.75, 66.96, 61.61, 47.16, 43.20, 33.49, 26.65, 25.93, 19.34.LC-MS (ESI): calcd for $C_{41}H_{39}N_5O$: 618.32274 $[M+H]^+$, found: 618.26 $[M+H]^+$, $R_t$ = 5.85 min; HR-MS found 618.32318 $[M+H]^+$.

**Example 30: Preparation of Ethyl 1-(1H-benzo[d]imidazol-2-yl)piperidine-4-carboxylate (S30)**

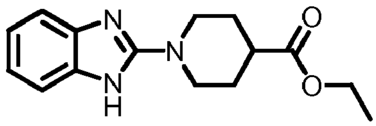

To a solution of 2-chloro benzimidazole (6.0 g, 39.3 mmol) in NMP (40 mL) was added 4-carboxyethyl piperidine (16.5 g, 105 mmol) and diisopropylethylamine (13.4 mL, 78.2 mmol). The reaction mixture was heated to 90°C and stirred for 16 h. Water (100 mL) was added and stirring was continued for an additional 20 min. The precipitate was collected by filtration and washed with water (3x 100 mL) and EtOAc (3x 30 mL) to yield compound **S30** (8.67 g, 81 % mmol).

$^{1}H$ NMR (400 MHz, $CDCl_3$) δ 7.33 - 7.08 (m, 2H), 7.03 - 6.81 (m, 2H), 4.18 - 3.98 (m, 4H), 3.20 - 3.00 (m, 2H), 2.52 - 2.38 (m, 1H), 1.94 (d, *J* = 12.5 Hz, 2H), 1.83 - 1.66 (m, 2H), 1.29 - 1.07 (m, 3H). $^{13}C$ NMR (101 MHz, $CDCl_3$) δ 174.59, 156.47, 120.45, 60.73, 46.33, 41.05, 27.65, 14.39. LC-MS (ESI): calcd for $C_{15}H_{19}N_3O_2$: 274.15500 $[M+H]^+$, found: 274.16 $[H]^+$, $R_t$ = 5.75 min; HR-MS found 274.15523 $[M+H]^+$.

**Example 31: Preparation of Ethyl 1-(1-benzy)-1H-benzo[d]imidazol-2-yl)piperidine-4-carboxylate (S31)**

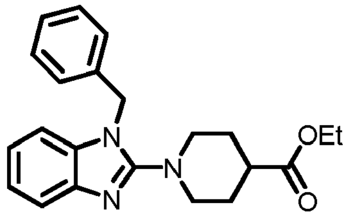

The product was synthesized by procedure A starting from compound **S30** (140 mg, 0.38 mmol, 85%). $^{1}H$ NMR (400 MHz, DMSO-$d_6$) δ 7.46 - 6.96 (m, 9H), 5.25 (s, 2H), 4.15 - 3.99 (m, 2H), 3.41 (d, *J* = 12.3 Hz, 2H), 2.95 (t, *J* = 11.2 Hz, 2H), 2.60 - 2.50 (m, 1H), 1.93 - 1.62 (m, 4H), 1.16 (t, *J* = 6.9 Hz, 3H). $^{13}C$ NMR (101 MHz, DMSO-$d_6$) δ 174.73, 158.51, 141.99, 137.66, 135.78, 129.37, 128.02, 127.09, 121.93, 121.50, 118.02, 110.43, 60.61, 50.42, 47.53, 40.57, 28.09, 14.77. LC-MS (ESI): calcd for $C_{22}H_{25}N_3O$: 364.19 $[M+H]^+$, found: 364.22 $[H]^+$, $R_t$ = 8.01 min.

**Example 32: Preparation of Ethyl 1-(1-(2-chloro-6-fluorobenzyl)-1H-benzo[d]imidazol-2-yl)piperidine-4-carboxylate (S32)**

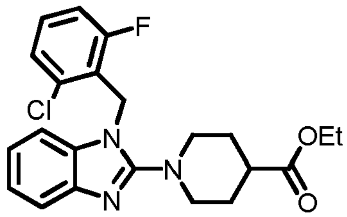

The product was synthesized from **S30** (100 mg, 0.37 mmol) according to General Procedure A for the alkylation of benzimidazoles. The crude product was purified by automated flash chromatography using a gradient of cyclohexane and ethyl acetate 20:1 to 1:2 to yield compound **S32** (141 mg, 0.34 mmol, 92%). $^{1}H$ NMR (400 MHz, DMSO-$d_6$) δ 7.42 - 7.33 (m, 2H), 7.31 - 7.26 (m, 1H), 7.25 - 7.17 (m, 1H), 7.00 (d, *J* = 8.4 Hz, 1H), 6.95 - 6.90 (m, 2H), 5.39 (s, 2H), 4.08 (q, *J* = 7.1 Hz, 2H), 3.43 - 3.37 (m, 2H), 3.01 - 2.89 (m, 2H), 2.58 - 2.49 (m, 1H), 1.95 - 1.83 (m, 2H), 1.80 - 1.67 (m, 2H), 1.18 (t, *J* = 7.1 Hz, 3H). $^{13}C$ NMR (101 MHz, DMSO-$d_6$) δ 174.81, 161.97 ($^{C-F}$ *J* = 249.2 Hz), 159.01, 142.06, 135.14 ($^{C-F}$ *J*=5.4 hz), 134.52, 131.53($^{C-F}$ *J*=9.9 Hz), 126.60 ($^{C-F}$ *J*=3.2 Hz), 122.17 ($^{C-F}$ *J*=16.5 Hz), 121.72, 121.42, 118.33, 115.41 ($^{C-F}$ *J*=22.2 Hz), 109.94, 60.61, 50.59, 28.22, 14.79.LC-MS (ESI): calcd for $C_{22}H_{23}ClFN_3O_2$: 416.15356 $[M+H]^+$, found: 416.13 $[H]^+$, $R_t$ = 7.03 min; HR-MS found 416.15341 $[M+H]^+$.

**Example 33: Preparation of Ethyl 1-(1-(thiophen-3-ylmethyl)-1H-benzo[d]imidazol-2-yl)piperidine-4-carboxylate (S33)**

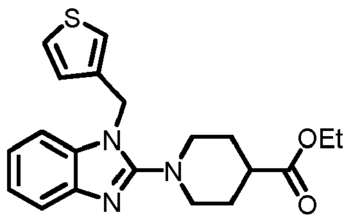

To a mixture of benzimidazole (2.0 g, 7.3 mmol), 3-hydroxymethyl thiophene (2.5 g, 22.0 mmol) and tri-*n*-butyl phosphine (5.4 mL, 22.0 mmol) in THF (20 mL) was added at 0°C TMAD (3.8 g, 22.0 mmol) in one portion. The resulting suspension was allowed to warm to room temperature and stirred at this temperature for 3 h. The reaction mixture was concentrated *in vacuo* and the residue was purified by column chromatography (petroleum ether : ethyl acetate 5:1 to 1:1) to afford compound **S33** (61%, 1.6 g). $^{1}H$ NMR (400 MHz, $CDCl_3$) δ 7.62 (dt, *J* = 7.9, 1.0 Hz, 1 H), 7.32 (dd, *J* = 5.0, 3.0 Hz, 1H), 7.21 - 7.16 (m, 1H), 7.12 - 7.09 (m, 2H), 7.02 (dd, *J* = 3.0, 1.3 Hz, 1H), 6.96 (dd, *J* = 5.0, 1.3 Hz, 1H), 5.18 (s, 2H), 4.16 (q, *J* = 7.1 Hz, 2H), 3.49 (dt, *J* = 6.7, 3.1 Hz, 2H), 3.02 (td, *J* = 12.5, 2.7 Hz, 2H), 2.48 (tt, *J* = 11.1, 4.1 Hz, 1H), 2.05 - 1.95 (m, 2H), 1.95 - 1.79 (m, 2H), 1.27 (t, *J* = 7.1 Hz, 3H). $^{13}C$ NMR (101 MHz, $CDCl_3$) δ 174.81, 158.38, 141.80, 137.71, 135.44, 127.28, 126.23, 122.15, 121.71, 121.70, 118.40, 109.49, 60.77, 50.73, 44.18, 41.10, 28.32, 14.45 LC-MS (ESI): calcd for $C_{20}H_{23}N_3O_2S$: 370.15837 $[M+H]^+$, found: 370.13 $[M+H]^+$, $R_t$ = 6.60 min; HR-MS found 370.15890 $[M+H]^+$.

**Example 34: Preparation of 1-(1-(Thiophen-3-ylmethyl)-1H-benzo[d]imidazol-2-yl)piperidine-4-carboxylic acid (S34)**

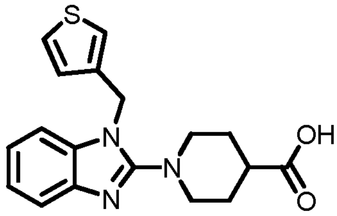

To ester **S33** (1.6 g, 4.5 mmol) in MeOH/$H_2O$ (40 mL, 10/1 vol/vol) was added KOH (1.3 g, 23 mmol) at room temperature. The resulting reaction mixture was stirred at room temperature for 5 h. The pH was adjusted with 20% acetic acid in $H_2O$ to 6 and the aqueous phase was extracted with $CH_2Cl_2$ (4 x 40 mL). The combined organic phases were dried over $Na_2SO_4$ and concentrated *in vacuo* to afford acid **S34** (89 %, 1.4 g).
$^1H$ NMR (400 MHz, DMSO-*d6*) δ 12.23 (s, 1H), 7.49 (dd, *J* = 5.0, 3.0 Hz, 1H), 7.40 (d, *J* = 7.1 Hz, 1H), 7.29 (s, 1H), 7.23 (d, *J* = 7.5 Hz, 1H), 7.08 - 7.00 (m, 2H), 6.94 (d, *J* = 4.9 Hz, 1H), 5.21 (s, 2H), 3.47 - 3.41 (m, 2H), 3.00 - 2.89 (m, 2H), 2.47 - 2.39 (m, 1H), 1.93 - 1.83 (m, 2H), 1.82 - 1.69 (m, 2H). $^{13}C$ NMR (151 MHz, DMSO) δ 176.50, 158.34, 138.47, 135.64, 127.85, 127.34, 125.99, 123.01, 121.88, 121.46, 117.96, 110.40, 50.59, 43.63, 28.22, 21.73. LC-MS (ESI): calcd for $C_{18}H_{19}N_3O_2S$: 342.12707 $[M+H]^+$, found: 342.10 $[M+H]^+$, $R_t$ = 5.74 min; HR-MS found 342.12747 $[M+H]^+$.

**Example 35: Preparation of 1-(1-Benzyl-1H-benzo[d]imidazol-2-yl)piperidine-4-carboxylic acid (S35)**

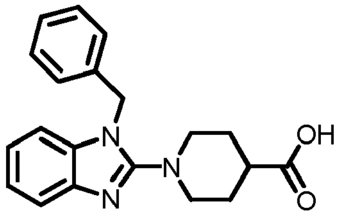

To ester **S31** (1.10 g, 3.0 mmol) in MeOH/$H_2O$ (30 mL, 10/1 vol/vol) was added KOH (0.84 g, 15 mmol) at room temperature. The resulting reaction mixture was stirred at room temperature for 5 h. The pH was adjusted with 20% acetic acid in $H_2O$ to 6 and the aqeous phase was extracted with $CH_2Cl_2$ (4x 30 mL). The combined organic phases were dried over $Na_2SO_4$, filtered and concentrated *in vacuo* to afford **S35** (92 %, 0.93 g).
$^1H$ NMR (400 MHz, DMSO-*$d_6$*) δ 12.17 (s, 1H), 7.43 (d, *J* = 7.8 Hz, 1H), 7.31 (t, *J* = 7.6 Hz, 2H), 7.24 (t, *J* = 7.3 Hz, 1H), 7.16 (d, *J* = 7.5 Hz, 3H), 7.09 (t, *J* = 7.4 Hz, 1H), 7.03 (t, *J* = 7.5 Hz, 1H), 5.27 (s, 2H), 3.49 - 3.40 (m, 2H), 3.04 - 2.90 (m, 2H), 2.47 - 2.39 (m, 1H), 1.88 - 1.80 (m, 2H), 1.78 - 1.63 (m, 2H). $^{13}C$ NMR (101 MHz, $CDCl_3$) δ 176.35, 172.61, 137.46, 135.50, 129.38, 128.06, 127.08, 122.17, 121.72, 117.56, 110.55, 50.42, 47.68, 28.12, 21.71. LC-MS (ESI): calcd for $C_{20}H_{21}N_3O_2$: 336.17065 $[M+H]^+$, found: 336.17 $[M+H]^+$, $R_t$ = 5.82 min; HR-MS found 336.17101 $[M+H]^+$.

**Example 36: Preparation of 1-(1-(2-Chloro-6-fluorobenzyl)-1H-benzo[d]imidazol-2-yl)piperidine-4-carboxylic acid (S36)**

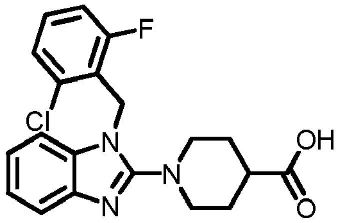

To a solution of the ester **S32** (0.82 g, 2.0 mmol) in HCl/dioxane (4 M, 10 mL) was added water until the formation of a precipitate was observed. The reaction mixture was stirred at room temperature for 2 d, concentrated *in vacuo,* the pH of the aqueous residue was adjusted to 6 with sat. $NaHCO_3$ and the aqueous layer was extracted with $CH_2Cl_2$ (5 x 40 mL). The combined organic phases were dried ($Na_2SO_4$), filtered and concentrated *in vacuo* to afford **S36** (0.65 g, 85%).

$^1$H NMR (400 MHz, DMSO-$d_6$) δ 7.41 - 7.34 (m, 2H), 7.29 (d, *J* = 7.8 Hz, 1H), 7.21 (t, *J* = 9.1 Hz, 1H), 7.04 - 6.97 (m, 1H), 6.97 - 6.90 (m, 2H), 5.40 (s, 2H), 3.46 - 3.37 (m, 2H), 3.02 - 2.87 (m, 2H), 2.47 - 2.39 (m, 1H), 1.90 (dd, *J* = 13.2, 3.2 Hz, 2H), 1.80 - 1.64 (m, 2H). $^{13}$C NMR (101 MHz, DMSO-$d_6$) δ 176.51, 163.22, 160.74, 159.08, 142.07, 135.18, 135.12, 134.53, 131.56, 131.46, 126.60, 126.57, 122.26, 122.10, 121.72, 121.40, 118.32, 115.51, 115.29, 109.94, 67.04, 50.74, 28.31. LC-MS (ESI): calcd for $C_{20}H_{19}ClFN_3O_2$: 388.12226 [M+H]$^+$, found: 388.10 [M+H]$^+$, $R_t$ = 6.27 min; HR-MS found 388.12330 [M+H]$^+$.

**Example 37: Preparation of 2-Cyclohexyl-2-(2-phenyl-1H-benzo[d]imidazol-1-yl)ethyl 1-(1-benzyl-1H-benzo[d]imidazol-2-yl)piperidine-4-carboxylate (S37)**

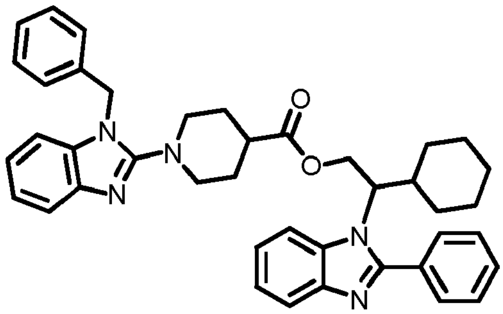

The product was synthesized from alcohol **S16** (40 mg, 0.12 mmol) and carboxylic acid **S35** according to Procedure E for the esterification via Mitsunobu reaction. The crude product was purified by automated flash chromatography using a gradient of cyclohexane and ethyl acetate 50:1 to 1:2 to yield the product (57 mg, 0.09 mmol, 61 %). $^1$H NMR (600 MHz, DMSO-*d6*) δ 8.03 (s, 1H), 7.77 - 7.69 (m, 3H), 7.66 - 7.56 (m, 3H), 7.54 (t, *J* = 7.8 Hz, 1H), 7.42 - 7.21 (m, 10H), 5.38 (s, 2H), 4.84 (t, *J* = 10.7 Hz, 1H), 4.62 - 4.52 (m, 1H), 4.43 (t, *J* = 8.0 Hz, 1H), 3.66 - 3.55 (m, 1H), 3.56 - 3.43 (m, 1H), 3.28 - 3.10 (m, 2H), 2.62 - 2.52 (m, 1H), 2.40 (d, *J* = 10.4 Hz, 1H), 1.87 (d, *J* = 10.8 Hz, 1H), 1.72 - 1.57 (m, 3H), 1.57 - 1.32 (m, 4H), 1.32 - 1.14 (m, 1H), 1.09 - 0.83 (m, 4H), 0.73 - 0.57 (m, 1H). $^{13}$C NMR (151 MHz, DMSO-*d6*) δ 173.73, 159.32, 159.09, 158.86, 158.63, 154.12, 153.28, 135.48, 132.98, 131.61, 130.74, 129.67, 129.63, 128.58, 126.89, 125.00, 124.71, 124.46, 118.46, 117.82, 115.87, 114.20, 113.41, 112.03, 109.99, 63.47, 62.19, 49.29, 48.97, 39.23, 37.42, 30.54, 29.64, 27.43, 27.36, 26.01, 25.75. LC-MS (ESI): calcd for $C_{41}H_{43}N_5O_2$: 638.34895 [M+H]$^+$, found: 638.34 [M+H]$^+$, $R_t$ = 7.79 min; HR-MS found 638.34934 [M+H]$^+$.

**Example 38: Preparation of 2-Cyclohexyl-2-(2-phenyl-1H-benzo[d]imidazol-1-yl)ethyl 1-(1-(2-chloro-6-fluorobenzyl)-1H-benzo[d]imidazol-2-yl)piperidine-4-carboxylate (S38)**

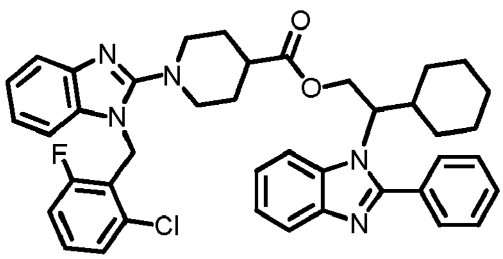

The product was synthesized from alcohol **S16** (40 mg, 0.12 mmol) and carboxylic acid **S36** according to Procedure E for the esterification via Mitsunobu reaction. The crude product was purified by automated flash chromatography using a gradient of cyclohexane and ethyl acetate 20:1 to 1:2 to yield the product (56 mg, 0.08 mmol, 56%). $^1$H NMR (400 MHz, $CDCl_3$) δ 7.86 - 7.77 (m, 1H), 7.70 (d, *J* = 5.5 Hz, 2H), 7.61 - 7.44 (m, 5H), 7.33 - 7.24 (m, 3H), 7.24 - 7.07 (m, 3H), 7.06 - 6.83 (m, 3H), 5.31 (s, 2H), 4.87 - 4.70 (m, 1H), 4.56 (dd, *J* = 11.7, 3.7 Hz, 1H), 4.49 (td, *J* = 10.3, 3.9 Hz, 1H), 3.47 - 3.11 (m, 2H), 3.09 - 2.84 (m, 2H), 2.48 - 2.15 (m, 2H), 1.98 - 1.83 (m, 1H), 1.86 - 1.51 (m, 7H), 1.29 - 0.93 (m, 5H), 0.80 - 0.53 (m, 1H). $^{13}$C NMR (101 MHz, $CDCl_3$) δ 174.36, 163.26, 160.77, 158.67, 141.76, 135.72,

135.67, 133.90, 130.46, 130.37, 130.18, 129.93, 128.83, 126.08, 126.05, 122.94, 122.66, 121.89, 121.71, 121.61, 121.45, 120.64, 118.66, 114.77, 114.54, 112.11, 109.78, 63.70, 61.52, 50.93, 50.70, 40.95, 40.00, 39.96, 38.37, 31.14, 30.40, 29.78, 28.08, 28.00, 26.05, 25.94, 25.84. LC-MS (ESI): calcd for $C_{41}H_{41}ClFN_5O_2$: 690.30056 $[M+H]^+$, found: 690.30 $[M+H]^+$, $R_t$ = 7.81 min; HR-MS found 690.30137 $[M+H]^+$.

**Example 39: Preparation of 2-Cyclohexyl-2-(2-phenyl-1H-benzo[d]imidazol-1-yl)ethyl 1-(1-(thiophen-3-ylmethyl)-1H-benzo[d]imidazol-2-yl)piperidine-4-carboxylate (S39)**

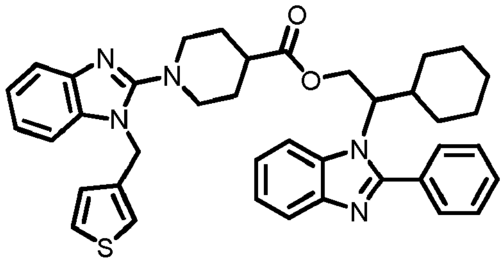

The product was synthesized from alcohol **S16** (40 mg, 0.12 mmol) and carboxylic acid **S34** according to Procedure E for the esterification via Mitsunobu reaction. The crude product was purified by automated flash chromatography using a gradient of cyclohexane and ethyl acetate 20:1 to 1:2 to yield the product (52 mg, 0.08 mmol, 65%). $^1H$ NMR (400 MHz, $CDCl_3$) δ 7.81 (d, *J* = 8.4 Hz, 1H), 7.72 - 7.63 (m, 2H), 7.63 - 7.42 (m, 4H), 7.34 - 7.21 (m, 4H), 7.21 - 7.15 (m, 1H), 7.14 - 7.05 (m, 2H), 7.01 - 6.97 (m, 1H), 6.92 (d, *J* = 5.0 Hz, 1H), 5.11 (s, 2H), 4.84 - 4.72 (m, 1H), 4.61 - 4.39 (m, 2H), 3.46 - 3.24 (m, 2H), 3.04 - 2.71 (m, 2H), 2.39 - 2.11 (m, 2H), 1.97 - 1.81 (m, 1H), 1.84 - 1.47 (m, 7H), 1.34 - 0.92 (m, 5H), 0.81 - 0.62 (m, 1H). $^{13}C$ NMR (101 MHz, $CDCl_3$) δ 174.29, 158.15, 141.72, 137.61, 135.39, 130.18, 129.95, 128.83, 127.29, 126.22, 122.94, 122.66, 122.16, 121.75, 121.73, 120.65, 118.40, 109.49, 63.65, 61.45, 50.56, 50.38, 44.10, 40.90, 38.39, 31.12, 29.80, 27.97, 27.94, 26.05, 25.94, 25.84.LC-MS (ESI): calcd for $C_{39}H_{41}N_5O_2S$: 644.30537 $[M+H]^+$, found: 644.31 $[M+H]^+$, $R_t$ = 7.53 min; HR-MS found 644.30594$[M+H]^+$.

**Example 40: Preparation of tert-butyl 4-(2-(1-(1-benzyl-1H-benzo[d]imidazol-2-yl)piperidine-4-carbonyloxy)-1-(2-phenyl-1H-benzo[d]imidazol-1-yl)ethyl)piperidine-1-carboxylate (S40)**

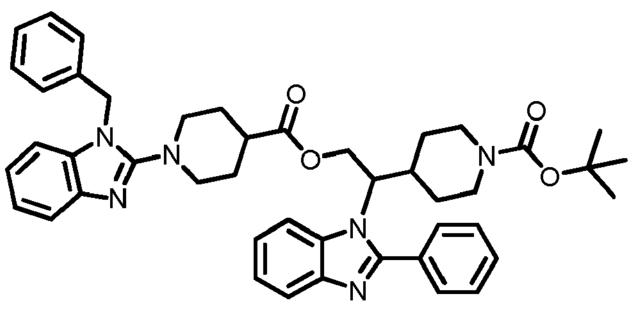

The product was synthesized from alcohol **S17** (63 mg, 0.15 mmol) and carboxylic acid **S35** according to Procedure E for the esterification via Mitsunobu reaction. The crude product was purified by automated flash chromatography using a gradient of cyclohexane and ethyl acetate 20:1 to 1:2 to yield compound **S40** (70 mg, 0.10 mmol, 64%). $^1H$ NMR (400 MHz, $CDCl_3$) δ 7.74 (d, *J* = 7.6 Hz, 1H), 7.63 - 7.50 (m, 3H), 7.50 - 7.38 (m, 4H), 7.31 - 7.16 (m, 5H), 7.15 - 6.98 (m, 4H), 6.95 (d, *J* = 7.8 Hz, 1H), 5.08 (s, 2H), 4.75 (t, *J* = 10.2 Hz, 1H), 4.51 - 4.30 (m, 2H), 4.14 - 3.96 (m, 1H), 3.95 - 3.69 (m, 1H), 3.41 - 3.14 (m, 2H), 2.97 - 2.72 (m, 2H), 2.63 (t, *J* = 12.0 Hz, 1H), 2.51 - 2.14 (m, 3H), 1.84 - 1.41 (m, 4H), 1.39 - 1.04 (m, 10H), 1.04 - 0.63 (m, 3H). $^{13}C$ NMR (101 MHz, $CDCl_3$) δ 174.16, 158.09, 155.58, 154.75, 143.80, 136.34, 135.40, 133.42, 130.67, 130.15, 130.10, 129.23, 128.99, 127.93, 126.24, 123.18, 122.92, 122.34, 121.91, 120.87, 118.24, 111.79, 109.67, 79.97, 63.33, 60.90, 50.50, 50.35, 47.86, 40.80, 36.99, 30.13, 28.82, 28.60, 27.92, 27.88. LC-MS (ESI): calcd for $C_{45}H_{50}N_6O_4$: 739.39663 $[M+H]^+$, found: 739.39 $[M+H]^+$, $R_t$ = 7.38 min; HR-MS found 739.39738 $[M+H]^+$. Chiral HPLC conditions: flow rate: 0.35 mL/min; solvent: 75 % ethanol in *iso*-hexane. $R_T$: (R)-26.5

min, (S)-56.8 min.

**Example 41: Preparation of tert-Butyl 4-(1-(2-phenyl-1H-benzo[d]imidazol-1-yl)-2-(1-(1-(thiophen-3-ylmethyl)-1H-benzo[d]imidazol-2-yl)piperidine-4-carbonyloxy)ethyl)piperidine-1-carboxylate (S41)**

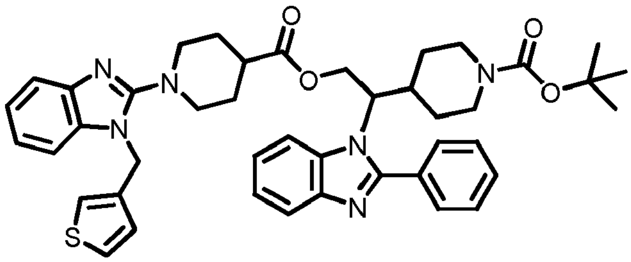

The product was synthesized from alcohol **S17** (80 mg, 0.19 mmol) and carboxylic acid **S34** according to Procedure E for the esterification via Mitsunobu reaction. The crude product was purified by automated flash chromatography using a gradient of cyclohexane and ethyl acetate 20:1 to 1:2 to yield compound **S41** (95 mg, 0.13 mmol, 67%). $^1$H NMR (400 MHz, $CDCl_3$) δ 7.82 (d, *J* = 7.7 Hz, 1H), 7.66 (s, 2H), 7.59 (t, *J* = 8.4 Hz, 1H), 7.57 - 7.45 (m, 4H), 7.36 - 7.22 (m, 3H), 7.23 - 7.14 (m, 1H), 7.14 - 7.05 (m, 2H), 6.97 (d, *J* = 1.6 Hz, 1H), 6.91 (d, *J* = 5.0 Hz, 1H), 5.11 (s, 2H), 4.83 (t, *J* = 10.0 Hz, 1H), 4.59 - 4.42 (m, 2H), 4.15 (d, *J* = 11.3 Hz, 1H), 4.03 - 3.86 (m, 1H), 3.50 - 3.23 (m, 2H), 3.02 - 2.79 (m, 2H), 2.78 - 2.62 (m, 1H), 2.54 - 2.25 (m, 3H), 1.85 (d, *J* = 11.8 Hz, 1H), 1.79 - 1.53 (m, 4H), 1.40 (s, 9H), 1.23 (d, *J* = 11.9 Hz, 1H), 1.00 (d, *J* = 26.4 Hz, 1H), 0.92 (d, *J* = 9.8 Hz, 1H). $^{13}$C NMR (101 MHz, $CDCl_3$) δ 174.21, 158.07, 155.60, 154.75, 143.82, 141.66, 137.58, 135.36, 133.42, 130.69, 130.16, 130.12, 129.00, 127.32, 126.20, 123.18, 122.93, 122.20, 121.79, 121.72, 120.88, 118.39, 111.79, 109.50, 79.98, 63.31, 60.90, 50.53, 50.36, 44.10, 40.88, 37.01, 30.13, 28.83, 28.60, 27.97, 27.93. LC-MS (ESI): calcd for $C_{43}H_{48}N_6O_4S$: 745.35305 $[M+H]^+$, found: 745.33 $[M+H]^+$, $R_t$ = 7.35 min; HR-MS found 745.35302 $[M+H]^+$. Chiral HPLC conditions: flow rate: 0.35 mL/min; solvent: 75 % ethanol in *iso*-hexane. $R_T$: (R)-26.8 min, (S)-54.6 min.

**Example 42: Preparation of tert-Butyl 4-(2-(1-(1-(2-chloro-6-fluorobenzyl)-1H-benzo[d]imidazol-2-yl)piperidine-4-carbonyloxy)-1-(2-phenyl-1H-benzo[d]imidazol-1-yl)ethyl)piperidine-1-carboxylate (S42)**

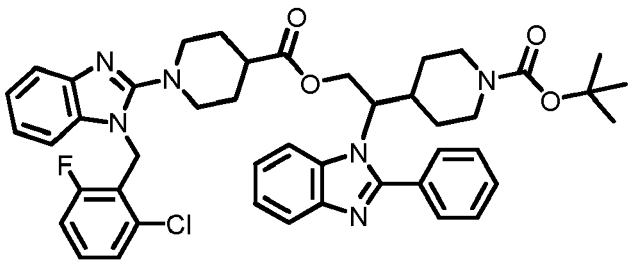

The product was synthesized from alcohol **S17** (75 mg, 0.18 mmol) and carboxylic acid **S36** according to Procedure E for the esterification via Mitsunobu reaction. The crude product was purified by automated flash chromatography using a gradient of cyclohexane and ethyl acetate 20:1 to 1:2 to yield compound **S42** (86 mg, 0.11 mmol, 61 %). $^1$H NMR (400 MHz, $CDCl_3$) δ 7.91 - 7.77 (m, 1H), 7.76 - 7.63 (m, 2H), 7.63 - 7.41 (m, 5H), 7.35 - 7.24 (m, 2H), 7.24 - 7.06 (m, 3H), 7.05 - 6.87 (m, 3H), 5.31 (s, 2H), 4.84 (t, *J* = 10.1 Hz, 1H), 4.61 - 4.44 (m, 2H), 4.23 - 4.06 (m, 1H), 4.00 - 3.85 (m, 1H), 3.42 - 3.24 (m, 2H), 3.06 - 2.88 (m, 2H), 2.71 (t, *J* = 12.2 Hz, 1H), 2.53 - 2.31 (m, 3H), 1.92 - 1.58 (m, 4H), 1.42 (s, 9H), 1.31 - 1.14 (m, 2H), 1.09 - 0.79 (m, 2H). $^{13}$C NMR (101 MHz, $CDCl_3$) δ 174.29, 163.25, 160.76, 158.60, 155.61, 154.76, 143.79, 141.68, 135.70, 135.65, 133.87, 133.43, 130.66, 130.50, 130.40, 130.16, 130.13, 129.00, 126.09, 126.05, 123.20, 122.94, 121.94, 121.76, 121.58, 121.42, 120.87, 118.64, 114.78, 114.55, 111.84, 109.82, 79.98, 63.35, 60.95, 50.88, 50.68, 40.93, 40.00, 39.96, 36.99, 30.14, 28.82, 28.60, 28.08, 28.00, 27.13. LC-MS (ESI): calcd for $C_{45}H_{48}ClFN_6O_4$: 791.34824 $[M+H]^+$, found: 791.30 $[M+H]^+$, $R_t$ = 8.00 min; HR-MS found 791.34737 $[M+H]^+$.

**Example 43: Preparation of 2-(2-Phenyl-1H-benzo[d]imidazol-1-yl)-2-(piperidin-4-yl)ethyl 1-(1-(2-chloro-6-fluorobenzyl)-1H-benzo[d]imidazol-2-yl)piperidine-4-carboxylate (S43)**

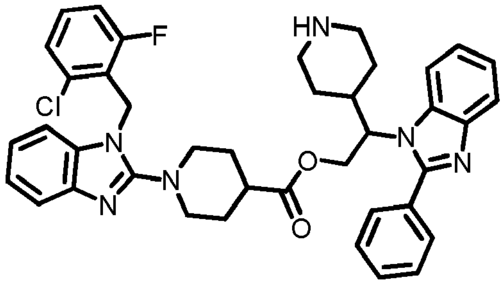

The product was synthesized according to General Procedure (D) starting from the protected piperidine **S42** (15 mg,0.02 mmol) to afford the HCl-salt in quantitative yield. The residue was dissolved in methanol (500 μL) and applied to a preparative C18-RP column eluting with a gradient ($CH_3CN$: $H_2O$ 1:5 to 9:1 containing 0.1% TFA), to afford the product (9 mg, 0.01 mmol, 65%) as the TFA-Salt.
$^1H$ NMR (400 MHz, DMSO-*d*$_6$) δ 9.58 - 9.37 (m, 1H), 9.34 - 9.07 (m, 1H), 8.38 - 8.20 (m, 1H), 7.98 - 7.81 (m, 3H), 7.80 - 7.71 (m, 3H), 7.64 - 7.55 (m, 3H), 7.49 - 7.37 (m, 1H), 7.37 - 7.15 (m, 5H), 7.16 - 7.01 (m, 1H), 5.54 (s, 2H), 4.94 - 4.78 (m, 1H), 4.77 - 4.46 (m, 2H), 3.88 - 3.73 (m, 2H), 3.40 - 3.32 (m, 2H), 3.32 - 3.15 (m, 1H), 3.07 - 2.96 (m, 1H), 2.96 - 2.77 (m, 2H), 2.77 - 2.57 (m, 2H), 2.09 - 1.88 (m, 1H), 1.80 - 1.40 (m, 4H), 1.40 - 1.12 (m, 3H). $^{13}C$ NMR (101 MHz, DMSO-*d*$_6$) δ 173.66, 162.99, 160.51, 153.22, 152.23, 134.92, 134.87, 133.23, 132.54, 132.44, 131.22, 131.03, 130.15, 129.97, 126.92, 126.61, 125.28, 124.71, 120.13, 119.98, 116.37, 115.90, 115.68, 115.39, 113.45, 111.98, 72.84, 71.20, 67.03, 62.98, 62.16, 60.84, 49.58, 49.24, 44.31, 42.94, 39.16, 27.72, 26.20, 25.68. LC-MS (ESI): calcd for $C_{40}H_{40}ClFN_6O_2$: 691.29581 $[M+H]^+$, found: 691.26 $[M+H]^+$, $R_t$ = 5.63 min; HR-MS found 691.29659 $[M+H]^+$.

**Example 44: Preparation of tert-Butyl 4-(2-(1,3-dioxoisoindolin-2-yl)-1-(2-phenyl-1H-benzo[d]imidazol-1-yl)ethyl) piperidine-1-carboxylate (S44)**

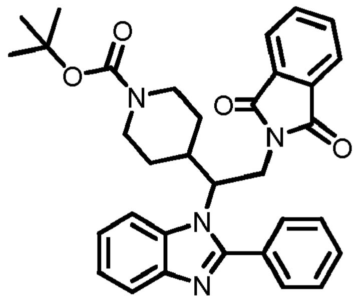

To a mixture of alcohol **S17** (300 mg, 0.71 mmol), phthalimide (157 mg, 1.07 mmol), polymer bound triphenyl phosphine (474 mg, 3 mmol/g) and toluene (5 mL) at 0°C was added di-*tert*-butyl azodicarboxylate (327 mg, 1.42 mmol). After 10 min the reaction mixture was allowed to warm to room temperature and stirred for 16 h. Due to incomplete conversion phthalimide (300 mg, 0.71 mmol), polymer bound triphenyl phosphine (400 mg) and di-*tert*-butyl azodicarboxylate (330 mg, 1.44 mmol) were added to the reaction mixture at room temperature. The mixture was stirred for an additional 16 h and filtered. The polymer resin was washed with a mixture of $CH_2Cl_2$ and ethyl acetate (50:50 vol%, 100 mL) and the combined filtrates were concentrated *in vacuo.* After purification by column chromatography (gradient cyclohexane : ethyl acetate 5:1 to 1:2) compound **S44** was isolated (320 mg, 0.58 mmol, 82 %).
$^1H$ NMR (400 MHz, $CDCl_3$) δ 7.87 - 7.74 (m, 1H), 7.73 - 7.59 (m, 5H), 7.41 - 7.29 (m, 3H), 7.24 - 7.06 (m, 4H), 4.53 - 4.43 (m, 1H), 4.41 - 4.31 (m, 1H), 4.24 (d, J = 13.0 Hz, 1H), 4.13 - 3.94 (m, 2H), 2.80 (t, J = 12.4 Hz, 1H), 2.65 - 2.39 (m, 2H), 2.08 (d, J = 13.0 Hz, 1H), 1.56 - 1.29 (m, 10H), 1.29 - 1.10 (m, 2H). 13C NMR (101 MHz, $CDCl_3$) δ 167.19, 155.21, 154.83, 143.82, 134.32, 133.99, 131.86, 130.11, 129.85, 129.71, 128.66, 123.59, 123.21, 122.92, 120.90, 111.92, 79.95, 60.92, 38.19, 37.84, 30.68, 29.07, 28.64, 27.12. LC-MS (ESI): calcd for $C_{33}H_{34}N_4O_4$: 551.26528 [M+H]+, found: 551.20 [M+H]+, Rt = 8.41 min; HR-MS found 551.26641 $[M+H]^+$.

**Example 45: Preparation of tert-Butyl 4-(2-amino-1-(2-phenyl-1H-benzo[d]imidazol-1-yl)ethyl)piperidine-1-carboxylate (S45)**

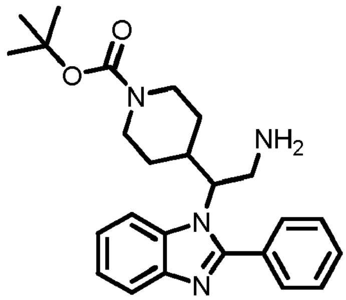

To a solution of phthalimide **S44** (300 mg, 0.54 mmol) in EtOH (3 mL) was added hydrazine-hydrate (0.22 mL, 0.44 mmol). The reaction mixture wass stirred at room temperature for 72 h. The reaction mixture was concentrated *in vacuo,* and coevaporated with EtOH (3x 5 mL). The colorless residue was taken up in $CH_2Cl_2$ (10 mL). To the resulting suspension was added sat. $NaHCO_3$ solution (10 mL). The aqueous layer was extracted with $CH_2Cl_2$ (3x 15mL). The combined organic layers were dried over $MgSO_4$, filtered and concentrated *in vacuo* to afford the free amine **S45** (200 mg, 0.47 mmol, 87%).
$^1$H NMR (400 MHz, DMSO-$d_6$) δ 7.86 - 7.74 (m, 3H), 7.69 - 7.62 (m, 1H), 7.57 - 7.46 (m, 3H), 7.26 - 7.14 (m, 2H), 4.13 - 4.00 (m, 1H), 3.85 (d, *J* = 13.2 Hz, 1H), 3.70 - 3.51 (m, 1H), 3.51 - 3.37 (m, 1H), 3.14 (d, *J* = 10.1 Hz, 1H), 2.74 - 2.52 (m, 1H), 2.38 - 2.11 (m, 2H), 1.77 (d, *J* = 13.1 Hz, 1H), 1.28 (s, 9H), 0.96 - 0.78 (m, 1H), 0.64 - 0.41 (m, 2H). LC-MS (ESI): calcd for $C_{25}H_{32}N_4O_2$: 421.25980 $[M+H]^+$, found: 421.16 $[M+H]^+$; HR-MS found 421.25938 $[M+H]^+$.

**Example 46: Preparation of tert-Butyl 4-(1-(2-phenyl-1H-benzo[d]imidazol-1-yl)-2-(1-(1-(thiophen-3-ylmethyl)-1H-benzo[d]imidazol-2-yl)piperidine-4-carboxamido)ethyl)piperidine-1-carboxylate (S46)**

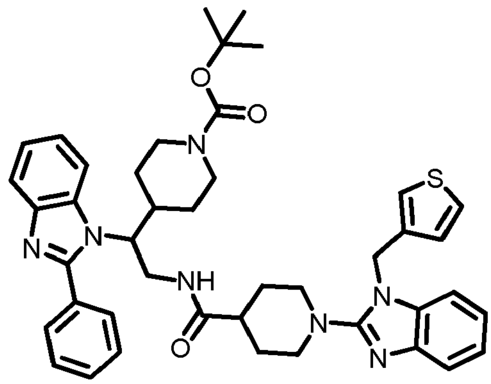

To a mixture of acid **S34** (122 mg, 0.36 mM), amine **S45** (100 mg, 0.24 mM) in DMF (2 mL) at room temperature was added DIPEA (51 μL, 0.36 mM) and PyBOP (185 mg, 0.36 mmol). The reaction mixture was stirred at room temperature for 2 d and then concentrated *in vacuo.* The residue was dissolved in methanol (500 μL) and applied to a preparative C18-RP column eluting with a gradient ($CH_3CN$:$H_2O$ 1:4 to 9:1 containing 0.1 % TFA), to afford compound **S46** (119 mg, 0.16 mmol, 67%).
$^1$H NMR (400 MHz, $CDCl_3$) δ 7.74 (d, *J* = 7.7 Hz, 1H), 7.58 - 7.46 (m, 4H), 7.44 - 7.36 (m, 3H), 7.30 - 7.19 (m, 3H), 7.17 - 7.12 (m, 1H), 7.08 (dd, *J* = 3.9, 2.1 Hz, 2H), 6.97 - 6.94 (m, 1H), 6.91 (dd, *J* = 5.0, 1.2 Hz, 1H), 5.12 (s, 2H), 4.37 (td, *J* = 10.3, 3.5 Hz, 1H), 4.29 - 4.19 (m, 1H), 4.09 (t, *J* = 11.1 Hz, 1H), 3.98 - 3.76 (m, 1H), 3.72 - 3.57 (m, 1H), 3.37 (d, *J* = 12.3 Hz, 2H), 2.83 - 2.72 (m, 2H), 2.66 (t, *J* = 12.3 Hz, 1H), 2.40 (t, *J* = 11.2 Hz, 1H), 2.26 (d, *J* = 11.2 Hz, 1H), 2.12 - 2.02 (m, 2H), 2.01 - 1.91 (m, 1H), 1.79 - 1.63 (m, 3H), 1.56 - 1.47 (m, 1H), 1.38 (s, 9H), 1.33 - 1.19 (m, 2H). $^{13}$C NMR

(101 MHz, $CDCl_3$) δ 175.14, 158.10, 155.72, 154.74, 143.65, 141.45, 137.57, 135.28, 133.31, 130.23, 130.20, 130.03, 129.02, 127.30, 126.22, 123.31, 123.10, 122.23, 121.84, 121.72, 120.71, 118.19, 111.91, 109.56, 79.86, 62.31, 50.62, 44.13, 42.72, 39.83, 38.14, 30.11, 28.88, 28.75, 28.59, 28.32. LC-MS (ESI): calcd for $C_{43}H_{49}N_7O_3S$: 744.36904 $[M+H]^+$, found: 744.36824 $[M+H]^+$; HR-MS found 744.36824 $[M+H]^+$.

**Example 47: Preparation of 1-(2-(4-(1-Benzyl-1H-benzo[d]imidazol-2-yl)piperazine-1-carbonyloxy)-1-(1-(tert-butoxycarbonyl)piperidin-4-yl)ethyl)-2-phenyl-1H-benzo[d]imidazol-6-ylium (S47)**

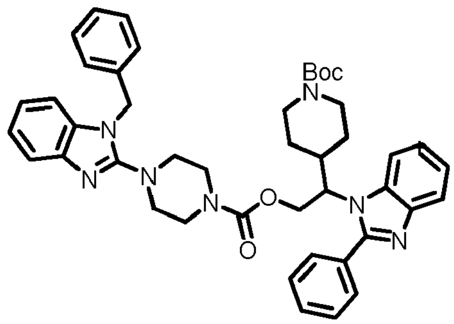

To 4-nitrophenyl chloroformate (65 mg, 0.32 mmol) in THF (1 mL) was added pyridine (26 μL, 0.32 mmol) and the mixture was stirred at room temperature for 30 min. Alcohol **S17** (90 mg, 0.21 mmol) was added and the reaction mixture was stirred at room temperature for 16 h. 4-nitrophenyl chloroformate (65 mg, 0.32 mmol) and pyridine (26 μL, 0.32 mmol) were added and stirring was continued for 16 h. The reaction mixture was concentrated *in vacuo,* the residue dissolved in $CH_2Cl_2$ (10 mL) and extracted with a sat. $NH_4Cl$-solution (10 mL). The aqueous layer was reextracted with $CH_2Cl_2$ (3 x 10 mL). The combined organic layers were dried over $Na_2SO_4$, filtered and concentrated *in vacuo.* To a solution of HOBT (48 mg, 0,32 mmol) in $CH_2Cl_2$ (3 mL) molecular sieves (4 Angstrom, 50 mg) were added and the mixture was stirred for 5 min. After 5 min piperazine[8] (94 mg, 0,32 mmol) was added and the resulting mixture was stirred for 16 h. The reaction mixture was concentrated *in vacuo,* the residue dissolved in $CH_2Cl_2$ (5 mL) and extracted with sat. $NaHCO_3$. The aqueous layer was reextracted with $CH_2Cl_2$ (3x 10 mL). The combined organic layers were dried over $Na_2SO_4$, filtered and concentrated *in vacuo.* The residue was purified by flash chromatography (gradient cyclohexane : ethyl acetate 10:1 to 1:2.5) to afford the product **S47** (71 mg, 46%) along with alcohol **S17** (25%).
$^1H$ NMR (400 MHz, $CDCl_3$) δ 7.81 (d, *J* = 7.4 Hz, 1H), 7.65 - 7.56 (m, 3H), 7.56 - 7.40 (m, 4H), 7.35 - 7.22 (m, 5H), 7.18 (t, *J* = 7.6 Hz, 1H), 7.14 - 7.06 (m, 3H), 7.02 (d, *J* = 7.8 Hz, 1H), 5.18 (s, 2H), 4.76 - 4.60 (m, 2H), 4.49 (t, *J* = 11.5 Hz, 1H), 4.19 - 4.05 (m, 1H), 3.98 - 3.76 (m, 1H), 3.55 - 2.95 (m, 6H), 2.77 - 2.59 (m, 1H), 2.53 - 2.30 (m, 2H), 1.89 (d, *J* = 11.8 Hz, 1H), 1.40 (s, 9H), 1.32 - 1.17 (m, 1H), 1.08 - 0.84 (m, 2H). $^{13}C$ NMR (101 MHz, $CDCl_3$) δ 157.39, 154.75, 154.59, 141.42, 136.21, 135.53, 130.59, 130.13, 130.00, 129.31, 128.95, 128.06, 126.19, 123.16, 122.89, 122.41, 122.08, 120.82, 118.49, 111.81, 109.68, 79.92, 64.24, 61.30, 50.40, 47.73, 43.56, 37.03, 30.24, 28.80, 28.60, 27.12. LC-MS (ESI): calcd for $C_{44}H_{49}N_7O_4$: 740.39188 $[M+H]^+$, found: 740.33 $[M+H]^+$, $R_t$ = 7.84 min; HR-MS found 740.39234 $[M+H]^+$.

**Example 48: Preparation of 2-(2-Pheny)-1H-benzo[d]imidazol-1-yl)-2-(piperidin-4-yl)ethyl 4-(1-benzyl-1H-benzo [d]imidazol-2-yl)piperazine-1-carboxylate (S48)**

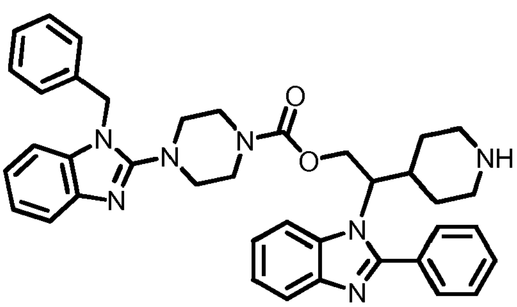

The product was synthesized according to General Procedure (D) starting from the protected piperidine **S47** (30 mg,0.040 mmol) to afford the HCl-salt in quantitative yield. The residue was dissolved in methanol (500 μL) and applied to a preparative C18-RP column eluting with a gradient ($CH_3CN$: $H_2O$ 1:5 to 9:1 containing 0.1% TFA), to afford the product (17 mg, 0.026 mmol, 66%) as the TFA-salt.
$^1H$ NMR (400 MHz, DMSO-$d_6$) δ 7.87 - 7.79 (m, 1H), 7.71 - 7.63 (m, 1H), 7.63 - 7.55 (m, 2H), 7.56 - 7.46 (m, 3H), 7.43 (d, *J* = 7.2 Hz, 1H), 7.35 - 7.27 (m, 2H), 7.27 - 7.20 (m, 3H), 7.20 - 7.11 (m, 3H), 7.11 - 6.98 (m, 2H), 5.28 (s, 2H), 4.69 (d, *J* = 10.0 Hz, 1H), 4.58 (d, *J* = 8.2 Hz, 1H), 4.39 (s, 1H), 2.94 - 2.81 (m, 1H), 2.70 (d, *J* = 13.6 Hz, 1H), 2.45 - 2.33 (m, 2H), 2.18 (t, *J* = 10.2 Hz, 1H), 1.80 (d, *J* = 10.9 Hz, 1H), 1.07 (d, *J* = 9.5 Hz, 1H), 0.91 - 0.71 (m, 2H). $^{13}C$ NMR (101 MHz, DMSO-$d_6$) δ 157.01, 154.82, 153.80, 143.05, 141.12, 136.82, 135.03, 133.32, 130.53, 129.67, 128.68, 128.58, 127.39, 126.54, 122.49, 122.03, 121.34, 121.03, 119.62, 117.54, 112.47, 109.90, 72.16, 70.51, 63.46, 61.28, 60.19, 49.73, 46.71, 45.56, 45.44, 42.84, 35.86, 30.93, 29.32. LC-MS (ESI): calcd for $C_{39}H_{41}N_7O_2$: 640.33945 $[M+H]^+$, found: 640.29 $[M+H]^+$, $R_t$ = 5.62 min; HR-MS found 640.33988 $[M+H]^+$.

**Example 49: Preparation of tert-Butyl 4-((S)-1-azido-2-oxo-2-((S)-2-oxo-4-phenyloxazolidin-3-yl)ethyl)piperidine-1-carboxylate (S49)**

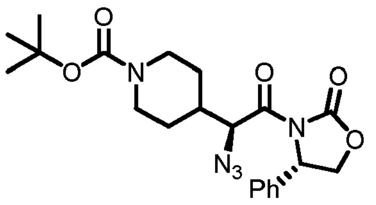

The product was synthesized according to literature methods.[1]
$^1H$ NMR (400 MHz, $CDCl_3$) δ 7.45 - 7.27 (m, 5H), 5.45 (dd, *J* = 8.7, 4.0 Hz, 1H), 4.98 (d, *J* = 7.8 Hz, 1H), 4.75 (t, *J* = 8.9 Hz, 1H), 4.33 (dd, *J* = 9.0, 4.0 Hz, 1H), 4.23 - 4.06 (m, 2H), 2.74 - 2.59 (m, 2H), 2.11-1.97 (m, 1H), 1.84-1.74 (m, 1H), 1.53-1.63 (m, 1H), 1.45 (s, 9H), 1.41-1.32 (m, 2H). Analytical data are consistent with literature data.

**Example 50: Preparation of (S)-tert-Butyl 4-(2-hydroxy-1-(2-nitrophenylamino) ethyl)piperidine-1-carboxylate (S50)**

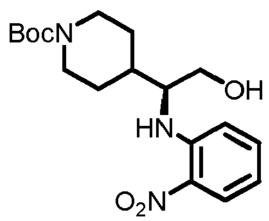

To a solution of azide **S49** (2.0 g, 4.7 mmol) in MeOH (5 mL) was added $NaBH_4$ (530 mg, 14.1 mmol) at room temperature. The reaction mixture was stirred at room temperature for 2 h and $NaBH_4$ (530 mg, 14.1 mmol) was added to the reaction mixture. After stirring for an additional 12 h sat. $NH_4Cl$-solution (20 mL) was added, followed by $CH_2Cl_2$ (30 mL). The aqueous layer was extracted with $CH_2Cl_2$ (3x 30 mL). The combined organic layers were dried ($Na_2SO_4$), filtered, evaporated to dryness and the residue was purified by flash chromatography (gradient cyclohexane : ethyl acetate 10:1 to 1:1). To a solution of the intermediate azido alcohol (1.0 g, 3.67 mmol) in degassed ethanol was added palladium on carbon (5%, 100 mg). The reaction vessel was evacuated and then refilled with argon three times. The reaction mixture was stirred under $H_2$ (1 atm) for 2 h at room temperature. The reaction vessel was then evacuated and refilled with argon, ethyl acetate (50 mL) was added and filtered over Celite. The filter cake was washed with ethanol and ethyl acetate (100 mL, respectively), evaporated to dryness and used without any further purification (LC-MS:ESI 244.89 $[M+H]^+$). To a solution of the free amine in NMP (10 mL) was added 1-fluoro-2-nitrobenzene (0.66 mL, 6.14 mmol), and DIPEA (0.35 mL, 2 mmol). The reaction mixture was heated to 90°C for 8 h, cooled to room temperature and diluted with $CH_2Cl_2$ (50 mL). To the reaction mixture was added a sat. of $NH_4Cl$ solution (40 mL). The aqueous

layer was extracted with $CH_2Cl_2$ (2x 50 mL). The combined organic layers were dried over $MgSO_4$, filtered and then evaporated to dryness. The crude product was purified by column chromatography to yield compound **S50** (1.35 g, 3.7 mmol, 45%).

$^1$H NMR (400 MHz, $CDCl_3$) δ 8.13 (d, *J* = 9.1 Hz, 1H), 8.09 (dd, *J* = 8.6, 1.5 Hz, 1H), 7.33 (dd, *J* = 8.4, 7.2 Hz, 1H), 6.85 (d, *J* = 8.8 Hz, 1H), 6.57 (dd, *J* = 8.4, 7.2 Hz, 1H), 4.19 - 3.97 (m, 2H), 3.77 (dd, *J* = 11.2, 4.2 Hz, 1H), 3.70 (dd, *J* = 11.1, 5.3 Hz, 1H), 3.59 - 3.48 (m, 1H), 2.61 (t, *J* = 12.3 Hz, 2H), 2.00-1.96 (m, 1H), 1.87 - 1.57 (m, 4H), 1.37 (s, 9H), 1.31 - 1.10 (m, 3H). $^{13}$C NMR (101 MHz, $CDCl_3$) δ 154.92, 145.89, 136.52, 132.42, 127.38, 115.81, 114.42, 79.82, 62.56, 58.73, 38.44, 29.21, 28.74, 28.64. LC-MS (ESI): calcd for $C_{18}H_{27}N_3O_5$: 388.18429 $[M+Na]^+$, $R_t$ = 9.41 min; HR-MS found 388.18459 $[M+Na]^+$; $[\alpha]_D^{20}$ : -243.0° (c=1.6, $CH_2Cl_2$).

**Example 51: Preparation of (S)-tert-Butyl 4-(1-(2-nitrophenylamino)-2-(1-(1-(thiophen-3-ylmethyl)-1H-benzo[d] imidazol-2-yl)piperidine-4-carbonyloxy)ethyl)piperidine-1-carboxylate (S51)**

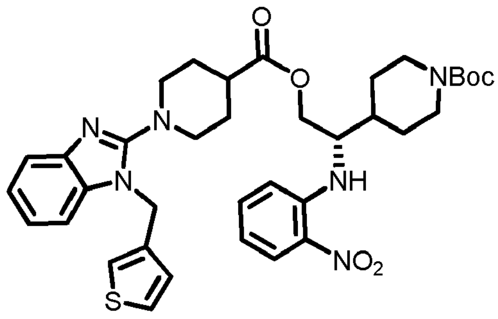

The product was synthesized according to Procedure D for the connection of two benzimidazoles via Mitsunobu reaction starting from alcohol **S50** (1.0 g. 2.74 mmol). The crude product was purified by automated flash chromatography using a gradient of cyclohexane: ethyl acetate 20:1 to 1:2 to yield compound **S51** (1.33 g, 1.94 mmol, 71 %).

$^1$H NMR (400 MHz, $CDCl_3$) δ 8.19 - 8.13 (m, 2H), 7.63 - 7.58 (m, 1H), 7.43 - 7.38 (m, 1H), 7.33 (dd, *J* = 5.0, 3.0 Hz, 1 H), 7.22 - 7.16 (m, 1 H), 7.13 - 7.10 (m, 2H), 7.04 - 6.99 (m, 1H), 6.97 - 6.89 (m, 2H), 6.64 (ddd, *J* = 8.1, 7.0, 1.1 Hz, 1H), 5.16 (s, 2H), 4.30 - 4.08 (m, 4H), 3.79 (td, *J* = 11.5, 5.5 Hz, 1H), 3.45 (d, *J* = 12.5 Hz, 2H), 2.96 (dd, *J* = 16.8, 7.0 Hz, 2H), 2.70 (t, *J* = 12.1 Hz, 2H), 2.47 (tt, *J* = 11.2, 4.1 Hz, 1H), 1.98 - 1.69 (m, 6H), 1.37 - 1.17 (m, 3H).

$^{13}$C NMR (101 MHz, $CDCl_3$) δ 174.55, 158.15, 154.85, 145.39, 141.69, 137.65, 136.53, 135.40, 132.60, 127.42, 127.32, 126.25, 122.20, 121.78, 121.74, 118.37, 116.08, 114.18, 109.52, 79.91, 64.16, 55.71, 50.60, 50.53, 44.17, 41.00, 38.96, 29.11, 28.65, 28.13. LC-MS (ESI): calcd for $C_{18}H_{27}N_3O_5$: 689.31158 $[M+H]^+$, found: 689.29 $[M+H]^+$ , $R_t$ = 8.47 min; HR-MS found 689.31133 $[M+H]^+$; $[\alpha]_D^{20}$ : -50.0°

(c=1.8, $CH_2Cl_2$).

**Example 52: Preparation of (S)-tert-Butyl 4-(1-(2-phenyl-1H-benzo[d]imidazol-1-yl)-2-(1-(1-(thiophen-3-ylmethyl)-1H-benzo[d]imidazol-2-yl)piperidine-4-carbonyloxy)ethyl)piperidine-1-carboxylate (*S*)-S41)**

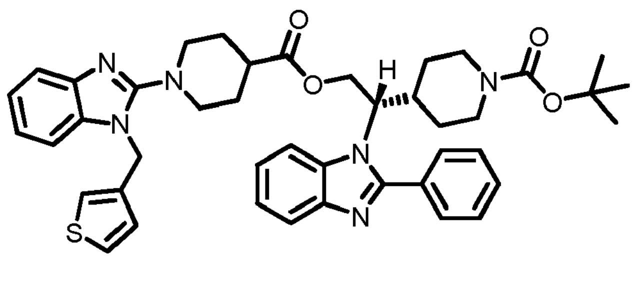

To a solution of **S51** (30 mg, 0,044 mmol) in MeOH, $H_2O$ and DMSO (8:2:1, 3 mL) was added benzaldehyde (9μL, 0.09 mmol) and sodium dithionite (23 mg, 0.14 mmol). The reaction mixture was heated to 65°C and after 0.5, 1, and 2 h sodium dithionite (3x 23 mg, 0.14 mmol) was added. After 5 h the reaction mixture was allowed to cool to room temperature. The reaction mixture was concentrated under reduced pressure and then extracted with $CH_2Cl_2$ (3x 20 mL). The combined organic phases were dried ($Na_2SO_4$), filtered and concentrated to dryness. Purification by flash chromatography (gradient cyclohexane : ethyl acetate 5:1 to 1:5) afforded enantiomerically pure (*S*)-**S41** (20 mg, 0.031 mmol, 61 %).

**Example 53: Preparation of (*S*)-tert-Butyl 4-(2-(4-(1-benzyl-1H-benzo[d]imidazol-2-yl)phenoxy)-1-(2-nitrophenylamino)ethyl)piperidine-1-carboxylate (S52)**

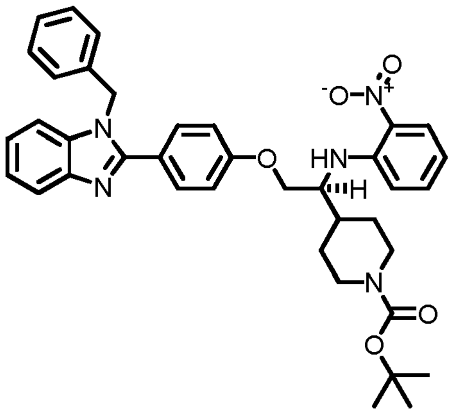

The product was synthesized according to Procedure C for the connection of two benzimidazoles via Mitsunobu reaction starting from alcohol **S50** (30 mg. 0.10 mmol) The crude product was purified by automated flash chromatography using a gradient of of cyclohexane: ethyl acetate 20:1 to 1:2 to yield compound **S52** (45 mg, 0.07 mmol, 70%). $^1H$ NMR (400 MHz, $CDCl_3$) δ 8.36 (d, *J* = 9.1 Hz, 1H), 8.17 (dd, *J* = 8.6, 1.6 Hz, 1H), 7.83 (t, *J* = 7.3 Hz, 1H), 7.63 - 7.58 (m, 2H), 7.45 - 7.39 (m, 1H), 7.36 - 7.26 (m, 4H), 7.24 - 7.15 (m, 2H), 7.13 - 7.07 (m, 2H), 6.97 - 6.89 (m, 3H), 6.70 - 6.62 (m, 1H), 5.42 (s, 2H), 4.35 - 4.01 (m, 3H), 3.97 - 3.77 (m, 1H), 2.72 (t, *J* = 12.0 Hz, 2H), 2.10 - 1.99 (m, 1H), 1.90 (d, *J* = 12.6 Hz, 1H), 1.77 (d, *J* = 12.7 Hz, 1H), 1.49 - 1.21 (m, 12H). $^{13}C$ NMR (101 MHz, $CDCl_3$) δ 159.86, 154.88, 154.08, 145.31, 143.28, 136.66, 136.51, 136.32, 132.56, 131.01, 129.29, 127.98, 127.45, 126.10, 123.34, 123.12, 122.87, 119.96, 115.91, 115.04, 114.10, 110.60, 79.84, 67.75, 56.27, 48.58, 38.80, 29.21, 28.70, 28.65. $[\alpha]_D^{20}$ : -62.5° (c=1.5, $CH_2Cl_2$).

**Example 54: Preparation of (*S*)-tert-Butyl 4-(1-(2-(3-cyanophenyl)-1H-benzo[d]imidazol-1-yl)-2-(1-(1-(thiophen-3-ylmethyl)-1H-benzo[d] imidazol-2-yl)piperidine-4-carbonyloxy)ethyl)piperidine-1-carboxylate (S53)**

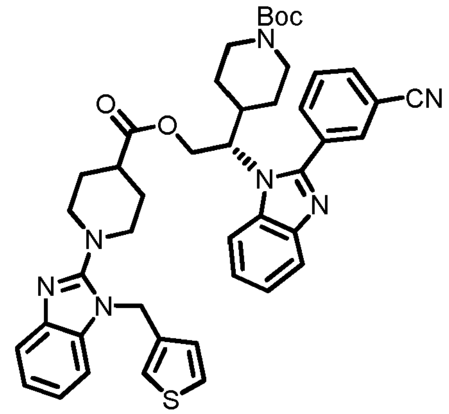

To a solution of **S51** (100 mg, 0.15 mmol) in MeOH, $H_2O$ and DMSO (8:2:1, 5 mL) was added 3-cyano benzaldehyde (43 mg, 0.33 mmol) and sodium dithionite (81 mg, 0.46 mmol). The reaction mixture was heated to 65°C and after 0.5, 1, and 2 h sodium dithionite (3x 81 mg, 0.46 mmol) was added. After 5 h the reaction mixture was allowed to cool to room temperature. The reaction mixture was concentrated under reduced pressure and then extracted with $CH_2Cl_2$ (3x 50 mL). The combined organic phases were dried ($Na_2SO_4$), filtered and concentrated to dryness. Purification by flash chromatography (gradient cyclohexane : ethyl acetate 5:1 to 1:5) afforded **S53** (74 mg, 0.010 mmol, 64%).
$^1H$ NMR (400 MHz, $CDCl_3$) $\delta$ 8.06 - 7.95 (m, 2H), 7.87 (dd, *J* = 12.8, 5.8 Hz, 2H), 7.74 (t, *J* = 7.4 Hz, 2H), 7.60 (d, *J* = 5.1 Hz, 1H), 7.46 - 7.27 (m, 5H), 7.17 (d, *J* = 7.9 Hz, 1H), 7.08 (d, *J* = 1.4 Hz, 1H), 6.93 (d, *J* = 5.0 Hz, 1H), 5.20 (s, 2H), 5.04 - 4.91 (m, 1H), 4.62 (dd, *J* = 11.9, 3.8 Hz, 1H), 4.38 (t, *J* = 7.9 Hz, 1H), 4.17 (d, *J* = 13.3 Hz, 1H), 4.00 - 3.83 (m, 1H), 3.77 - 3.56 (m, 2H), 3.42 - 3.22 (m, 2H), 2.70 (t, *J* = 12.3 Hz, 1H), 2.62 - 2.34 (m, 3H), 2.00 - 1.79 (m, 3H), 1.79 - 1.53 (m, 2H), 1.40 (s, 9H), 1.24 (t, *J* = 10.1 Hz, 1H), 0.96 - 0.67 (m, 2H). $^{13}C$ NMR (101 MHz, $CDCl_3$) $\delta$ 173.09, 161.89, 161.53, 154.66, 153.12, 152.33, 141.54, 134.45, 134.10, 133.52, 132.74, 132.02, 130.82, 130.63, 130.54, 128.67, 125.59, 125.53, 124.92, 124.74, 124.36, 122.56, 120.44, 118.01, 117.93, 115.05, 113.60, 112.28, 110.59, 80.21, 63.52, 61.71, 49.35, 46.05, 39.68, 37.35, 29.93, 28.83, 28.57, 27.50. LC-MS (ESI): calcd for $C_{18}H_{27}N_3O_5$: 689.31158 $[M+H]^+$, found: 689.29 $[M+H]^+$, $R_t$ = 8.47 min; HR-MS found 689.31133 $[M+H]^+$.

**Example 55: Preparation of 1-((19R,21R)-1-(3',6'-Dihydroxy-3-oxo-3H-spiro[isobenzofuran-1,9'-xanthene]-5-ylamino)-19,21-dihydroxy-17-oxo-1-thioxo-6,9,12-trioxa-2,16-diazatricosan-23-yl)-5-(4-fluorophenyl)-2-isopropyl-N,4-diphenyl-1H-pyrrole-3-carboxamide (S54)**

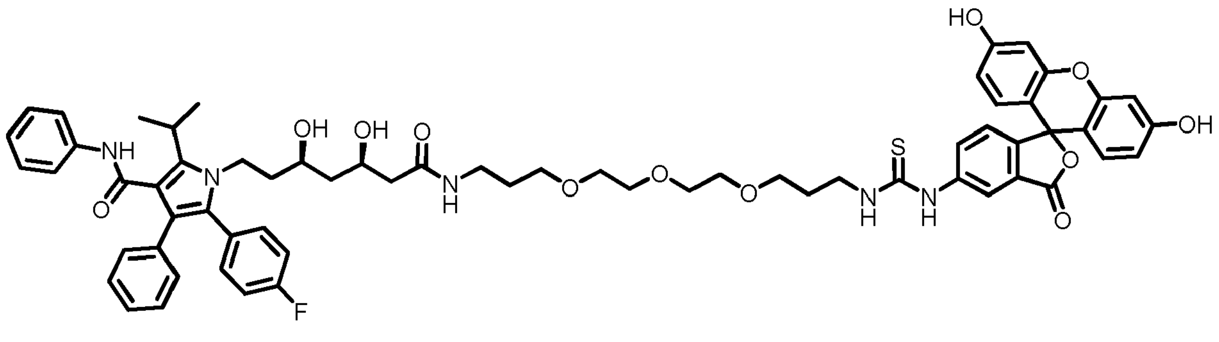

To Atorvastatin (20 mg, 35 µmol) in DMF (200 µL) was added DIPEA (24 µL, 140 µmol), FITC-PEG$_3$-NH$_2$[9] (90 mg, 148 µmol) and PyBOP (27 mg, 50 µmol). The reaction mixture was stirred for 36 hours and then directly applied to a reverse phase C-18 column (gradient water: acetontrile 9:1 to 1:5 containing 0.1% TFA) to yield fluorescein-labeled atorvastatin (17 mg, 15 µmol, 41 %).
$^1H$ NMR (600 MHz, DMSO-$d_6$) $\delta$ 9.89 (s, 1H), 9.77 (s, 1H), 8.19 (s, 1H), 8.05 (s, 1H), 7.75 (t, *J* = 5.6 Hz, 1H), 7.69 (s, 1H), 7.47 (d, *J* = 8.0 Hz, 2H), 7.24 - 7.13 (m, 8H), 7.07 - 7.01 (m, 4H), 6.99 - 6.92 (m, 2H), 6.66 - 6.63 (m, 2H), 6.59 - 6.52 (m, 4H), 3.94 - 3.87 (m, 1H), 3.79 (dd, *J* = 11.9, 7.1 Hz, 1H), 3.75 - 3.68 (m, 2H), 3.24 - 3.15 (m, 2H), 3.10 - 2.98 (m, 3H), 2.09 (d, *J* = 6.3 Hz, 2H), 1.78 (p, *J* = 6.5 Hz, 2H), 1.64 - 1.53 (m, 4H), 1.53 - 1.43 (m, 1H), 1.41 - 1.30 (m, 8H), 1.28 - 1.17 (m, 2H). $^{13}C$ NMR (151 MHz, DMSO-$d_6$) $\delta$ 171.26, 169.20, 166.81, 161.35, 160.14, 152.53, 136.57, 135.55,

129.79, 129.68, 129.32, 129.11, 128.30, 127.93, 126.03, 123.65, 121.22, 120.04, 118.06, 116.11, 115.96, 113.25, 110.37, 102.88, 70.42, 70.39, 70.23, 70.19, 68.73, 66.63, 66.46, 44.45, 44.26, 41.48, 39.38, 36.36, 29.94, 29.19, 22.98; LC-MS (ESI): calcd for $C_{64}H_{68}FN_5O_{12}S$: 1150.46420 [M+H]$^+$, found: 1150.21 [M+H]$^+$, $R_t$ = 8.81 min; HR-MS found 1151.46229 [M+H]$^+$.

**Example 56: Preparation of 1-Benzyl-2-phenyl-1H-benzo[d]imidazole (1)**

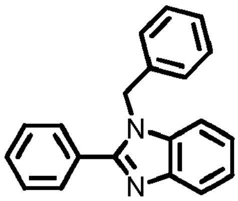

The N-benzylation of 2-phenyl benzimidazole (185 mg, 0.92 mmol) was performed following the general procedure described above. The crude product was purified by flash column chromatography (gradient of cyclohexane : ethyl acetate 10:1 to 1:1) to yield compound 1 (212 mg, 0.74 mmol, 86%).
$^1$H NMR (400 MHz, DMSO-$d_6$) δ 7.76 - 7.67 (m, 3H), 7.57 - 7.47 (m, 3H), 7.47 - 7.42 (m, 1H), 7.31 - 7.16 (m, 5H), 6.98 (d, J = 7.3 Hz, 2H), 5.57 (s, 2H). $^{13}$C NMR (101 MHz, DMSO-$d_6$) δ 153.94, 143.37, 137.60, 136.57, 130.84, 130.50, 129.72, 129.45, 128.15, 126.77, 123.37, 122.89, 119.95, 111.78, 48.15. LC-MS (ESI): calcd for $C_{20}H_{16}N_2$ : 285.13863 [M+H]$^+$, found 285.01 [M+H]$^+$, $R_t$ = 5.90 min; HR-MS found 285.13883 [M+H]$^+$.

**Example 57: Preparation of 6-Pyiridyl-5,6-dihydrobenzo[4-5]imidazo[1,2-c]quinazoline (2)**

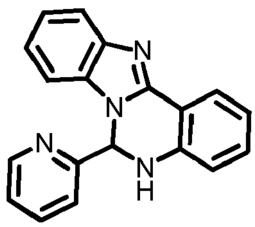

A mixture of 2-pyridinecarboxaldehyde (99 mg, 0.93 mmol) and 2-(2-aminophenyl)-1H-benzimid-azole (200 mg, 0.93 mmol) was dissolved in acetic acid (2 mL). The reaction mixture was stirred at 65°C for 16 h. After cooling to room temperature the reaction mixture was diluted with $CH_2Cl_2$ (15 mL) and a sat. $NaHCO_3$ - solution was slowly added until a basic pH was reached. The aqueous layer was extracted with $CH_2Cl_2$ (2 x 15 mL). The combined organic phases were dried over $Na_2SO_4$, filtered and the solvent was removed under reduced pressure. The crude product was purified by flash column chromatography (gradient of $CH_2Cl_2$: MeOH 50:1 to 5:1) to yield compound **2.** (196 mg, 0.66 mmol, 71 %).
$^1$H NMR (400 MHz, DMSO-$d_6$) δ 8.51 - 8.35 (m, 1H), 7.92 (d, *J* = 7.7 Hz, 1H), 7.73 (td, *J* = 6.1, 3.0 Hz, 1H), 7.69 (d, *J* = 2.0 Hz, 1H), 7.65 - 7.59 (m, 1H), 7.35 - 7.30 (m, 1H), 7.30 - 7.24 (m, 1H), 7.24 - 7.06 (m, 5H), 6.84 (d, *J* = 8.2 Hz, 1H), 6.81 - 6.70 (m, 1H). $^{13}$C NMR (101 MHz, DMSO-*$d_6$*) δ 159.05, 150.14, 147.51, 144.46, 143.64, 138.10, 133.69, 132.20, 125.25, 124.61, 122.85, 122.70, 120.87, 119.25, 118.70, 115.36, 112.70, 111.07, 69.04. LC-MS (ESI): calcd for $C_{19}H_{14}N_4$: 299.12912 [M+H]$^+$, found 299.01 [M+H]$^+$, $R_t$ = 2.70 min; HR-MS found 299.12929 [M+H]$^+$.

**Example 58: Preparation of 1-Benzyl-2-(4-(2-(2-phenyl-1H-benzo[d]imidazol-1-yl)pent-4-enyloxy)phenyl)-1H-benzo[d]imidazole (3)**

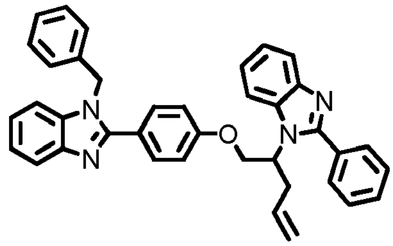

The product was synthesized from phenol **S7** (76 mg, 0.25 mmol) and alcohol **S20** according to Procedure C for the synthesis of phenol ethers via Mitsunobu reaction. The crude product was purified by automated flash chromatography using a gradient of of cyclohexane: ethyl acetate 10:1 to 1:7 to yield the product (100 mg, 0.18 mmol, 72 %). $^{1}$H NMR (400 MHz, DMSO-$d_6$) δ 7.99 - 7.89 (m, 1H), 7.75 - 7.63 (m, 4H), 7.57 (t, *J* = 11.0 Hz, 2H), 7.54 - 7.43 (m, 3H), 7.38 (dt, *J* = 8.8, 4.5 Hz, 1H), 7.34 - 7.06 (m, 8H), 6.98 (dd, *J* = 7.7, 5.4 Hz, 4H), 5.51 (s, 2H), 5.50 - 5.33 (m, 1H), 4.91 - 4.71 (m, 4H), 4.44 (dd, *J* = 10.1, 3.6 Hz, 1H), 3.08 - 2.91 (m, 1H), 2.81 - 2.68 (m, 1H). $^{13}$C NMR (101 MHz, DMSO-$d_6$) δ 159.51, 155.59, 153.74, 143.91, 143.37, 137.66, 136.60, 133.94, 133.85, 131.47, 131.14, 130.59, 130.20, 129.47, 129.18, 128.11, 126.66, 123.48, 123.12, 123.01, 122.78, 122.61, 120.29, 119.73, 119.09, 115.33, 113.31, 111.59, 68.29, 56.84, 48.12, 33.72. LC-MS (ESI): calcd for $C_{38}H_{32}N_4O$: 561.26489 [M+H]$^+$, found: 561.36 [M+H]$^+$, Rt = 7.19 min; HR-MS found 561.26523 [M+H]$^+$.

**Example 59: Preparation of 1-Benzyl-2-(4-(2-cyclohexyl-2-(2-phenyl-1H-benzo[d]imidazol-1-yl)ethoxy)phenyl)-1H-benzo[d]imidazole (4)**

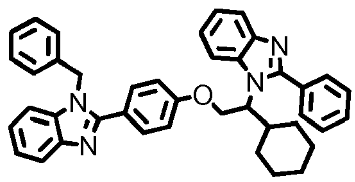

The product was synthesized from phenol **S7** (50 mg, 0.17 mmol) and alcohol **S16** according to Procedure C for the synthesis of phenol ethers via Mitsunobu reaction. The crude product was purified by automated flash chromatography using a gradient of of cyclohexane: ethyl acetate 10:1 to 1:7 to yield the product (63 mg, 0.11 mmol, 63%). $^{1}$H NMR (600 MHz, DMSO-$d_6$) δ 7.91 (d, *J* = 7.3 Hz, 1H), 7.73 (d, *J* = 6.9 Hz, 2H), 7.66 (td, *J* = 7.1, 4.6 Hz, 2H), 7.58 (t, *J* = 10.6 Hz, 2H), 7.57 - 7.49 (m, 3H), 7.39 (d, *J* = 7.6 Hz, 1H), 7.28 - 7.15 (m, 7H), 7.04 (d, *J* = 8.6 Hz, 2H), 6.96 (d, *J* = 7.4 Hz, 2H), 5.51 (s, 2H), 4.85 (t, *J* = 9.9 Hz, 1H), 4.61 - 4.54 (m, 1H), 4.45 (t, *J* = 8.4 Hz, 1H), 2.37 (d, *J* = 10.8 Hz, 1H), 1.99 - 1.92 (m, 1H), 1.64 (d, *J* = 13.2 Hz, 1H), 1.53 - 1.35 (m, 3H), 1.28 - 1.17 (m, 1H), 1.05 - 0.75 (m, 4H), 0.59 - 0.49 (m, 1H). $^{13}$C NMR (151 MHz, DMSO-$d_6$) δ 159.71, 155.88, 153.73, 143.76, 143.29, 137.68, 136.58, 133.93, 131.13, 130.66, 130.27, 129.47, 129.28, 128.10, 126.64, 123.39, 123.13, 123.06, 122.80, 122.63, 120.24, 119.68, 115.39, 113.35, 111.61, 109.99, 67.38, 62.19, 48.08, 37.36, 30.74, 29.64, 26.11, 25.84, 25.79. LC-MS (ESI): calcd for $C_{41}H_{38}N_4O$: 603.31184 [M+H]$^+$, found: 603.39 [M+H]$^+$, $R_t$ = 8.14 min; HR-MS found 603.31156 [M+H]$^+$.

**Example 60: Preparation of 4-(4-(1-Benzyl-1H-benzo[d]imidazol-2-yl)phenoxy)-3-(2-phenyl-1H-benzo[d]imidazol-1-yl)butanoic acid (5)**

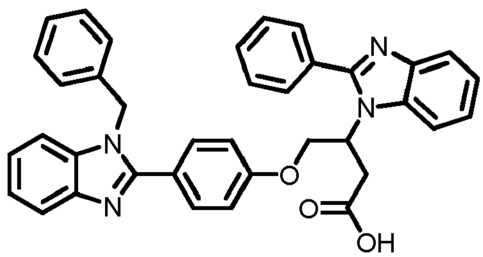

To a suspension of **3** (20 mg, 36 μmol) in a mixture of $CCl_4$, acetonitrile and water 1:1:1.5 (2 mL) was added sodium periodate (31 mg, 140 μM) and $RuCl_3$ (0.3 mg). After 24h of stirring, sodium periodate (31 mg, 140 μM) and $RuCl_3$ (0.3 mg) were added. The reaction mixture was stirred for an additional 2 d. The reaction mixture was concentrated *in vacuo.* The aqueous residue was extracted with $CH_2Cl_2$ (3x10 mL). The combined organic phases were dried ($Na_2SO_4$), filtered and concentrated *in vacuo.* The crude reaction mixture was subjected to automated flash chromatography (gradient $CH_2Cl_2$: MeOH 100:1 to 15:1) to afford compound 5 (13 mg, 22 μmol, 62%).
$^1$H NMR (400 MHz, DMSO-$d_6$) δ 7.91 (d, *J* = 7.1 Hz, 1H), 7.76 (dd, *J* = 7.2, 2.0 Hz, 2H), 7.65 (dd, *J* = 10.7, 4.8 Hz, 2H), 7.56 (d, *J* = 8.7 Hz, 2H), 7.48 (dd, *J* = 8.2, 2.8 Hz, 3H), 7.39 (d, *J* = 6.4 Hz, 1H), 7.32 - 7.14 (m, 7H), 6.96 (t, *J* = 8.0 Hz, 4H), 5.50 (s, 2H), 5.23 (s, 1H), 4.71 (t, *J* = 9.9 Hz, 1H), 4.39 (dd, *J* = 10.7, 4.0 Hz, 1H), 3.25 - 3.14 (m, 2H).$^{13}$C NMR (101 MHz, DMSO-$d_6$) δ 159.42, 155.70, 153.73, 143.95, 143.35, 137.67, 136.57, 133.90, 131.60, 131.12, 130.59, 130.07, 129.47, 128.95, 128.11, 126.68, 123.49, 123.11, 122.77, 122.60, 120.24, 119.72, 115.25, 113.18, 111.60, 68.23, 53.52, 48.10, 34.96, 29.68. LC-MS (ESI): calcd for $C_{37}H_{30}N_4O_3$: 579.23907 $[M+H]^+$, found: 579.36 $[M+H]^+$, $R_t$ = 6.26 min; HR-MS found 579.23912 $[M+H]^+$.

**Example 61: Preparation of 1-Benzyl-2-(4-(2-(2-phenyl-1H-benzo[d]imidazol-1-yl)ethoxy)phenyl)-1H-benzo[d] imidazole (6)**

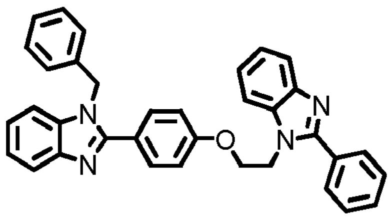

The product was synthesized from phenol **S7** (50 mg, 0.17 mmol) and alcohol **S21** according to Procedure C for the synthesis of phenol ethers via Mitsunobu reaction. The crude product was purified by automated flash chromatography using a gradient of of cyclohexane: ethyl acetate 6:1 to 1:9 to yield the product (52 mg, 0.10 mmol, 59%).
$^1$H NMR (400 MHz, DMSO-$d_6$) δ 7.86 — 7.79 (m, 2H), 7.77 (d, *J* = 7.9 Hz, 1H), 7.70 - 7.62 (m, 2H), 7.60 - 7.45 (m, 5H), 7.38 (d, *J* = 6.8 Hz, 1H), 7.33 - 7.13 (m, 7H), 6.96 (d, *J* = 7.2 Hz, 2H), 6.86 (d, *J* = 8.7 Hz, 2H), 5.49 (s, 2H), 4.70 (t, *J* = 5.0 Hz, 2H), 4.36 (t, *J* = 5.1 Hz, 2H). $^{13}$C NMR (101 MHz, DMSO-$d_6$) δ 159.51, 154.48, 153.74, 143.35, 137.64, 136.56, 136.25, 131.22, 131.09, 130.27, 129.45, 129.29, 128.10, 126.68, 123.36, 123.14, 123.10, 122.77, 119.84, 119.71, 115.17, 111.94, 111.59, 66.71, 48.09, 44.41. LC-MS (ESI): calcd for $C_{35}H_{28}N_4O$: 521.23359 $[M+H]^+$, found: 521.34 $[M+H]^+$, Rt = 6.37 min; HR-MS found 521.23314 $[M+H]^+$.

**Example 62: Preparation of N-(18-(4-(1-Benzyl-1H-benzo[d]imidazol-2-yl)phenoxy)-15-oxo-17-(2-phenyl-1H-benzo[d]imidazol-1-yl)-4,7,10-trioxa-14-azaoctadecyl)-5-((3aS,4S,6aR)-2-oxohexahydro-1H-thieno[3,4-d]imidazol-4-yl)pentanamide (7)**

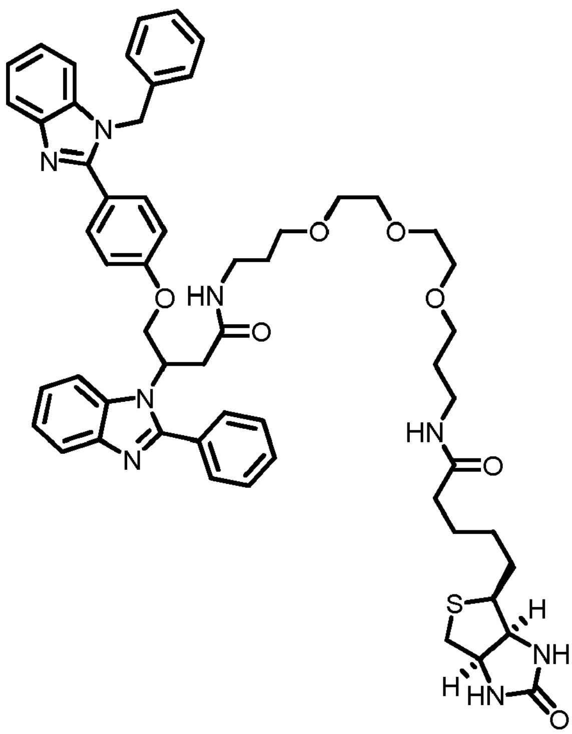

To a mixture of acid 5 (13 mg, 22 μmol), Biotin-PEG-$NH_2$·TFA (38 mg, 67 μmol) in DMF (1 mL) at room temperature was added DIPEA (32 μL, 180 μmol) and PyBOP (54 mg, 100 μmol). The reaction mixture was stirred at room temperature for 2 d and then concentrated *in vacuo.* The residue was dissolved in methanol (500 μL) and applied to a preparative C18-RP column eluting with a gradient ($CH_3CN$:$H_2O$ 1:4 to 9:1 containg 0.1% TFA), to afford the product as a mixture of diastereomers (16 mg, 16 μmol, 71%).
$^1$H NMR (400 MHz, DMSO-$d_6$) δ 8.12 (d, *J* = 7.3 Hz, 1H), 8.06 (t, *J* = 5.6 Hz, 1H), 7.91 - 7.84 (m, 2H), 7.84 - 7.74 (m, 2H), 7.75 - 7.62 (m, 4H), 7.62 - 7.54 (m, 3H), 7.53 - 7.39 (m, 4H), 7.35 - 7.22 (m, 3H), 7.14 - 7.01 (m, 4H), 6.40 (s, 2H), 5.64 (s, 2H), 5.35 (s, 1H), 4.83 - 4.74 (m, 1H), 4.47 (dd, *J* = 11.0, 3.7 Hz, 1H), 4.28 (dd, *J* = 7.8, 5.0 Hz, 1H), 4.10 (dd, *J* = 7.7, 4.5 Hz, 1H), 3.51 - 3.38 (m, 6H), 3.38 - 3.30 (m, 4H), 3.30 - 3.20 (m, 1H), 3.18 - 3.10 (m, 3H), 3.10 - 2.97 (m, 4H), 2.96 - 2.86 (m, 1H), 2.83 — 2.73 (m, 1H), 2.55 (d, *J* = 12.4 Hz, 1H), 2.51 - 2.44 (m, 2H), 2.10 — 1.96 (m, 2H), 1.68 - 1.54 (m, 3H), 1.54 - 1.36 (m, 5H), 1.36 - 1.15 (m, 2H). $^{13}$C NMR (101 MHz, DMSO-$d_6$) δ 172.62, 168.60, 163.40, 160.84, 159.10, 158.76, 153.98, 151.92, 135.88, 134.17, 132.06, 131.90, 130.91, 129.60, 129.45, 128.65, 127.04, 125.90, 125.68, 125.22, 118.05, 117.71, 116.58, 115.82, 115.36, 114.63, 113.38, 70.41, 70.37, 70.20, 70.14, 68.78, 68.27, 61.74, 59.90, 56.10, 54.84, 48.86, 36.41, 36.36, 35.89, 35.21, 30.09, 29.71, 28.89, 28.72, 25.98. LC-MS (ESI): calcd for $C_{57}H_{66}N_8O_7S$: 1007.48479 [M+H]$^+$, found: 1007,60 [M+H]+, Rt = 5.89 min; HR-MS found 1007.48529 [M+H]$^+$.

**Example 63: Preparation of tert-Butyl-4-(2-(4-(1-benzyl-1H-benzo[d]imidazol-2-yl)phenoxy)-1-(2-phenyl-1H-benzo[d]imidazol-1-yl)ethyl)piperidine-1-carboxylate (8)**

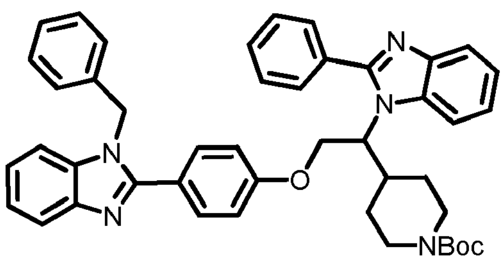

The product was synthesized from phenol **S7** (60 mg, 0.14 mmol) and alcohol **S17** according to Procedure C for the synthesis of phenol ethers via Mitsunobu reaction. The crude product was purified by automated flash chromatography using a gradient of of cyclohexane: ethyl acetate 10:1 to 1:7 to yield the product (66 mg, 0.09 mmol, 66%). $^1$H NMR (400 MHz, DMSO-$d_6$) δ 7.94 (d, *J* = 6.3 Hz, 1H), 7.78 - 7.64 (m, 4H), 7.64 - 7.49 (m, 5H), 7.40 (d, *J* = 7.9 Hz, 1H), 7.31 - 7.15 (m, 7H), 7.04 (d, *J* = 8.2 Hz, 2H), 6.97 (d, *J* = 7.2 Hz, 2H), 5.51 (s, 2H), 4.85 (d, *J* = 9.5 Hz, 1H), 4.58 (d, *J* = 9.6 Hz, 1H), 4.48 (s, 1H), 3.91 (d, *J* = 12.6 Hz, 1H), 3.70 (s, 1H), 2.80 - 2.52 (m, 2H), 2.45 - 2.31 (m, 1H), 1.92 (d, *J* = 11.6 Hz, 1H), 1.31 (s, 9H), 1.19-1.00 (d, *J* = 9.6 Hz, 2H), 0.92 - 0.63 (m, 2H). $^{13}$C NMR (101 MHz, DMSO-$d_6$) δ 159.68, 155.83, 154.29, 153.69, 143.76, 143.18, 137.64, 136.56, 131.16, 130.69, 130.31, 129.46, 129.29, 128.11, 126.66, 123.39, 123.17, 122.84, 122.75, 120.29, 119.65, 115.42, 113.29, 111.62, 79.30, 67.12, 61.64, 48.13, 35.89, 29.79, 29.08, 28.71. LC-MS (ESI): calcd for $C_{45}H_{45}N_5O_3$ : 704.35952 [M+H]$^+$, found: 704.31 [M+H]$^+$, $R_t$ = 7.68 min; HR-MS found 704.36024 [M+H]$^+$. Chiral, analytical HPLC conditions: flow rate: 0.5 mL/min; solvent: 40 % ethanol in *iso*-hexane. (*R*)-**8** $R_t$ =90.1 min, (*S*)-**8** 100.9 min.

**Example 64: Preparation of (S)-tert-Butyl 4-(2-(4-(1-benzyl-1H-benzo[d]imidazol-2-yl)phenoxy)-1-(2-phenyl-1H-benzo[d]imidazol-1-yl)ethyl)piperidine-1-carboxylate (S)-(8)**

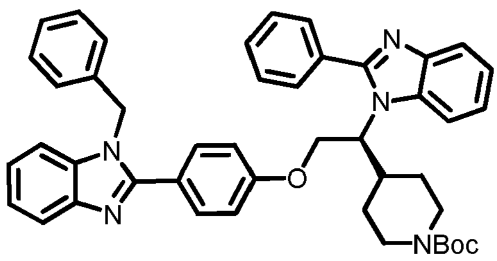

To a solution of **S52** (40 mg, 0.066 mmol) in MeOH, $H_2O$ and DMSO (8:2:1, 2 mL) was added benzaldehyde (18 μL, 0.18 mmol) and sodium dithionite (23 mg, 0.14 mmol). The reaction mixture was heated to 65°C and after 0.5, 1, and 2 h sodium dithionite (3x 23 mg, 0.14 mmol) was added. After 6 h the reaction mixture was allowed to cool to room temperature. The reaction mixture was concentrated under reduced pressure and then extracted with $CH_2Cl_2$ (3x 20 mL). The combined organic phases were dried ($Na_2SO_4$), filtered and concentrated to dryness. Purification by flash chromatography (gradient cyclohexane : ethyl acetate 5:1 to 1:5) afforded enantiomerically pure (***S***)-8 (25 mg, 0.035mmol, 53%).

**Example 65: Preparation of 1-Benzyl-2-(4-(2-(2-phenyl-1H-benzo[d]imidazol-1-yl)-2-(piperidin-4-yl)ethoxy)phenyl)-1H-benzo[d]imidazole (9)**

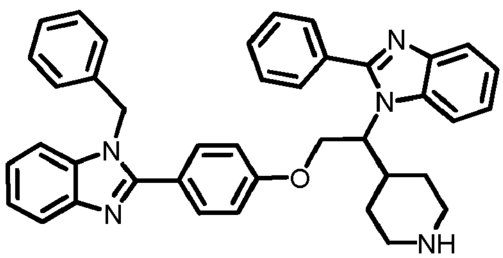

The product was synthesized according to General Procedure (D) starting from **8** (32 mg, 0.046 mmol) to afford the HCl-salt in quantitative yield. The residue was dissolved in methanol (500 μL) and applied to a preparative C18-RP column eluting with a gradient ($CH_3CN$: $H_2O$ 1:5 to 9:1 containing 0.1% TFA), to afford the desired piperdine (20 mg, 0.033 mmol, 71 %) as a TFA-salt.
$^1$H NMR (400 MHz, DMSO-$d_6$) δ 7.92 (d, *J* = 6.8 Hz, 1H), 7.77 - 7.71 (m, 2H), 7.69 - 7.63 (m, 2H), 7.63 - 7.49 (m, 5H), 7.41 - 7.36 (m, 1H), 7.30 - 7.14 (m, 7H), 7.05 (d, *J* = 8.6 Hz, 2H), 6.97 (d, *J* = 7.3 Hz, 2H), 5.51 (s, 2H), 4.86 (t, *J* = 9.9 Hz, 1H), 4.62 — 4.52 (m, 1H), 4.47 (t, *J* = 8.5 Hz, 1H), 2.89 (d, *J* = 12.0 Hz, 1H), 2.68 (d, *J* = 12.0 Hz, 1H), 2.46 — 2.34 (m, 1H), 2.28 — 2.11 (m, 1H), 1.89 — 1.80 (m, 1H), 1.13 — 0.98 (m, 1H), 0.77 - 0.51 (m, 2H). $^{13}$C NMR (101 MHz, DMSO-$d_6$) δ 159.70, 155.88, 153.74, 143.78, 143.36, 137.68, 136.61, 133.94, 131.49, 131.14, 130.68, 130.27, 129.46,

129.28, 128.10, 126.65, 123.48, 123.10, 122.78, 122.66, 120.27, 119.71, 115.40, 113.27, 111.58, 67.15, 62.29, 48.11, 46.11, 46.01, 36.24, 31.25, 29.88. LC-MS (ESI): calcd for $C_{40}H_{37}N_5O$: 604.30709 $[M+H]^+$, found: 604.26 $[M+H]^+$, $R_t$ = 5.34 min; HR-MS found: 604.30716 $[M+H]^+$.

**Example 66: Preparation of (S)-1-Benzyl-2-(4-(2-(2-phenyl-1H-benzo[d]imidazol-1-yl)-2-(piperidin-4-yl)ethoxy) phenyl)-1H-benzo[d]imidazole ((S)-9)**

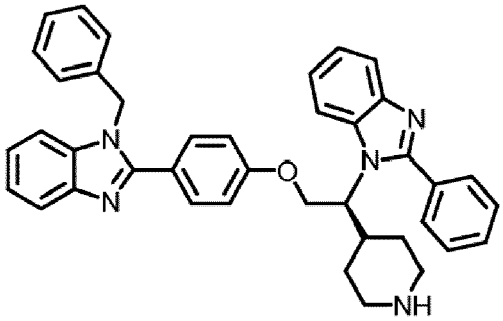

The product was synthesized according to General Procedure (D) starting from (S)-**8** (16 mg, 0.023 mmol) to afford the HCl-salt in quantitative yield. The residue was dissolved in methanol (400 μL) and applied to a preparative C18-RP column eluting with a gradient ($CH_3CN$: $H_2O$ 1:5 to 9:1 containing 0.1% TFA), to afford the desired piperdine (10 mg, 0.0165 mmol, 71%) as a TFA-salt.
$^1H$ NMR (400 MHz, DMSO-$d_6$) δ 7.92 (d, *J* = 6.8 Hz, 1H), 7.77 - 7.71 (m, 2H), 7.69 - 7.63 (m, 2H), 7.63 - 7.49 (m, 5H), 7.41 - 7.36 (m, 1H), 7.30 - 7.14 (m, 7H), 7.05 (d, *J* = 8.6 Hz, 2H), 6.97 (d, *J* = 7.3 Hz, 2H), 5.51 (s, 2H), 4.86 (t, *J* = 9.9 Hz, 1H), 4.62 — 4.52 (m, 1H), 4.47 (t, *J* = 8.5 Hz, 1H), 2.89 (d, *J* = 12.0 Hz, 1H), 2.68 (d, *J* = 12.0 Hz, 1H), 2.46 — 2.34 (m, 1H), 2.28 — 2.11 (m, 1H), 1.89 — 1.80 (m, 1H), 1.13 — 0.98 (m, 1H), 0.77 - 0.51 (m, 2H). $^{13}C$ NMR (101 MHz, DMSO-$d_6$) δ 159.70, 155.88, 153.74, 143.78, 143.36, 137.68, 136.61, 133.94, 131.49, 131.14, 130.68, 130.27, 129.46, 129.28, 128.10, 126.65, 123.48, 123.10, 122.78, 122.66, 120.27, 119.71, 115.40, 113.27, 111.58, 67.15, 62.29, 48.11, 46.11, 46.01, 36.24, 31.25, 29.88. LC-MS (ESI): calcd for $C_{40}H_{37}N_5O$: 604.30709 $[M+H]^+$, found: 604.26 $[M+H]^+$, $R_t$ = 5.34 min; HR-MS found: 604.30716 $[M+H]^+$.

**Example 67: Preparation of N-(2-(2-Phenyl-1H-benzo[d]imidazol-1-yl)-2-(piperidin-4-yl)ethyl)-1-(1-(thiophen-3-ylmethyl)-1H-benzo[d]imidazol-2-yl)piperidine-4-carboxamide (10)**

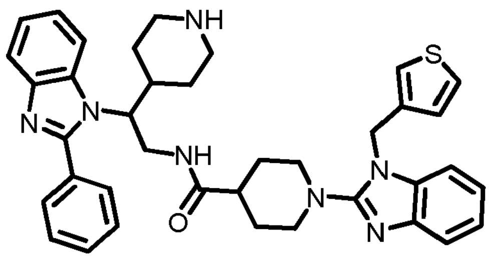

The product was synthesized according to General Procedure (D) starting from the protected piperidine (61 mg, 0.13 mmol) to afford the HCl-salt in quantitative yield. The residue was dissolved in methanol (500 μL) and applied to a preparative C18-RP column eluting with a gradient ($CH_3CN$: $H_2O$ 1:5 to 9:1 containing 0.1% TFA), to afford the desired free piperidine (66 mg, 0.010 mmol, 59%).
$^1H$ NMR (400 MHz, DMSO-$d_6$) δ 9.19 (s, 1H), 8.99 (s, 1H), 8.29 (t, *J* = 5.7 Hz, 1H), 8.15 (d, *J* = 7.9 Hz, 1H), 7.90 (d, *J* = 7.8 Hz, 1H), 7.77 (d, *J* = 7.5 Hz, 2H), 7.70 (t, *J* = 7.4 Hz, 1H), 7.64 (t, *J* = 7.5 Hz, 2H), 7.62 - 7.53 (m, 4H), 7.49 - 7.47 (m, 1H), 7.38 (t, *J* = 7.1 Hz, 1H), 7.36 - 7.32 (m, 1H), 7.31 - 7.27 (m, 1H), 7.08 - 7.04 (m, 1H), 5.39 (s, 2H), 4.19 (t, *J* = 8.8 Hz, 1H), 3.94 (dd, *J* = 11.7, 7.0 Hz, 1H), 3.77 (d, *J* = 9.6 Hz, 2H), 3.61 - 3.53 (m, 3H), 3.33 - 3.16 (m, 4H), 3.03 (dt, *J* = 12.3, 7.3 Hz, 2H), 2.90 - 2.61 (m, 4H), 2.28 (t, *J* = 10.8 Hz, 1H), 2.16 (d, *J* = 13.4 Hz, 1H), 1.76 - 1.71 (m, 2H), 1.71

- 1.62 (m, 1H), 1.62 - 1.45 (m, 3H), 1.44 - 1.29 (m, 3H), 1.16 — 1.09 (m, 1H). $^{13}C$ NMR (151 MHz, DMSO-$d_6$) δ 174.60, 152.43, 136.25, 132.57, 131.05, 129.92, 128.50, 127.09, 125.08, 124.53, 123.57, 115.17, 112.92, 112.06, 67.69, 64.33, 49.49, 49.32, 46.14, 45.95, 43.20, 43.12, 39.11, 34.30, 28.61, 27.51, 26.84, 25.80, 25.64. LC-MS (ESI): calcd for $C_{38}H_{41}N_7OS$ : 644.31661 $[M+H]^+$, found: 644.32 $[M+H]^+$, $R_t$ = 5.30 min; HR-MS found 644.31661 $[M+H]^+$.

**Example 68: Preparation of 2-(2-Phenyl-1H-benzo[d]imidazol-1-yl)-2-(piperidin-4-yl)ethyl 1-(1-benzyl-1H-benzo [d]imidazol-2-yl)piperidine-4-carboxylate (11)**

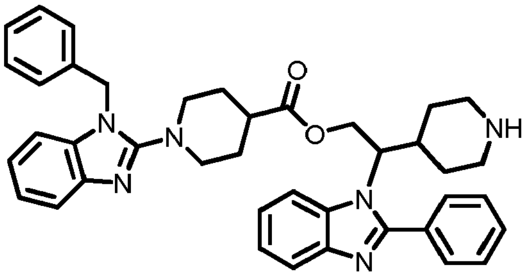

The product was synthesized according to General Procedure (D) starting from the protected piperidine **S40** (15 mg,0.02 mmol) to afford the HCl-salt in quantitative yield. The residue was dissolved in methanol (500 μL) and applied to a preparative C18-RP column eluting with a gradient ($CH_3CN$: $H_2O$ 1:5 to 9:1 containing 0.1 % TFA), to afford piperidine **11** (9 mg, 0.01 mmol, 71 %) as a TFA-salt.

$^1H$ NMR (600 MHz, DMSO-$d_6$) δ 9.08 (s, 1H), 8.78 (s, 1H), 8.07 (s, 1H), 7.81 - 7.72 (m, 3H), 7.72 - 7.59 (m, 3H), 7.56 (d, *J* = 7.8 Hz, 1H), 7.43 (s, 2H), 7.39 - 7.30 (m, 5H), 7.28 (t, *J* = 7.7 Hz, 1H), 7.24 (d, *J* = 7.5 Hz, 2H), 5.39 (s, 2H), 4.83 (t, *J* = 10.4 Hz, 1H), 4.57 (d, *J* = 11.3 Hz, 1H), 4.54 — 4.43 (m, 1H) ,3.65 (d, *J* = 12.1 Hz, 1H), 3.57 (d, *J* = 11.9 Hz, 1H), 3.33 - 3.17 (m, 3H), 3.04 (d, *J* = 11.3 Hz, 1H), 2.85 - 2.72 (m, 2H), 2.72 - 2.54 (m, 2H), 1.97 (d, *J* = 12.3 Hz, 1H), 1.69 - 1.58 (m, 2H), 1.58 - 1.44 (m, 2H), 1.40 (d, *J* = 10.8 Hz, 1H), 1.28 - 1.07 (m,2H). LC-MS (ESI): calcd for $C_{40}H_{42}N_6O_2$: 639.34420 $[M+H]^+$, found: 639.36 $[M+H]^+$, $R_t$ = 5.41 min; HR-MS found 639.34491 $[M+H]^+$.

**Example 69: Preparation of 2-(2-Phenyl-1H-benzo[d]imidazol-1-yl)-2-(piperidin-4-yl)ethyl 1-(1-(thiophen-3-ylmethyl)-1H-benzo[d]imidazol-2-yl)piperidine-4-carboxylate (12)**

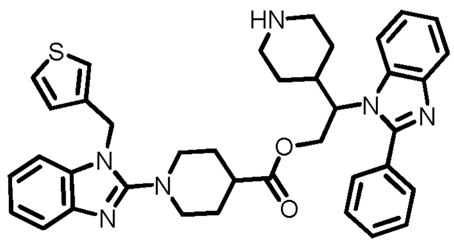

The product was synthesized according to General Procedure (D) starting from the protected piperidine (61 mg, 0.08 mmol) to afford the HCl-salt in quantitative yield. The residue was dissolved in methanol (500 μL) and applied to a preparative C18-RP column eluting with a gradient ($CH_3CN$: $H_2O$ 1:5 to 9:1 containing 0.1 % acetic acid), to afford free piperidine **12** (33 mg, 0.005 mmol, 59%).

$^1H$ NMR (600 MHz, DMSO-$d_6$) δ 7.84 (d, *J* = 6.0 Hz, 1H), 7.69 - 7.62 (m, 3H), 7.58 - 7.50 (m, 3H), 7.51 - 7.46 (m, 1H), 7.39 (t, *J* = 8.0 Hz, 1H), 7.26 - 7.17 (m, 4H), 7.08 - 6.99 (m, 2H), 6.92 - 6.87 (m, 1H), 5.15 (s, 2H), 4.89 - 4.77 (m, 1H), 4.54 (dd, *J* = 12.0, 3.5 Hz, 1H), 4.48 - 4.34 (m, 1H), 3.32 (d, *J* = 12.6 Hz, 1H), 3.24 (d, *J* = 12.5 Hz, 1H), 3.01 - 2.78 (m, 4H), 2.72 (d, *J* = 11.8 Hz, 1H), 2.45 - 2.36 (m, 1H), 2.26 — 2.18 (m, 1H), 1.80 — 1.76 (m, 1H), 1.64 — 1.38 (m, 4H), 1.14 — 1.05 (m, 1H), 0.86 - 0.70 (m, 2H). $^{13}C$ NMR (151 MHz, DMSO-$d_6$) δ 174.30, 158.13, 155.48, 143.70, 141.96, 138.43, 135.64, 133.83, 131.19, 130.50, 130.37, 129.35, 127.87, 127.28, 123.22, 122.93, 122.78, 121.88, 121.47, 120.28, 117.96, 113.16, 110.36, 109.99, 63.22, 61.45, 50.12, 45.73, 45.62, 43.61, 36.18, 29.43, 27.81, 27.68. LC-MS (ESI): calcd for $C_{38}H_{40}N_6O_2S$: 645.30062 $[M+H]^+$, found: 645.27 $[M+H]^+$, $R_t$ = 5.31 min; HR-MS found 645.30123 $[M+H]^+$.

**Example 70: Preparation of Tamra-(S)-2-(2-(3-(3,12-dioxo-5,8-dioxa-2,11-diazatridecyl) phenyl)-1H-enzo[d]imidazol-1-yl)-2-(piperidin-4-yl)ethyl 1-(1-(thiophen-3-ylmethyl)-1H-benzo[d]imidazol-2-yl)piperidine-4-carboxylate = Tamra-(S)-12**

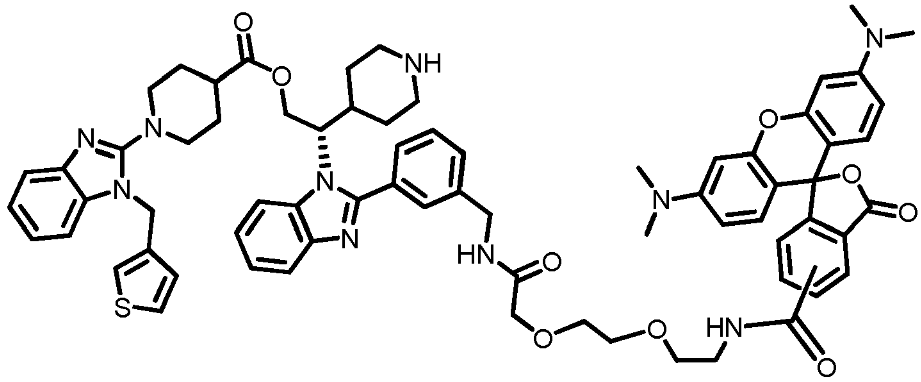

Raney Nickel (Aldrich, W.R. Grace and Co. Raney®2800, slurry, in $H_2O$, 40 mg) was added to an argon filled flask containing ammonia in ethanol (2.0 M, 1 mL). After 5 minutes of stirring the liquid was removed via syringe. The flask was evacuated and flushed with argon. Ammonia in ethanol (2.0 M, 4 mL) was then added, followed by compound **S53** (15 mg, 19 μmol). The solution was purged with argon for 2 minutes and kept under an atmosphere of hydrogen (1 atm, balloon). The resulting reaction mixture was stirred at room temperature for 3 h to afford the free amine (LC-MS: 6.36 min retention time, $[M+H]^+$ 774.36). The reaction mixture was filtered over a pad of Celite, the filter cake was washed with EtOH (30 mL) and ethylacetate (20 ml) and the filtrate was concentrated under reduced pressure. To the crude amine in DMF (1 mL) was added PyBOP (12 mg, 23 μmol) and Tamra-PEG-COOH (13 mg, 23 μmol). The reaction mixture was stirred at room temperature overnight. The reaction mixture was concentrated under reduced pressure. To the residue was added HCl in dioxane (4 M) and the mixture was stirred for 2 h at room temperature. The reaction mixture was concentrated under reduced pressure, the residue was dissolved in methanol and purified by reverse phase chromatography to yield Tamra-(*S*)-**12** (7 mg, 6 μmol, 29 %).
$^1$H NMR (400 MHz, DMSO-$d_6$) δ 8.95 (t, *J* = 5.5 Hz, 1H), 8.63 (s, 1H), 8.54 — 8.46 (m, 1H), 8.46 — 8.38 (m, 1H), 8.30 — 8.23 (m, *J* = 8.1 Hz, 1H), 8.11 — 7.98 (m, 1H), 7.84 (d, *J* = 7.6 Hz, 1H),7.67 - 7.60 (m, 2H), 7.58 — 7.39 (m, 6H), 7.35 - 7.22 (m, 4H), 7.22 - 7.11 (m, 2H), 7.06 - 6.87 (m, 6H), 5.23 (s, 2H), 4.89 (s, 1H), 4.56-4.38 (m, *J* = 45.4, 9.5 Hz, 3H), 3.97 (s, 2H), 3.08 — 2.90 (m, 3H), 2.88 — 2.76 (m, 1H), 2.75 - 2.61 (m, 1H) 2.08 — 1.91 (m, 2H), 1.71 - 1.27 (m, 7H), 1.27 - 1.13 (m, 2H), 1.15 — 0.91 (m, 3H). LC-MS (ESI): found: 1231.58 $[M+H]^+$, $R_t$ = 5.76 min; HR-MS calcd for $C_{70}H_{74}N_{10}O_9S$: 411.18597 $[M+3H]^{3+}$, found 411.18576 $[M+3H]^{3+}$.

**Example 71**

**Preparation of compound (13)**

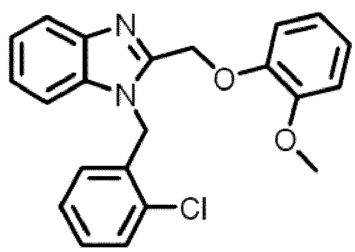

Example 72

Preparation of compound (14)

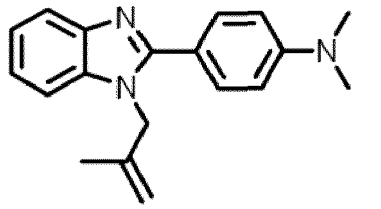

Example 73

Preparation of compound (15)

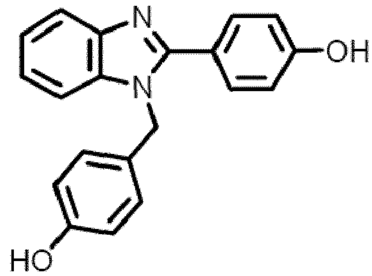

Example 74

Preparation of compound (16)

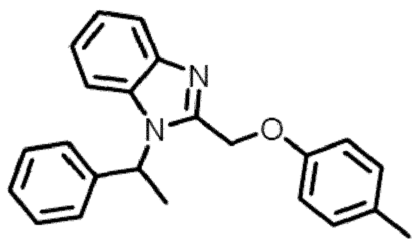

Example 75

Preparation of compound (17)

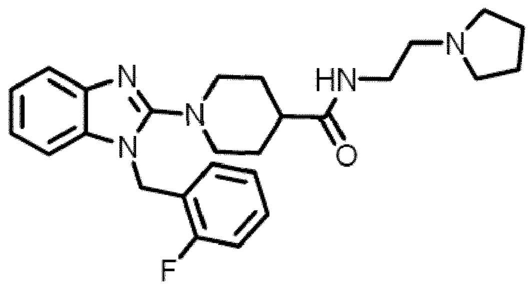

Example 76

Preparation of compound (18)

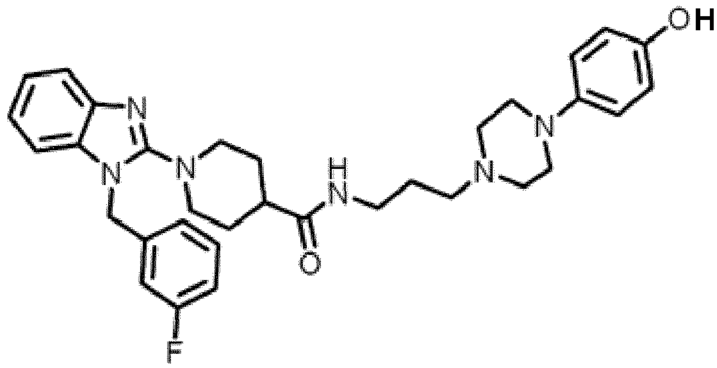

Example 77

Preparation of compound (19)

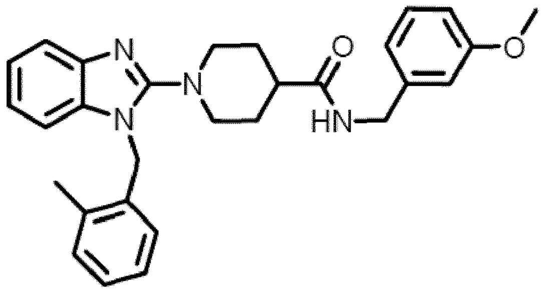

Example 78

Preparation of compound (20)

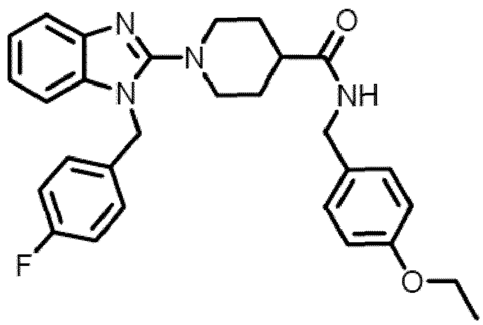

Example 79

Preparation of compound (21)

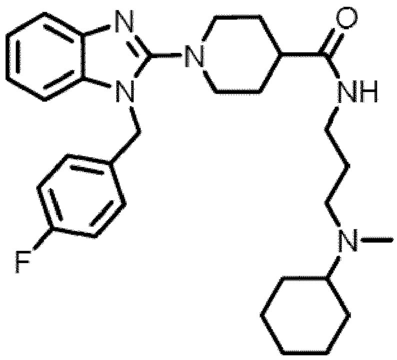

Example 80

Preparation of compound (22)

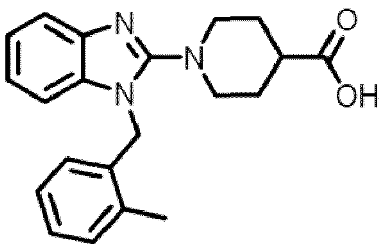

Example 81

Preparation of compound (23)

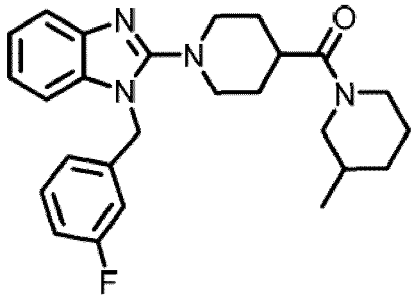

Example 82

Preparation of compound (24)

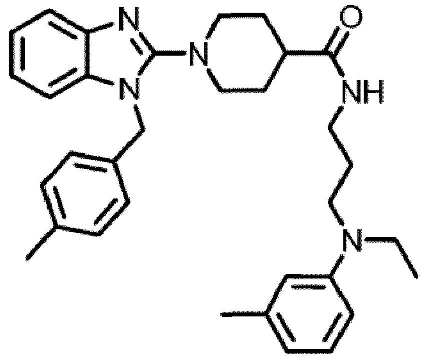

Example 83

Preparation of compound (25)

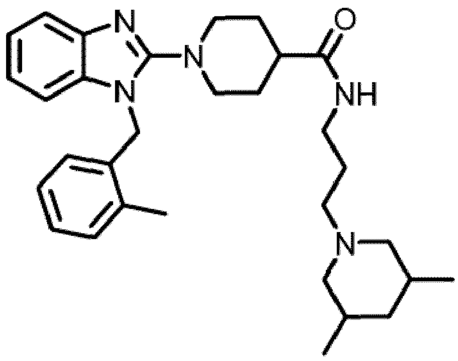

Example 84

Preparation of compound (26)

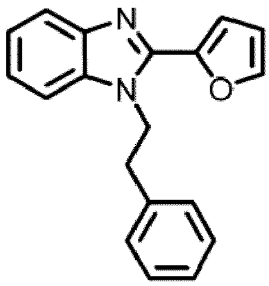

Example 85

Preparation of compound (27)

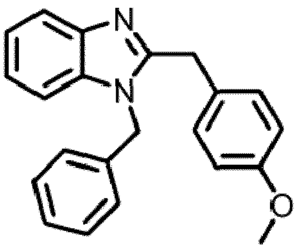

Example 86

Preparation of compound (28)

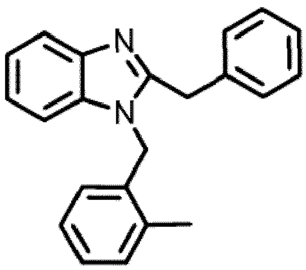

Example 87

Preparation of compound (29)

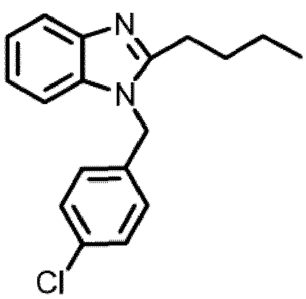

**Example 88**

**Preparation of compound (30)**

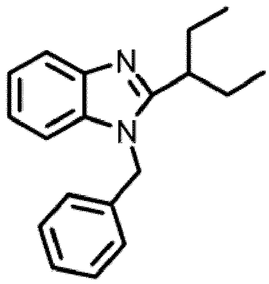

Example 89

Preparation of compound (31)

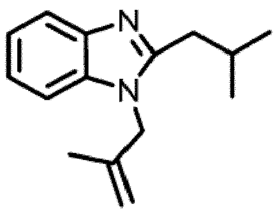

Example 90

Preparation of compound (32)

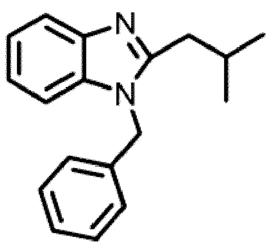

Example 91

Preparation of compound (33)

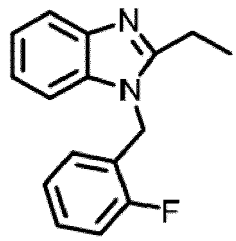

Example 92

Preparation of compound (34)

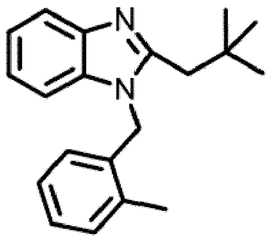

Example 93

Preparation of compound (35)

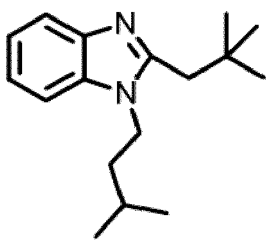

Example 94

Preparation of compound (36)

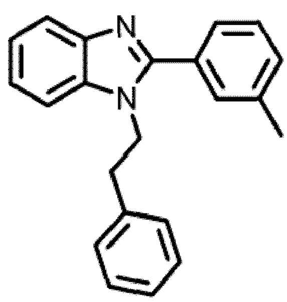

Example 95

Preparation of compound (37)

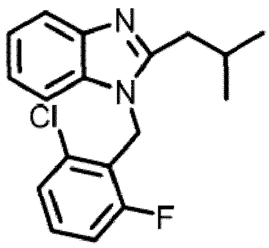

Example 96

Preparation of compound (38)

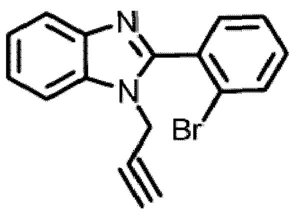

Example 97

Preparation of compound (39)

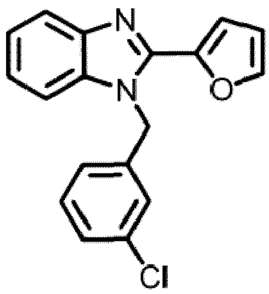

Example 98

Preparation of compound (40)

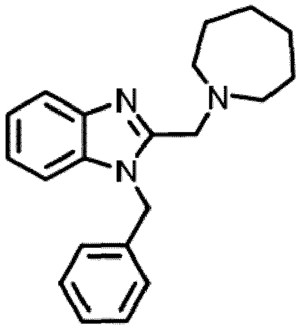

Example 99

Preparation of compound (41)

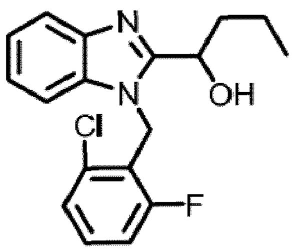

Example 100

Preparation of compound (42)

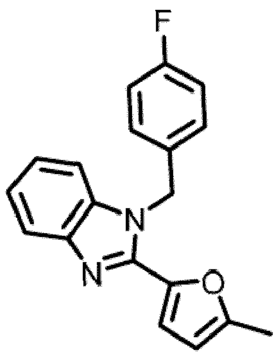

Example 101

Preparation of compound (43)

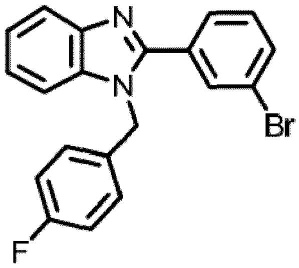

Example 102

Preparation of compound (44)

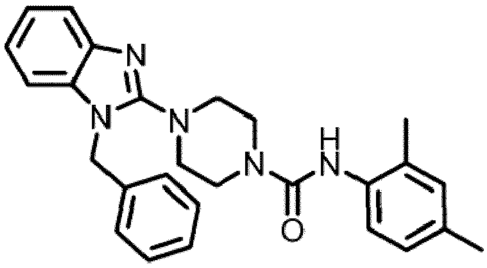

Example 103

Preparation of compound (45)

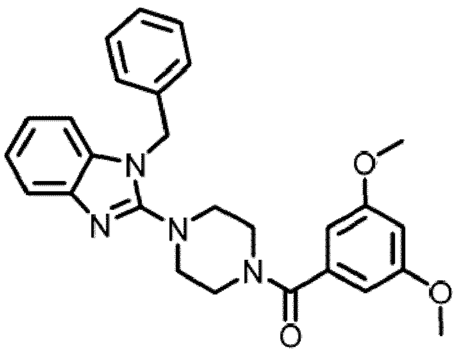

Example 104

Preparation of compound (46)

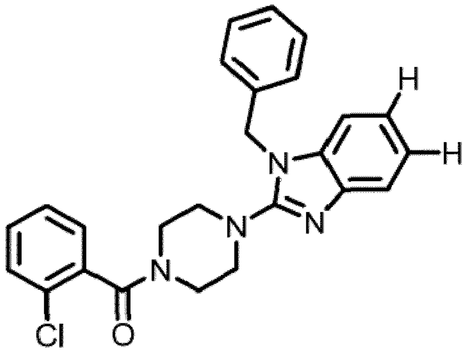

Example 105

Preparation of compound (47)

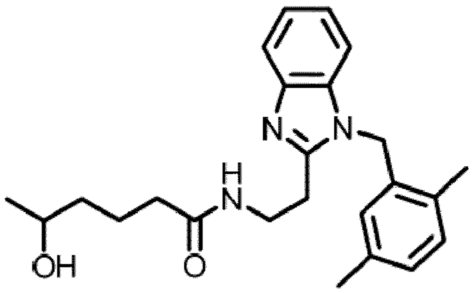

Example 106

Preparation of compound (48)

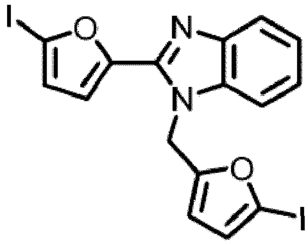

Example 107

Preparation of compound (49)

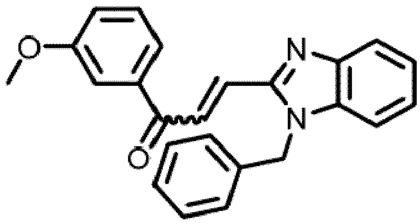

Example 108

Preparation of compound (50)

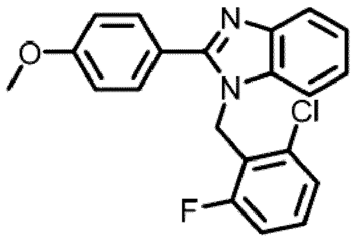

Example 109

Preparation of compound (51)

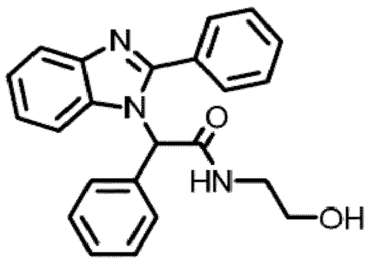

BIOCHEMICAL EXPERIMENTS

Example 110

Alpha-Screen:

Screening based on Alpha-technology was conducted in white, non/binding 1536-well plates (Corning) in a final volume of 6 μL. For the screen a mixture of $His_6$-PDEδ, and biotinylated K-Ras-peptide (final concentrations 100 nM and 250 nM in HEPES 20 mM, 100 mM NaCl, 0,005% Chaps pH 7.5) were added to the 1536-well plates. Compound solutions were directly added from 10 mM DMSO stock solutions to a final concentration of 10 μM and the resulting mixture was incubated for 30 min (For Dose response curves, compounds were tested at concentrations betwen 10 μM and 5 nM). Premixed Nickel Chelate Acceptor Beads and Streptavidin Donor Beads were added to a final concentration of 10 μg/mL. The resulting mixture was incubated at 4°C overnight. Plates were read on a Paradigm reader (Molecular devices, Alphascreen 1536 HTS detection cartridge, temperature 29°C-33°C).

**Example 111**

**Fluorescence polarization measurements for the determination of the binding constant $K_D$ of Tamra-labeled (*S*)-12**

PDEδ was serially diluted in a solution containing 100 nM of Tamra-labeled **(*S*)-12** in 200 μL PBS-buffer (containing 0.05% Chaps, 0.5% DMSO). The 96-well plates were incubated at room temperature for 3 h. The fluorescence polarization values (Ex: 530 nM, Em: 580) were read on a Safire II plate reader (Tecan, temperature ~30°C).
Data was corrected for changes in fluorescence intensity and the fraction bound was fit to a one site binding model derived from the law of mass action using $K_D$ as the only fiiting parameter.[2] Non-linear regression was performed in OriginPro 8.6.

$$FSB = \frac{A - A_{free}}{A - A_{free} + Q(A_{bound} - A)}$$

FSB=fraction bound; $A_{free}$ : Anisotropy of free fluorophore $A_{bound}$ :anisotropy of bound fluorophore; Q : change in fluorescence intensity between free and bound state; A: observed Anisotropy

$$FSB = \frac{K_D + L_{Tot} + P - \sqrt{(K_D + L_{Tot} + P)^2 - 4L_{Tot}P}}{2L_{Tot}}$$

$L_{Tot}$: total concentration of fluorophore, P: protein concentration
Data for Atorvastatin-PEG-Fluorescein were fit analogously.

**Example 112**

**Displacement titrations of labeled probe for the determination of $K_D$ values:**

To fluorescein-labeled Atorvastatin **S54** (24 nM) and $His_6$-tagged PDEδ (40 nM) in 200 μL PBS-buffer (containing 0.05% Chaps, 1% DMSO) in a black, non-binding 96-well plate (Greiner) was added the small molecule from a DMSO stock solution. The resulting solution was serially diluted, holding the concentrations of **S54,** PDEδ and DMSO constant. The sealed plates were shaken overnight at room temperature and centrifuged at the beginning of the next day. The fluorescence polarization values (Ex: 470 nm, Em: 525 nm) were read on a Safire II plate reader (Tecan, temperature 28-32°C).
For the fitting of the data to a competition model the exact mathematical solution derived from the law of mass action was used as described by Roehrl *et al.*[3] Fraction bound data was fit using KD2 as the only fitting parameter and total ligand concentration as independent variable (OriginPro8.6, function codec from origin).

FSB=(2*(sqrt((KD1+KD2+LST+LT-RT)^2-3*((LT-RT)*KD1+(LST-RT)*KD2+KD1*KD2)))*cos((acos((-2*(KD1+KD2+LST+LT-RT)^3+9*(KD1+KD2+LST+LT-RT)*((LT-RT)*KD1+(LST-RT)*KD2+KD1*KD2)-27*(-KD1*KD2*RT))/(2*sqrt(((KD1+KD2+LST+LT-RT)^2-3*((LT-RT)*KD1+(LST-RT)*KD2+KD1*KD2))^3))))/3)-(KD1+KD2+LST+LT-RT))/(3*KD1+2*sqrt((KD1+KD2+LST+LT-RT)^2-3*((LT-RT)*KD1+(LST-RT)*KD2+KD1*KD2))*cos((acos((-2*(KD1+KD2+LST+LT-RT)^3+9*(KD1+KD2+LST+LT-RT)*((LT-RT)*KD1+(LST-RT)*KD2+KD1*KD2)-27*(-KD1*KD2*RT))/(2*sqrt(((KD1+KD2+LST+LT-RT)^2-3*((LT-RT)*KD1+(LST-RT)*KD2+KD1*KD2))^3))))/3)-(KD1+KD2+LST+LT-RT)).

FSB: Fraction bound of fluorescent probe
KD1: Binding constant of fluorescent Probe determined from direct titration
KD2: Fitting parameter (Binding constant of unlabeld competitor)
LST: Total concentration of unlabeld small molecule competitor
RT: Total protein concentration

For the fitting of data to a competition model with cooperative binding of two benzimidazole fragments the program Scientist 3.0 was used. An experimental system was defined as a series of partial equations including the equilibrium relationships between the various species. Anisotropy data was converted to fraction bound and fitting was carried out in anlogy to a previously described set of equilibrium relationships by Goody and coworkers.[4].

$$K_{D1} = \frac{[A][PDED_f]}{[APDED]}$$

$$K_{D2} = \frac{[L^2][PDED_f]}{[LPDED]}$$

A:equilibrium conentration of free fluorophore
L: equilibrium concentration of free ligand
APDED: equilibrium concentration of bound fluorescent probe
LPDED: equilibrium conentration of bound small molecule
PDEDf: equilibrium conentration of free protein

This model was used (together with equations describing the mass balance) to numerically simulate the FSB of fluorophore and compared with the experimental data.
Scientist input file:

```
// Micromath Scientist Model File
IndVars: LST
DepVars: A,L,APDED,LPDED,PDEDf,FSB
Params: ATOT,PDED,KD1,KD2
LPDED= L^2*PDEDf/KD2
APDED=A*PDEDf/KD1
ATOT=A+APDED
LST=L+LPDED
PDED=PDEDf+APDED+LPDED
0<A<ATOT
0<L<LST
0<PDEDf<PDED
FSB=1-A/ATOT
LST=0
```

**Example 113: Cell culture.**

All cells were routinely maintained in MEM Eagle's medium supplemented with 10% FBS, 1% non-essential amino acids and 1% L-glutamine. For live-cell microscopy, cells were cultured 4-well Lab-Tek chambers (NUNC) and transferred to low-bicarbonate DMEM without phenol red supplemented with 25 mM HEPES at pH 7.4 (imaging medium). Transfections were carried out with Effectene (Qiagen).

**Example 114: Western blots.**

hPDAC cells were seeded at a density of 3 × $10^5$ cells/ml in 6-well plates. Cells were washed with ice-cold PBS and 0.1 ml ice-cold lysis buffer containing protease inhibitors (Roche) and phosphatase inhibitor I and II (Sigma) was added to each dish. Cells were scraped off after 5 min on ice and centrifuged at 14,000g for 20 min at 4° C. SDS-PAGE was carried out with 30 μg of whole-cell lysate from each sample. The gels were blotted onto PVDF membrane and blocked for 1 h at room temperature. Antibodies used for western blotting were: anti-Erk (Abcam-AB36991; 1:2,000), anti-pErk (Cell Signaling-9101; 1:2,000), anti-GAPDH (Calbiochem-CB1001; 1:2000), and infrared secondary antibodies (LI-COR). Blots were scanned on a LI-COR Odyssey imaging system.

**Example 115**

**Time-domain FLIM (Fluorescence lifetime imaging microscopy)**

Fluorescence lifetime images were acquired using a confocal laser scanning microscope (FV1000, Olympus) equipped with a time-correlated single-photon counting module (LSM Upgrade Kit, Picoquant). For detection of mCitrine, the sample was excited using a 470nm diode laser (LDH 470, Picoquant) at 40 MHz repetition rate. Fluorescence signal was collected through an oil immersion objective (60x/1.35 UPlanSApo, Olympus) and spectrally filtered using a narrowband emission filter (HQ 525/15, Chroma). Photons were detected using a single-photon counting avalanche photodiode (PDM Series, MPD) and timed using a single-photon counting module (PicoHarp 300, Picoquant). (for figure 1.)

**Example 116: Real time cell analysis (RTCA)**

Cells were seeded in 200 μl growth medium at a density of 5000 cells per well into a E-Plate 16 well. Cell attachment and growth were monitored using the Roche RTCA DP Analyser. The next day the PDEδ inhibitor (*S*)-**9** was administrated at a concentration of 5 μM and the proliferation was monitored for at least three days.

The activity of the compounds was classified according to their Kd for the PDEδ farnesyl pocket into the following ranges:

| | |
|---|---|
| 1 nM < $K_D$ ≤ 25 nM | ++++ |
| 25 nM < $K_D$ ≤ 100 nM | +++ |
| 100 nM < $K_D$ ≤ 1000 nM | ++ |
| $K_D$ > 1000 nM | + |

Table 3. Evaluation of the $K_D$ values of the compounds of the present invention

| compound | $K_D$ [nM] | compound | $K_D$ [nM] |
|---|---|---|---|
| 01 | ++ | S01 | |
| 02 | + | S02 | + |
| 03 | +++ | S03 | + |
| 04 | ++++ | S04 | + |
| 05 | ++ | S05 | n.d. |
| 06 | ++ | S06 | ++ |
| 07 | ++ | S07 | +++ |
| 08 | + | S08 | + |
| (*S*)-9 | +++ | S09 | ++ |
| (*R*)-9 | ++ | S10 | n.d. |
| 10 | ++ | S11 | n.d. |
| 11 | ++++ | S12 | ++ |
| 12 | ++++ | S13 | n.d. |
| (*S*)-12 | ++++ | S14 | |
| (*R*)-12 | +++ | S15 | |
| 13 | ++ | S16 | + |
| 14 | ++ | S17 | |

(continued)

| compound | $K_D$ [nM] | compound | $K_D$ [nM] |
|---|---|---|---|
| 15 | +++ | S18 | n.d. |
| 16 | ++ | S19 | n.d. |
| 17 | ++ | S20 | n.d. |
| 18 | ++ | S21 | n.d. |
| 19 | ++ | S22 | |
| 20 | +++ | S23 | |
| 21 | ++ | S24 | |
| 22 | + | S25 | |
| 23 | + | S26 | n.d. |
| 24 | + | S27 | ++ |
| 25 | ++ | S28 | ++ |
| 26 | ++ | S29 | ++ |
| 27 | ++ | S30 | |
| 28 | + | S31 | n.d. |
| 29 | ++ | S32 | n.d. |
| 30 | + | S33 | n.d. |
| 31 | ++ | S34 | n.d. |
| 32 | ++ | S35 | n.d. |
| 33 | ++ | S36 | n.d. |
| 34 | +++ | S37 | ++++ |
| 35 | ++ | S38 | ++ |
| 36 | + | S39 | ++++ |
| 37 | +++ | S40 | |
| 38 | + | S41 | |
| 39 | ++ | S42 | |
| 40 | ++ | S43 | +++ |
| 41 | ++ | S44 | |
| 42 | ++ | S45 | |
| 43 | + | S46 | |
| 44 | ++ | S47 | |
| 45 | ++ | S48 | n.d. |
| 46 | ++ | S49 | |
| 47 | + | S50 | |
| 48 | +++ | S51 | |
| 49 | + | S52 | |
| 50 | ++ | S53 | n.d. |

(continued)

| compound | $K_D$ [nM] | compound | $K_D$ [nM] |
|---|---|---|---|
| 51 | + | | |
| n.d. = not determined. | | | |

## Claims

**1.** Compound of the general formula (I)

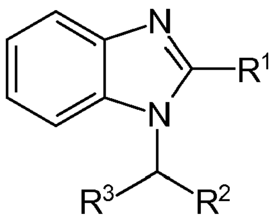 **(I)**

wherein

**$R^1$** represents $—R^4$, $—CH_2R^4$, $—CH_2—OR^4$, $—CH(OH)—R^4$, $—CH{=}CH—CO—R^4$, or $—C_2H_4—NH—CO—R^4$;
**$R^2$** represents $—C(CH_3){=}CH_2$, $—CH_2—CH(CH_3)_2$, $—C{\equiv}CH$, $—CO—NH—R^{11}$,

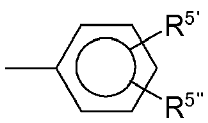 , 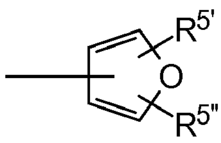 , 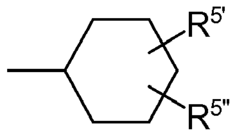 , 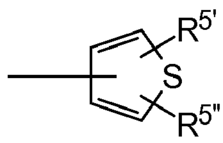 ,

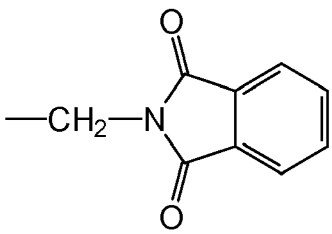 , 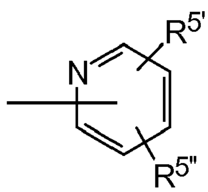 , 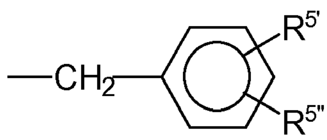 ,

$— CO—N(R^{11})—COOC(CH_3)_3$, $—CH_2OH$, $—CH_2NH_2$, $—CH_2—CH{=}CH_2$, $—CH{=}CH_2$, or $—COOCH_3$;
**$R^3$** represents -H, $—CH_3$,

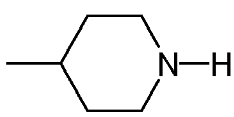 , 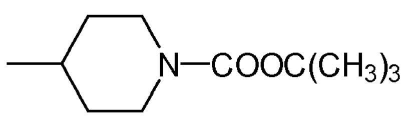 ,

$— C_2H_5$, $—CH_2OH$, $—COOCH_3$, $—cyclo\text{-}C_6H_{11}$, or —Ph,
or **$R^3$** together with **$R^5$** is the residue —NH—;
**$R^4$** represents $—CH_3$, $—C_2H_5$, $—C_3H_7$, $—CH(CH_3)_2$, $—C_4H_9$, $—CH_2—CH(CH_3)_2$, $—CH(CH_3)—C_2H_5$, $—C(CH_3)_3$, $—C_5H_{11}$, $—CH(CH_3)—C_3H_7$, $—CH_2—CH(CH_3)—C_2H_5$, $—CH(CH_3)—CH(CH_3)_2$, $—C(CH_3)_2—C_2H_5$, $—CH_2—C(CH_3)_3$, $—CH(C_2H_5)_2$, $—C_2H_4—CH(CH_3)_2$, $—C_6H_{13}$, $—C_3H_6—CH(CH_3)_2$, $—C_2H_4—CH(CH_3)—C_2H_5$, $—CH(CH_3)—C_4H_9$, $—CH_2—CH(CH_3)—C_3H_7$, $—CH(CH_3)—CH_2—CH(CH_3)_2$, $—CH(CH_3)—CH(CH_3)$

$—C_2H_5$, $—CH_2—CH(CH_3)—CH(CH_3)_2$, $—CH_2—C(CH_3)_2—C_2H_5$,$—C(CH_3)_2—C_3H_7$, $—C(CH_3)_2—CH(CH_3)_2$, $—C_2H_4—C(CH_3)_3$, $—CH_2—CH(C_2H_5)_2$, $—CH(CH_3)—C(CH_3)_3$, $—C(C_2H_5)_3$, $—CH_2—C(C_2H_5)_3$, $—C_3H_6—CH(CH_3)R^5$,

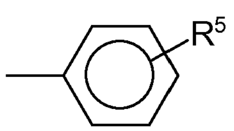,
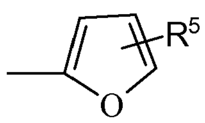,
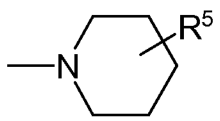,
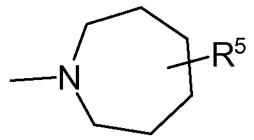,

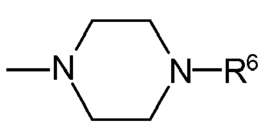,
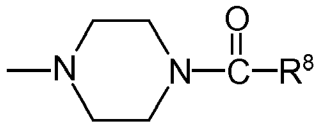,
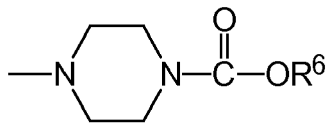,

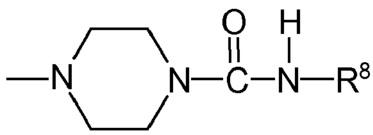

**$R^5$, $R^{5'}$, $R^{5''}$** represent independently of each other -H, $—OCH_3$, $—OC_2H_5$, $—CO—NH—R^6$, $—OR^6$, $—N(CH_3)_2$, $—N(C_2H_5)_2$, $—CO—R^7$, $—CH_2—CH{=}CH_2$, -OH, $—NH_2$, —F, —Cl, -Br, —I, $—CH_3$, $—C_2H_5$, -COOH, or $—COO—R^6$,
**$R^6$** represents $—CH_2—R^7$, $—C_2H_4—R^7$, $—C_3H_6—R^7$,

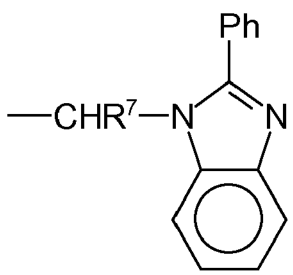

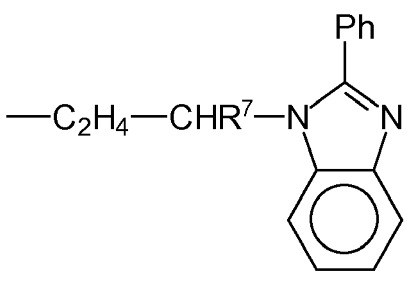,
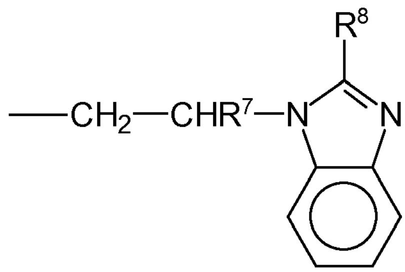

or

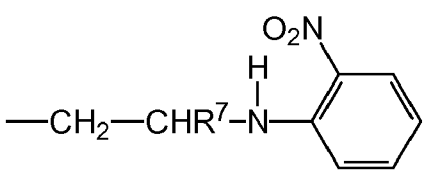;

**R7** represents -H, $—CH_3$, $—CH=CH_2$, $—CH_2COOH$, $—CH_2—CH=CH_2$, $—NHR^8$, $—N(CH_3)R^8$, $—N(C_2H_5)R^8$, $—CH_2CONHC_3H_6(OC_2H_4)_3CH_2NHR^8$,

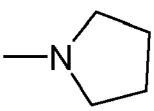 , 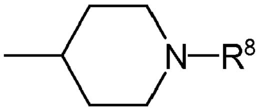 , 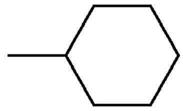 ,

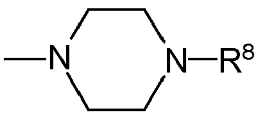 , 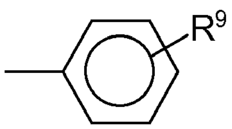 , 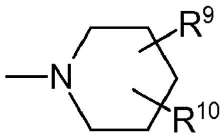 ;

**R8** represents -H, $—CO_2C(CH_3)_3$,

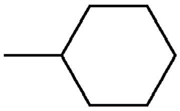 , 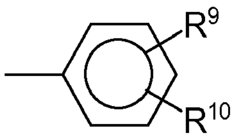 ,

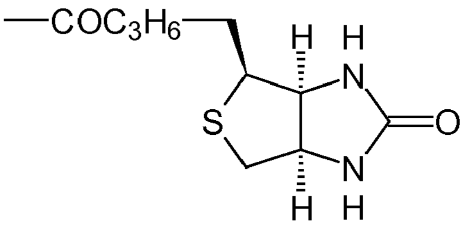 ;

**R9** and **R10** represent independently of each other -H, -F, -Br, —Cl, —I, -CN, -OH, $—OCH_3$, $—OC_2H_5$, $—CH_3$, or $—C_2H_5$;
**R11** represents $—CH_2—R^{12}$, or $—C_2H_4—R^{12}$;
**R12** represents -Ph, or -OH;

and enantiomers, stereoisomeric forms, mixtures of enantiomers, diastereomers, mixtures of diastereomers, hydrates, solvates, acid salt forms, tautomers, and racemates of the above mentioned compounds and pharmaceutically acceptable salts thereof.

**2.** Compound according to claim 1, wherein

**R1** represents $—R^4$, $—CH_2R^4$, $—CH_2—OR^4$, $—CH(OH)—R^4$, $—CH=CH—CO—R^4$ or $—C_2H_4—NH—CO—R^4$;
**R2** represents $—C(CH_3)=CH_2$, $—CH_2—CH(CH_3)_2$, $—C{\equiv}CH$, $—CO—NH—R^{11}$,

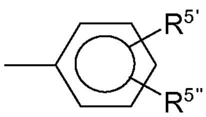 , 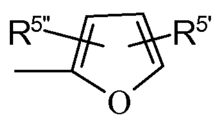 , 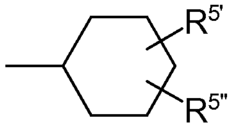 , 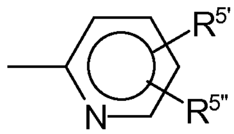 ,

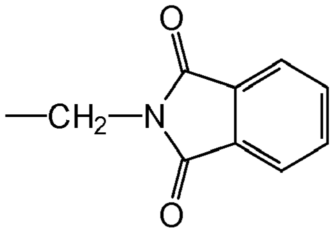, 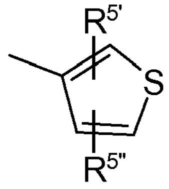, 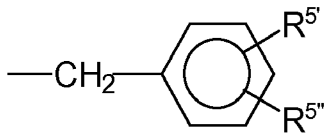,

— CO—N($R^{11}$)—COOC($CH_3$)$_3$, —$CH_2OH$, —$CH_2NH_2$, —$CH_2$—CH=$CH_2$, or — $COOCH_3$;
**$R^3$** represents -H, —$CH_3$,

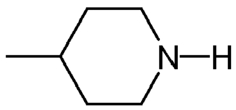, 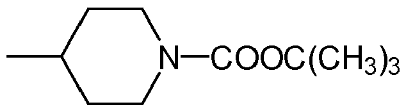,

— $CH_2OH$, —$COOCH_3$, —cyclo-$C_6H_{11}$, or —Ph,
or **$R^3$** together with **$R^5$** is the residue —NH—;
**$R^4$** represents —$CH_3$, —$C_2H_5$, —$C_3H_7$, —CH($CH_3$)$_2$, —$C_4H_9$, —$CH_2$—CH($CH_3$)$_2$, —CH($CH_3$)—$C_2H_5$, —C($CH_3$)$_3$, —$C_5H_{11}$, —CH($CH_3$)—$C_3H_7$, —$CH_2$—CH($CH_3$)—$C_2H_5$, —CH($CH_3$)—CH($CH_3$)$_2$, —C($CH_3$)$_2$—$C_2H_5$, —$CH_2$—C($CH_3$)$_3$, —CH($C_2H_5$)$_2$, —$C_2H_4$—CH($CH_3$)$_2$, —$C_6H_{13}$, —$C_3H_6$—CH($CH_3$)$_2$, —$C_2H_4$—CH($CH_3$)—$C_2H_5$, —CH($CH_3$)—$C_4H_9$, —$CH_2$—CH($CH_3$)—$C_3H_7$, —CH($CH_3$)—$CH_2$—CH($CH_3$)$_2$, —CH($CH_3$)—CH($CH_3$)—$C_2H_5$, —$CH_2$—CH($CH_3$)—CH($CH_3$)$_2$, —$CH_2$—C($CH_3$)$_2$—$C_2H_5$, —C($CH_3$)$_2$—$C_3H_7$, —C($CH_3$)$_2$—CH($CH_3$)$_2$, —$C_2H_4$—C($CH_3$)$_3$, —$CH_2$—CH($C_2H_5$)$_2$, —CH($CH_3$)—C($CH_3$)$_3$, —C($C_2H_5$)$_3$, —$CH_2$—C($C_2H_5$)$_3$, —$C_3H_6$—CH($CH_3$)$R^5$,

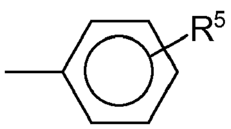, 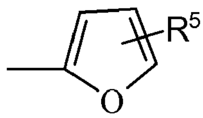, 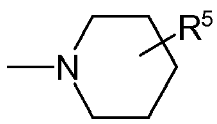, 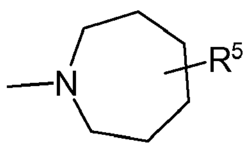,

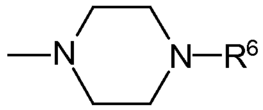, 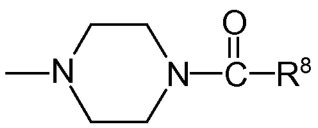, 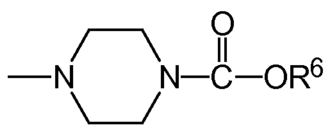,

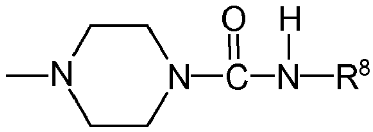

**$R^5$, $R^{5'}$, $R^{5''}$** represent independently of each other -H, —$OCH_3$, —$OC_2H_5$, —CO—NH—$R^6$, —$OR^6$, —N($CH_3$)$_2$, —N($C_2H_5$)$_2$, —CO—$R^7$, —$CH_2$—CH=$CH_2$, -OH, —$NH_2$, -F, —Cl, -Br, —I, —$CH_3$, —$C_2H_5$, -COOH, or —COO—$R^6$,
**$R^6$** represents —$CH_2$—$R^7$, —$C_2H_4$—$R^7$, —$C_3H_6$—$R^7$,

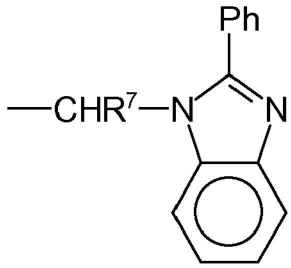

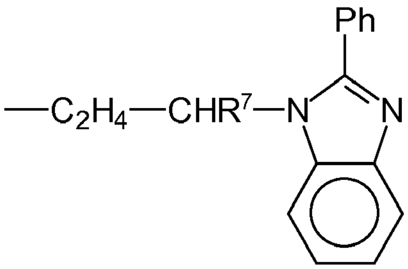

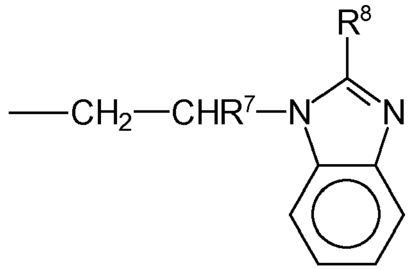

or

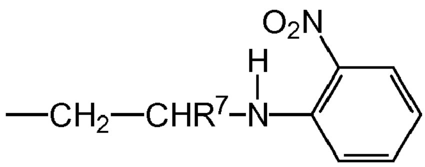

;

**$R^7$** represents -H, $-CH_2COOH$, $-NHR^8$, $-N(CH_3)R^8$, $-N(C_2H_5)R^8$, $-CH_3$, $-CH=CH_2$, $-CH_2-CH=CH_2$, $-CH_2CONHC_3H_6(OC_2H_4)_3CH_2NHR^8$,

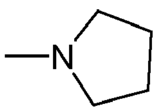

,

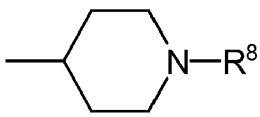

,

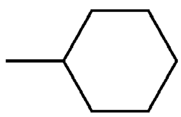

,

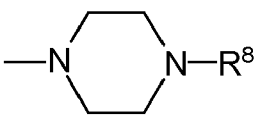

,

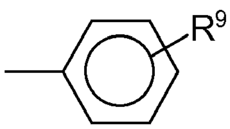

,

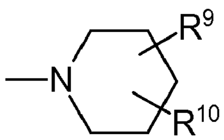

;

**$R^8$** represents -H, $-CO_2C(CH_3)_3$,

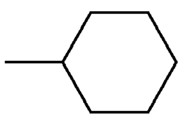

,

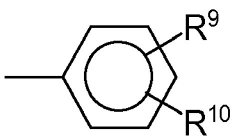

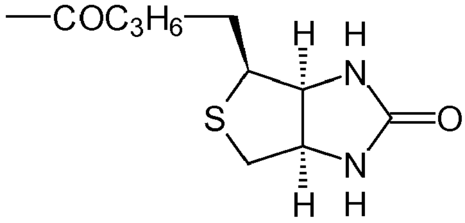

;

$R^9$ and $R^{10}$ represent independently of each other -H, -F, -Br, —Cl, —I, -CN, -OH, $—OCH_3$, $—OC_2H_5$, $—CH_3$, or $—C_2H_5$;
$R^{11}$ represents $—CH_2—R^{12}$ or $—C_2H_4—R^{12}$;
$R^{12}$ represents -Ph or -OH;

and enantiomers, stereoisomeric forms, mixtures of enantiomers, diastereomers, mixtures of diastereomers, hydrates, solvates, acid salt forms, tautomers, and racemates of the above mentioned compounds and pharmaceutically acceptable salts thereof.

**3.** Compound according to claim 1, wherein $R^6$ represents

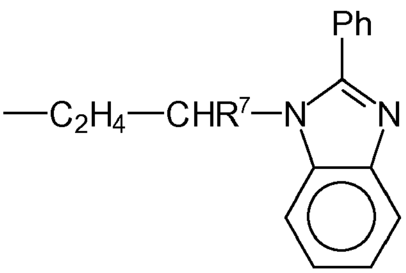

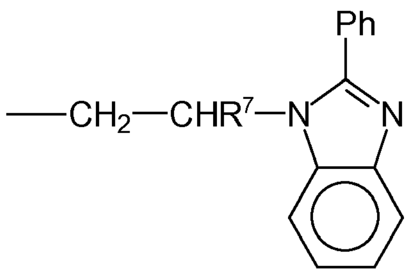

or

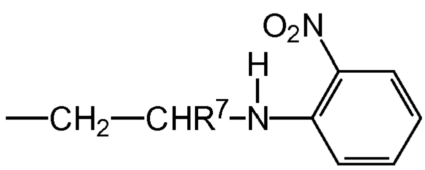

**4.** Compound according to claim 1, wherein $R^2$ represents p-hydroxyphenyl or

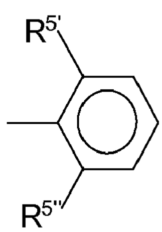

wherein $R^5$, $R^{5'}$, $R^{5''}$ represent independently of each other -F, —Cl, or -Br.

**5.** Compound according to claim 1 selected from the following list:

Compound S02: 2-(4-(Allyloxy)phenyl)-1-benzyl-1H-benzo[d]imidazole
Compound S03: 2-(4-(Allyloxy)phenyl)-1-(pyridin-2-ylmethyl)-1H-benzo[d]imidazole
Compound S04: 2-(4-(Allyloxy)phenyl)-1-(2-methylbenzyl)-1H-benzo[d]imidazole

Compound S05: 2-(4-(Allyloxy)phenyl)-1-(3-methylbenzyl)-1H-benzo[d]imidazole
Compound S06: 2-(4-(Allyloxy)phenyl)-1-(4-fluorobenzyl)-1H-benzo[d]imidazole
Compound S07: 4-(1-Benzyl-1H-benzo[d]imidazol-2-yl)phenol
Compound S08: 4-(1-(Pyridin-2-ylmethyl)-1H-benzo[d]imidazol-2-yl)phenol
Compound S09: 4-(1-(4-Fluorobenzyl)-1H-benzo[d]imidazol-2-yl)phenol
Compound S10: 4-(1-(2-Methylbenzyl)-1H-benzo[d]imidazol-2-yl)phenol
Compound S11: 4-(1-(3-Methylbenzyl)-1H-benzo[d]imidazol-2-yl)phenol
Compound S12: N-Benzyl-2-cyclohexyl-2-(2-phenyl-1H-benzo[d]imidazol-1-yl)acetamide
Compound S13: N-Benzyl-2-(2-phenyl-1H-benzo[d]imidazol-1-yl)-2-(piperidin-4-yl)acetamide
Compound S16: 2-Cyclohexyl-2-(2-phenyl-1H-benzo[d]imidazol-1-yl)ethanol
Compound S18: Methyl 2-(2-phenyl-1H-benzo[d]imidazol-1-yl)acetate
Compound S19: Methyl 2-(2-phenyl-1H-benzo[d]imidazol-1-yl)pent-4-enoate
Compound S20: 2-(2-Phenyl-1H-benzo[d]imidazol-1-yl)pent-4-en-1-ol
Compound S21: 2-(2-Phenyl-1H-benzo[d]imidazol-1-yl)ethanol
Compound S26: 2-Phenyl-1-(1-(piperidin-4-yl)-2-(4-(1-(pyridin-2-ylmethyl)-1H-benzo[d]imidazol-2-yl)phenoxy)ethyl)-1H-benzo[d]imidazole
Compound S27: 1-(3-Methylbenzyl)-2-(4-(2-(2-phenyl-1H-benzo[d]imidazol-1-yl)-2-(piperidin-4-yl)ethoxy)phenyl)-1H-benzo[d]imidazole
Compound S28: 1-(4-Fluorobenzyl)-2-(4-(2-(2-phenyl-1H-benzo[d]imidazol-1-yl)-2-(piperidin-4-yl)ethoxy)phenyl)-1H-benzo[d]imidazole
Compound S29: 1-(2-Methylbenzyl)-2-(4-(2-(2-phenyl-1H-benzo[d]imidazol-1-yl)-2-(piperidin-4-yl)ethoxy)phenyl)-1H-benzo[d]imidazole
Compound S31: Ethyl 1-(1-benzyl-1H-benzo[d]imidazol-2-yl)piperidine-4-carboxylate
Compound S32: Ethyl 1-(1-(2-chloro-6-fluorobenzyl)-1H-benzo[d]imidazol-2-yl)piperidine-4-carboxylate
Compound S33: Ethyl 1-(1-(thiophen-3-ylmethyl)-1H-benzo[d]imidazol-2-yl)piperidine-4-carboxylate
Compound S34: 1-(1-(Thiophen-3-ylmethyl)-1H-benzo[d]imidazol-2-yl)piperidine-4-carboxylic acid
Compound S35: 1-(1-Benzyl-1H-benzo[d]imidazol-2-yl)piperidine-4-carboxylic acid
Compound S36: 1-(1-(2-Chloro-6-fluorobenzyl)-1H-benzo[d]imidazol-2-yl)piperidine-4-carboxylic acid
Compound S37: 2-Cyclohexyl-2-(2-phenyl-1H-benzo[d]imidazol-1-yl)ethyl 1-(1-benzyl-1H-benzo[d]imidazol-2-yl)piperidine-4-carboxylate
Compound S38: 2-Cyclohexyl-2-(2-phenyl-1H-benzo[d]imidazol-1-yl)ethyl 1-(1-(2-chloro-6-fluorobenzyl)-1H-benzo[d]imidazol-2-yl)piperidine-4-carboxylate
Compound S39: 2-Cyclohexyl-2-(2-phenyl-1H-benzo[d]imidazol-1-yl)ethyl 1-(1-(thiophen-3-ylmethyl)-1H-benzo[d]imidazol-2-yl)piperidine-4-carboxylate
Compound S43: 2-(2-Phenyl-1H-benzo[d]imidazol-1-yl)-2-(piperidin-4-yl)ethyl 1-(1-(2-chloro-6-fluorobenzyl)-1H-benzo[d]imidazol-2-yl)piperidine-4-carboxylate
Compound S48: 2-(2-Phenyl-1H-benzo[d]imidazol-1-yl)-2-(piperidin-4-yl)ethyl 4-(1-benzyl-1H-benzo[d]imidazol-2-yl)piperazine-1-carboxylate
Compound S53: (S)-tert-Butyl 4-(1-(2-(3-cyanophenyl)-1H-benzo[d] imidazol-1-yl)-2-(1-(1-(thiophen-3-ylmethyl)-1H-benzo[d]imidazol-2-yl)piperidine-4-carbonyloxy)ethyl) piperidine-1-carboxylate
Compound 01: 1-Benzyl-2-phenyl-1H-benzo[d]imidazole
Compound 02: 6-Pyiridyl-5,6-dihydrobenzo[4-5]imidazo[1,2-c]quinazoline
Compound 03: 1-Benzyl-2-(4-(2-(2-phenyl-1H-benzo[d]imidazol-1-yl)pent-4-enyloxy)phenyl)-1H-benzo[d]imidazole
Compound 04: 1-Benzyl-2-(4-(2-cyclohexyl-2-(2-phenyl-1H-benzo[d]imidazol-1-yl)ethoxy)phenyl)-1H-benzo[d]imidazole
Compound 05: 4-(4-(1-Benzyl-1H-benzo[d]imidazol-2-yl)phenoxy)-3-(2-phenyl-1H-benzo[d]imidazol-1-yl)butanoic acid
Compound 06: 1-Benzyl-2-(4-(2-(2-phenyl-1H-benzo[d]imidazol-1-yl)ethoxy)phenyl)-1H-benzo[d]imidazole
Compound 07: N-(18-(4-(1-Benzyl-1H-benzo[d]imidazol-2-yl)phenoxy)-15-oxo-17-(2-phenyl-1H-benzo[d]imidazol-1-yl)-4,7,10-trioxa-14-azaoctadecyl)-5-((3aS,4S,6aR)-2-oxohexahydro-1H-thieno[3,4-d]imidazol-4-yl)pentanamide
Compound 08: tert-Butyl-4-(2-(4-(1-benzyl-1H-benzo[d]imidazol-2-yl)phenoxy)-1-(2-phenyl-1H-benzo[d]imidazol-1-yl)ethyl)piperidine-1-carboxylate
Compound (*S*)-08: (S)-tert-Butyl 4-(2-(4-(1-benzyl-1H-benzo[d]imidazol-2-yl)phenoxy)-1-(2-phenyl-1H-benzo[d]imidazol-1-yl)ethyl)piperidine-1-carboxylate
Compound 09: 1-Benzyl-2-(4-(2-(2-phenyl-1H-benzo[d]imidazol-1-yl)-2-(piperidin-4-yl)ethoxy)phenyl)-1H-benzo[d]imidazole

Compound (*S*)-09: (*S*)-1-Benzyl-2-(4-(2-(2-phenyl-1H-benzo[d]imidazol-1-yl)-2-(piperidin-4-yl)ethoxy)phenyl)-1H-benzo[d]imidazole
Compound (*R*)-09: (*R*)-1-Benzyl-2-(4-(2-(2-phenyl-1H-benzo[d]imidazol-1-yl)-2-(piperidin-4-yl)ethoxy)phenyl)-1H-benzo[d]imidazole
Compound 10: N-(2-(2-Phenyl-1H-benzo[d]imidazol-1-yl)-2-(piperidin-4-yl)ethyl)-1-(1-(thiophen-3-ylmethyl)-1H-benzo[d]imidazol-2-yl)piperidine-4-carboxamide
Compound 11: 2-(2-Phenyl-1H-benzo[d]imidazol-1-yl)-2-(piperidin-4-yl)ethyl 1-(1-benzyl-1H-benzo[d]imidazol-2-yl)piperidine-4-carboxylate
Compound (*S*)-12: (*S*)-2-(2-Phenyl-1H-benzo[d]imidazol-1-yl)-2-(piperidin-4-yl)ethyl 1-(1-(thiophen-3-ylmethyl)-1H-benzo[d]imidazol-2-yl)piperidine-4-carboxylate
Compound (*R*)-12: (*R*)-2-(2-Phenyl-1H-benzo[d]imidazol-1-yl)-2-(piperidin-4-yl)ethyl 1-(1-(thiophen-3-ylmethyl)-1H-benzo[d]imidazol-2-yl)piperidine-4-carboxylate

**6.** Compound according to any one of claims 1 - 5 for use as pharmaceutically active agent in medicine.

**7.** Compound according to any one of claims 1 - 5 for use in the treatment or prophylaxis of proliferative diseases, tumors or cancer.

**8.** Compound according to claim 7, wherein the proliferative disease, tumor or cancer is selected from the group comprising: adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma and tongue cancer.

**9.** Pharmaceutical composition comprising at least one compound according to claim 1 as an active ingredient, together with at least one pharmaceutically acceptable carrier, excipient and/or diluent.

**10.** Pharmaceutical composition according to claim 9 for use in the treatment or prophylaxis of proliferative diseases, tumors or cancer.

Figure 1

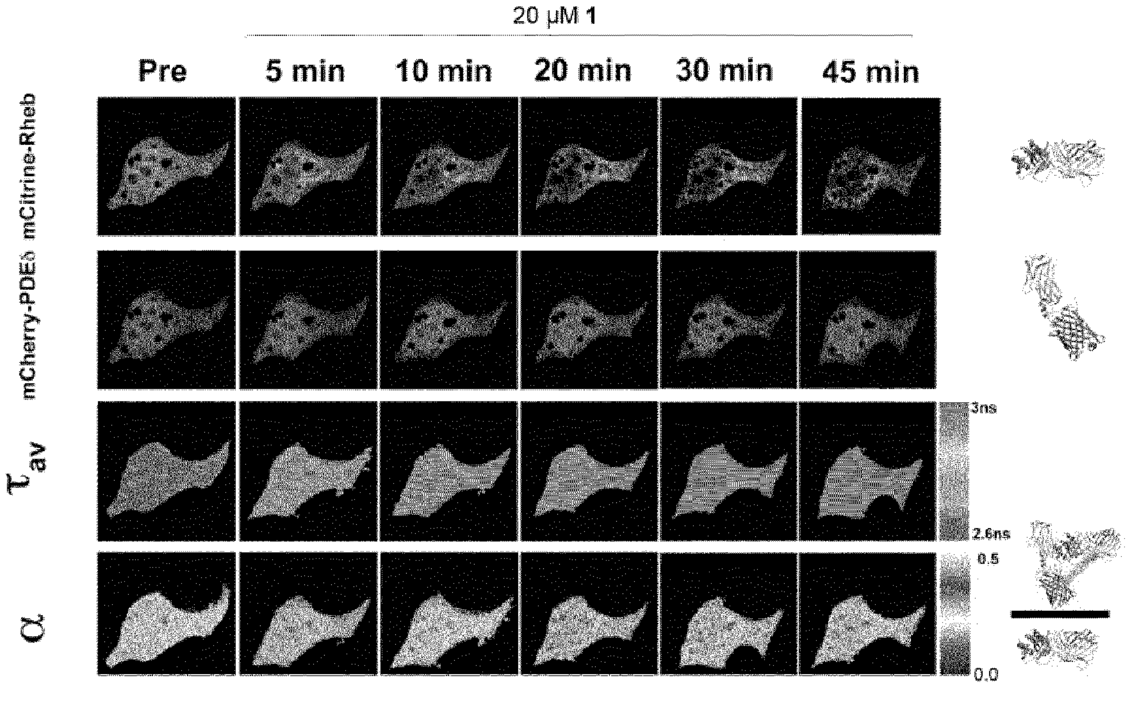
20 µM 1
Pre
5 min
10 min
20 min
30 min
45 min
mCherry-PDEδ mCitrine-Rheb
$\tau_{av}$
α
3ns
2.6ns
0.5
0.0

Figure 2

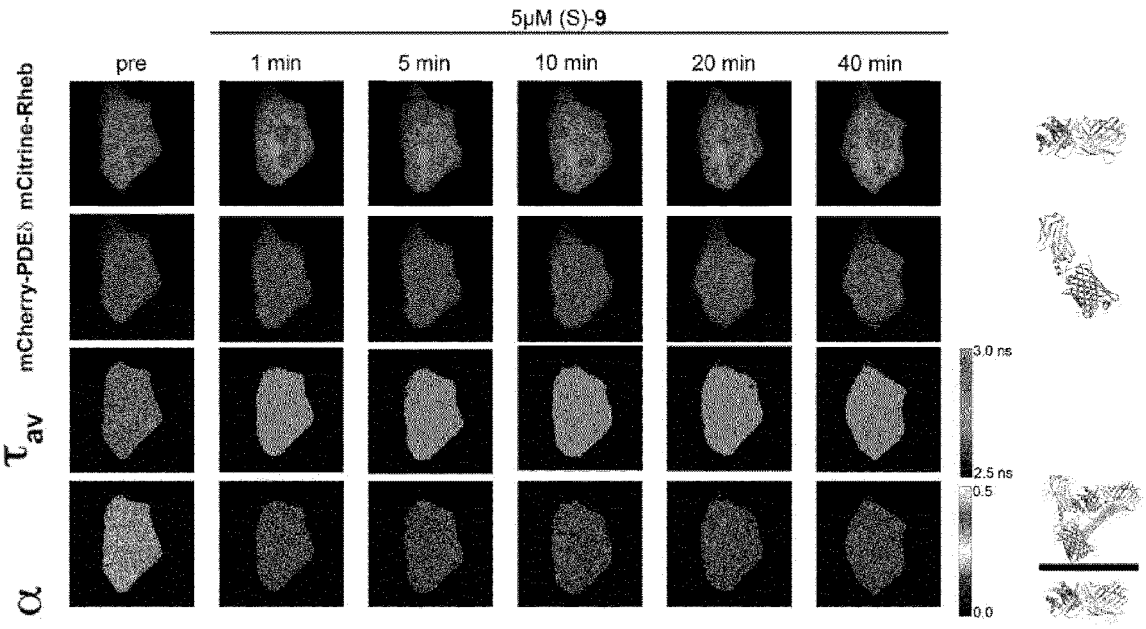
5μM (S)-9
pre
1 min
5 min
10 min
20 min
40 min
mCherry-PDEδ mCitrine-Rheb
τav
α
3.0 ns
2.5 ns
0.5
0.0

Figure 3

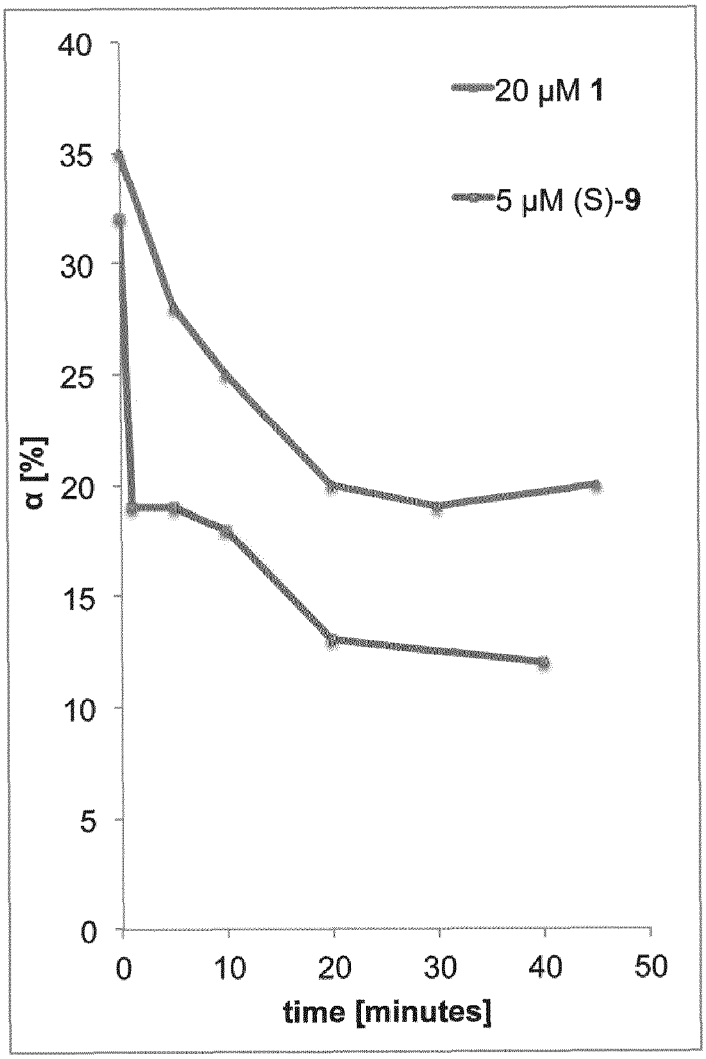

Figure 4

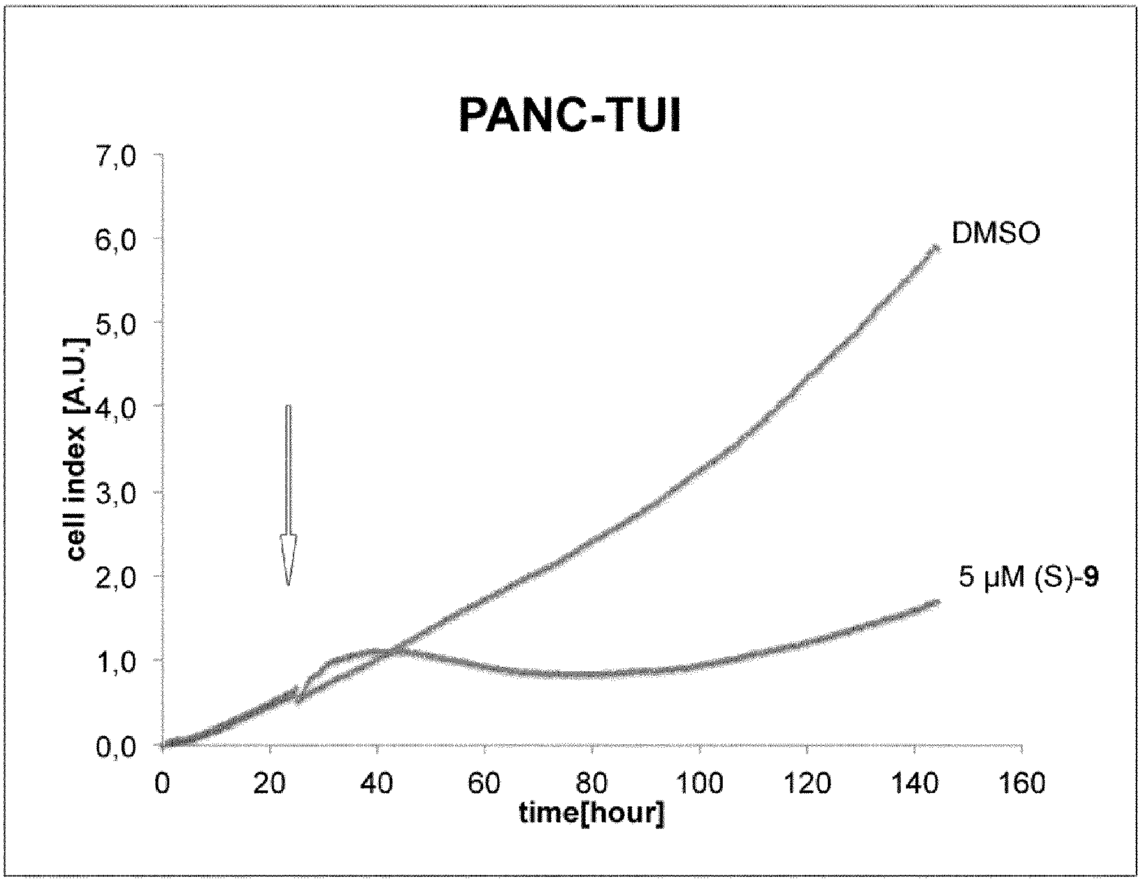
PANC-TUI
7,0
6,0
5,0
4,0
3,0
2,0
1,0
0,0
cell index [A.U.]
0
20
40
60
80
100
120
140
160
time[hour]
DMSO
5 µM (S)-9

Figure 5

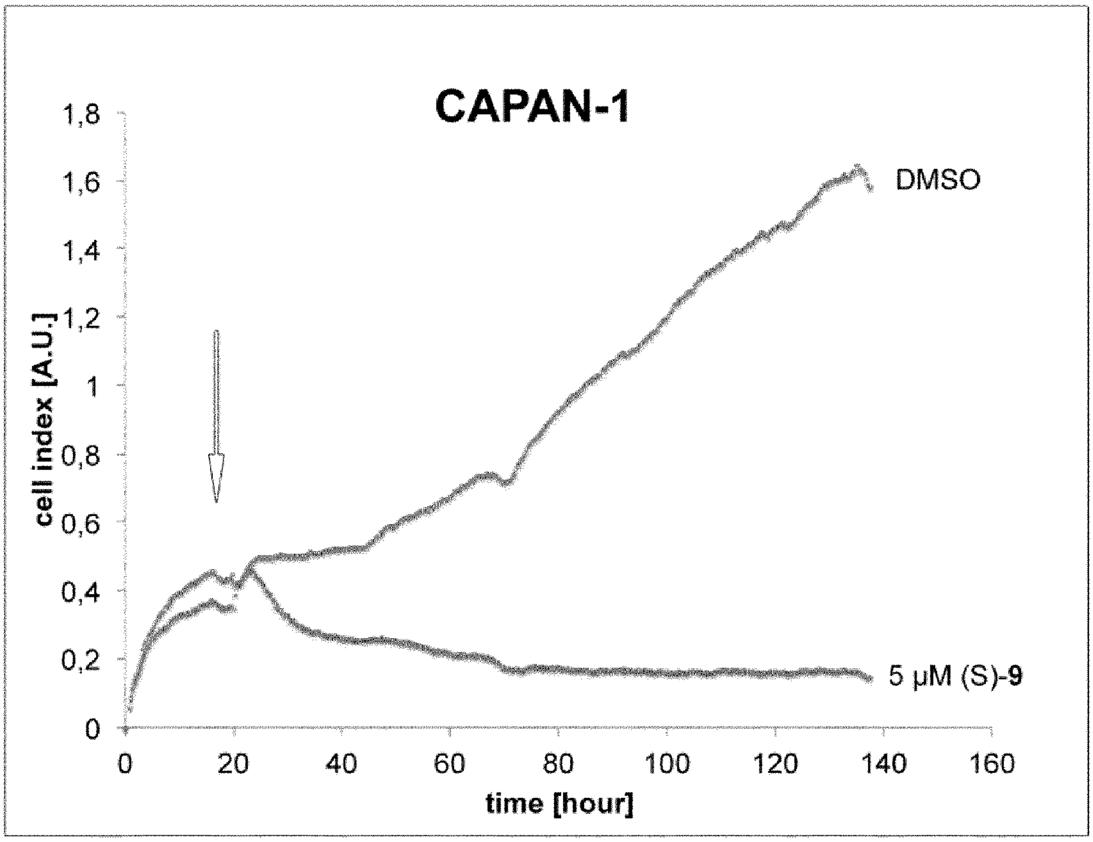
CAPAN-1
1,8
1,6
1,4
1,2
1
0,8
0,6
0,4
0,2
0
cell index [A.U.]
DMSO
5 µM (S)-9
0
20
40
60
80
100
120
140
160
time [hour]

Figure 6

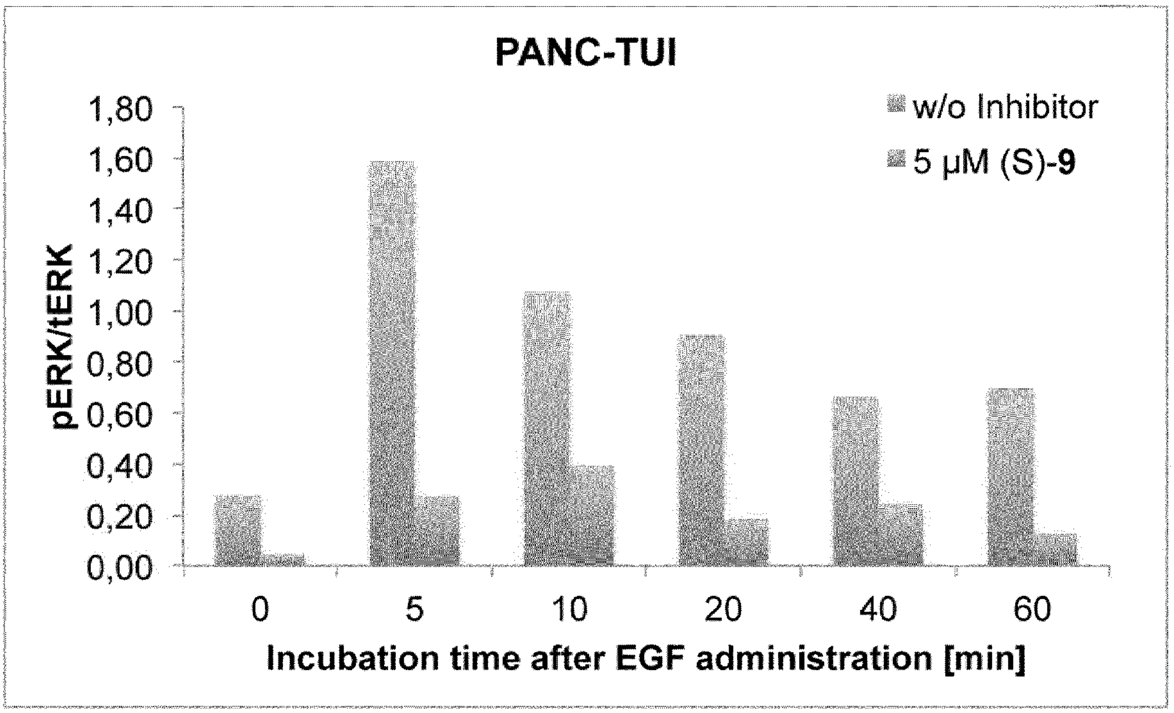
PANC-TUI
w/o Inhibitor
5 µM (S)-9
pERK/tERK
1,80
1,60
1,40
1,20
1,00
0,80
0,60
0,40
0,20
0,00
0
5
10
20
40
60
Incubation time after EGF administration [min]

Figure 7

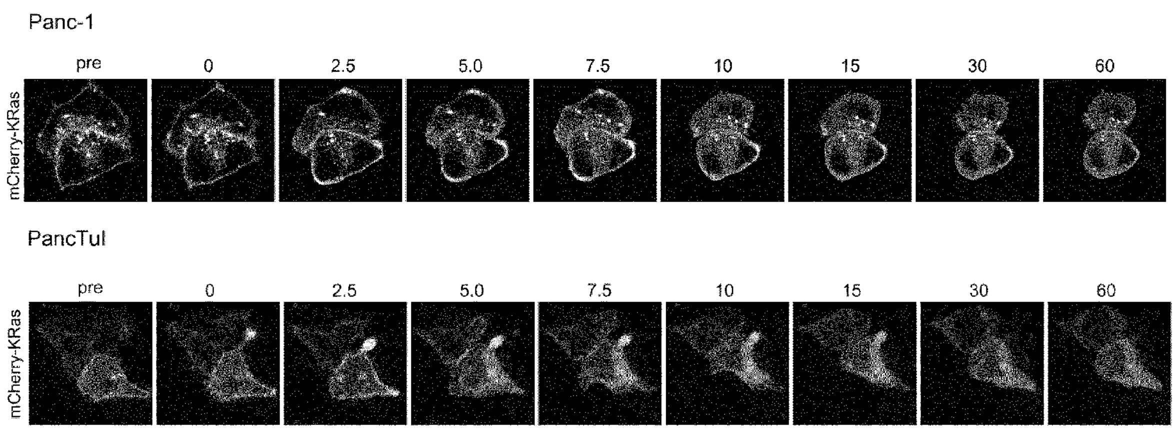
Panc-1
pre
0
2.5
5.0
7.5
10
15
30
60
mCherry-KRas
PancTuI
pre
0
2.5
5.0
7.5
10
15
30
60
mCherry-KRas

Europäisches Patentamt
European Patent Office
Office européen des brevets

# EUROPEAN SEARCH REPORT

Application Number
EP 12 18 0482

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LIU, T. ET AL: "Synthesis and evaluation of 2-[2-(phenylthiomethyl)-1-benzo[] imidazol-1-yl)acetohydrazide derivatives as antitumor agents", BIOORGANIC & MEDICINAL CHEMISTRY LETTERS, vol. 22, no. 9, 15 March 2012 (2012-03-15), pages 3122-3125, XP028410868, PERGAMON, ELSEVIER SCIENCE; GB ISSN: 0960-894X, DOI: 10.1016/J.BMCL.2012.03.061 [retrieved on 2012-03-21] * page 3122, column 1, line 1 - line 4 * * scheme 1; page 3123; table 1 * | 1-10 | INV. C07D235/08 C07D235/12 C07D235/14 C07D235/18 C07D235/20 C07D401/04 C07D401/06 C07D401/14 C07D403/04 C07D403/12 C07D405/04 C07D405/14 C07D409/04 C07D487/04 C07D493/10 A61K31/404 A61P35/00 |
| A | US 2010/179146 A1 (HUANG JIANN-JYH [TW] ET AL) 15 July 2010 (2010-07-15) * page 1, paragraph 2 - paragraph 4 * * page 1; compound (I) * * page 2; compounds (II), (III) * * page 3 - page 26; table 1 * | 1-10 | |
| | | | TECHNICAL FIELDS SEARCHED (IPC) |
| | | | C07D |

The present search report has been drawn up for all claims

| Place of search | Date of completion of the search | Examiner |
|---|---|---|
| Berlin | 21 December 2012 | Hoepfner, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

Europäisches Patentamt
European Patent Office
Office européen des brevets

Application Number
EP 12 18 0482

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

## LACK OF UNITY OF INVENTION SHEET B

Application Number
EP 12 18 0482

The Search Division considers that the present European patent application does not comply with the requirements of unity of invention and relates to several inventions or groups of inventions, namely:

1. claims: 1-10(partially)

   Compounds of Formula (I) having a group R1 which is a linear or cyclic hydrocarbon having no heteroatoms.

   ---

2. claims: 1-10(partially)

   Compounds of Formula (I) having a group R1 comprising at least one heteroatom which is oxygen.

   ---

3. claims: 1-10(partially)

   Compounds of Formula (I) having a group R1 comprising at least one heteroatom which is nitrogen.

   ---

4. claims: 1-10(partially)

   Compounds of Formula (I) having a group R1 comprising at least one heteroatom which is halogen.

   ---

## ANNEX TO THE EUROPEAN SEARCH REPORT ON EUROPEAN PATENT APPLICATION NO. EP 12 18 0482

This annex lists the patent family members relating to the patent documents cited in the above-mentioned European search report.
The members are as contained in the European Patent Office EDP file on
The European Patent Office is in no way liable for these particulars which are merely given for the purpose of information.

21-12-2012

| Patent document cited in search report | Publication date | Patent family member(s) | Publication date |
|---|---|---|---|
| US 2010179146 A1 | 15-07-2010 | TW 201026665 A | 16-07-2010 |
| | | US 2010179146 A1 | 15-07-2010 |

EPO FORM P0459

For more details about this annex : see Official Journal of the European Patent Office, No. 12/82